# EUROPEAN PATENT APPLICATION

(11) **EP 1 914 235 A1**
(43) Date of publication of application: **23.04.2008**
(21) Application number: 06291582.2
(22) Date of filing: 10.10.2006
(51) Int. Cl.: C07D 471/04, A61K 31/4985, A61P 33/06

(54) **CHIRAL TETRA-HYDRO BETA-CARBOLINE DERIVATIVES AND APPLICATIONS THEREOF AS ANTIPARASITIC COMPOUNDS**

(71) Applicant: Université de Lille 2 Droit et Santé, 59800 Lille (FR); INSTITUT PASTEUR DE LILLE, 59000 Lille (FR)
(72) Inventor: Deprez, Benoît, 59000 Lille (FR); Beghyn, Terence, 59320, Haubourdin (FR); Laconde, Guillaume, 59000 Lille (FR); Charton, Julie, 59320, Haubourdin (FR)
(74) Representative: Goulard, Sophie

(57) **Abstract**

The invention relates to the use of chiral tetra-hydro β-carboline derivatives of formula (I) for the preparation of pharmaceutical composition for the prevention and/or the treatment of parasitic diseases involving parasites having a phosphodiesterase activity: 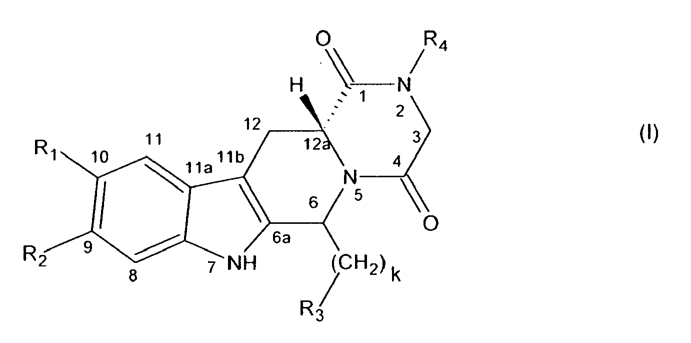 or a pharmaceutically acceptable salt thereof, in which:
- R₁ and R₂, identical or different, represent a hydrogen atom or a fluorine atom;
- k is an integer equal to 0 or 1;
- R₃ is selected from the group consisting of:
■ a phenyl ring optionally substituted
■ a 3'-N-pyridine ring optionally substituted
- R₄ is a group selected in the group consisting of the following groups: -NH-A-R₅, -NHC(O)-R₅ and the groups of formulas (II-a) to (II-c) below: 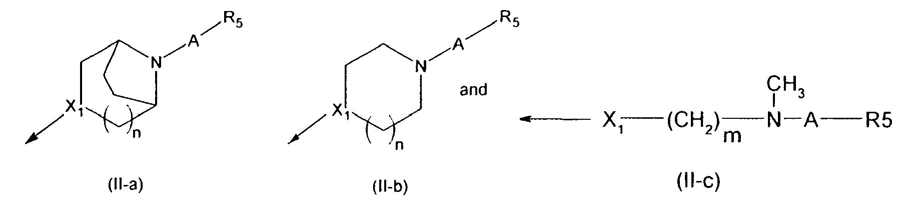 The invention also relates to some new chiral tetra-hydro β-carboline derivatives.

## Description

The Invention relates to the use of chiral tetra-hydro β-carboline derivatives for the preparation of a pharmaceutical composition for the prevention and/or the treatment of parasitic diseases involving parasites having a phosphodiesterase activity. The invention also relates to some new chiral tetra-hydro β-carboline derivatives.

Malaria kills a child somewhere in the world every 30 seconds. It infects about 350-500 million people each year, causing approximately 1.3-3 million deaths annually (Campbell, Neil A. et al. "Biology" Seventh edition. Menlo Park, CA: Addison Wesley Longman, Inc. 2005). Ninety per cent of those who die are in Africa, where malaria accounts for about one in five of all childhood deaths. The disease also contributes greatly to anaemia among children, a major cause of poor growth and development. During pregnancy, malaria is associated with maternal illness and severe anaemia. It contributes to low birth weight among newborn infants -- one of the leading risk factors for infant mortality. Malaria also has serious economic impacts in Africa, slowing economic growth and development and perpetuating the vicious cycle of poverty.

Malaria is caused by protozoan parasites of the genus *Plasmodium* (phylum Apicomplexa): *P. falciparum, P. malariae, P. ovale,* and *P. vivax.* Their primary hosts and transmission vectors are female mosquitos of genus *Anopheles*; humans act as intermediate hosts. The *P. falciparum* variety of the parasite accounts for 80% of cases and 90% of deaths. Children under the age of five and pregnant women are the most vulnerable to the severe forms of malaria.

There are treatments and tools that can help. Sleeping under insecticide treated nets can reduce child mortality by 20 per cent. However, the insecticide loses its effectiveness over time, so the nets must be retreated.

Prompt access to effective treatment can further reduce deaths. Intermittent preventive treatment of Malaria during pregnancy can significantly reduce the proportion of low birth weight infants and maternal deaths.

Several drugs can be taken preventively. Generally, these drugs are taken daily or weekly, at a lower dose than would be used for treatment of a person who had actually contracted the disease. Use of prophylactic drugs is seldom practical for full-time residents of malaria-endemic areas, and their use is usually restricted to short-term visitors and travelers to malarial regions. This is due to the potentially high cost of purchasing the drugs, because long-term use of some drugs may have negative side effects, and because some effective anti-malarial drugs are difficult to obtain outside of wealthy nations.

Quinine was used starting in the seventeenth century as a prophylactic against malaria. The development of more effective alternatives such as quinacrine, chloroquine, and primaquine in the twentieth century reduced the reliance on quinine. Today, quinine is still used to treat chloroquine resistant *Plasmodium falciparum*, as well as severe and cerebral stages of malaria, but is not generally for malaria prophylaxis.

Modern drugs used preventively include mefloquine (Lariam®), doxycycline (available generically), and atovaquone proguanil hydrochloride (Malarone®). The choice of which drug to use is usually driven by what drugs the parasites in the area are resistant to, as well as side-effects and other considerations.

To be effective as a treatment in patients who have actually contracted malaria, these drugs have to be administered at daily or weekly higher dose. This has for inevitable consequences the increasing of negative side effects and to the development of an increased number of resistant to treatment parasites.

There is therefore an evident need for new effective drugs which could be used for the treatment and/or the prevention of malaria, and more generally all parasitic diseases due to parasites having a phosphodiesterase activity, at a lower daily or weekly dose than the drugs actually on the market and which could also be alternatively used to prevent and or treat parasitic diseases caused by resistant-to-treatment parasites.

The inventors have developed the subject of the invention in order to remedy these problems.

In particular, the Inventors have found that judiciously selected tetra-hydro β-carboline type compounds, responding to hereafter defined formula (I), have an enhanced antiparasitic activity against the genus *Plasmodium,* and more generally against all parasites having a phosphodiesterase (PDE) activity, and can therefore advantageously be used, as an active principle, for the preparation of an antiparasitic pharmaceutical composition for the prevention and/or the treatment of parasitic diseases involving parasites having a PDE activity.

A first subject of the Invention is therefore the use, as an active principle, of at least one chiral tetra-hydro β-carboline derivative of general formula (I) below: 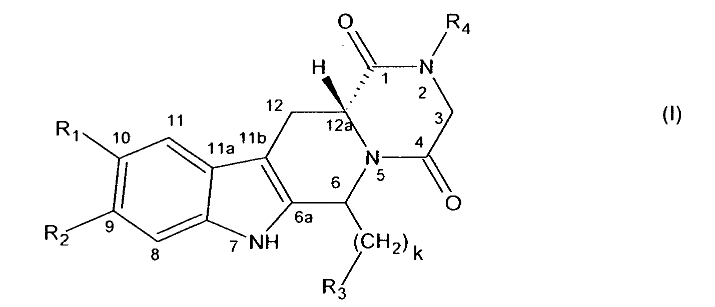 or a pharmaceutically acceptable salt thereof, in which:
- R₁ and R₂, identical or different, represent a hydrogen atom or a fluorine atom;
- k is an integer equal to 0 or 1;
- R₃ is selected from the group consisting of:
   ■ a phenyl ring optionally substituted by a methylenedioxy radical, a difluoromethylenedioxy radical attached to the phenyl ring at the 3'-4' carbon atoms, a thiadiazole fused with the phenyl ring at the 3'-4' positions, a difluoromethoxy radical or a chlorine atom in the para 4' position with regard to the attachment point of said phenyl ring to the piperidine cycle directly bearing R₃ when k = 0 or to the carbon atom of the -CH₂- group when k = 1;
   ■ a 3'-*N*-pyridine ring optionally substituted by a substituent chosen from a chlorine atom, a fluorine atom, a methoxy group, and a methylthio group, said substituent being in the 4' position with regard to the attachment point of said 3'-N-pyridine ring to the piperidine cycle directly bearing R₃ when k = 0 or to the carbon atom of the -CH₂- group when k = 1;
- R₄ is a group selected in the group consisting of the following groups: -NH-A-R₅; -NHC(O)-R₅ and the groups of formulas (II-a) to (II-c) below: 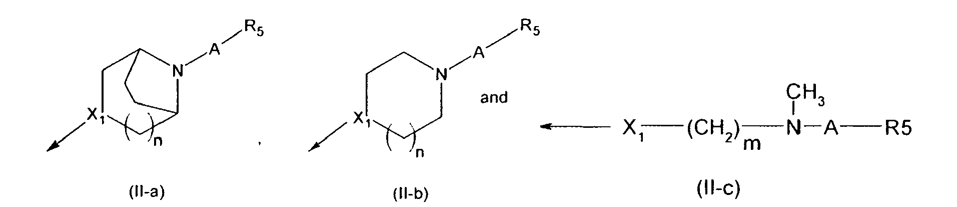
in which:
■ n is an integer equal to 0 or 1,
■ m is an integer equal to 0, 1 or 2,
■ the arrows represent the attachment point of groups of formulas (II-a), (II-b) or (II-c) on the formula (I) derivative piperazine-dione cycle;
■ X₁ is a carbon or a nitrogen atom;
   - A represents a single bond or a C₁ to C₃ linear or branched alkyl chain optionally interrupted or terminated by an oxygen atom;
   - R₅ is selected in the group consisting of:
      **i)** a group of formula (III) below: 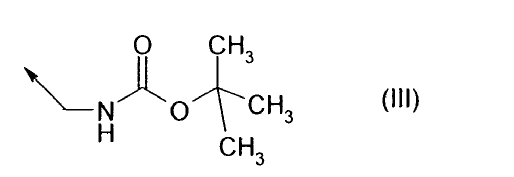 in which the arrow represents the attachment point of said group of formula (III) to A *via* a covalent bond;
      **ii)** a group of formulas (IV) or (V) below: 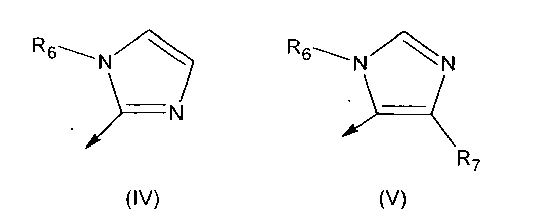 in which the arrows represent the attachment point of said group of formulas (IV) and (V) to A *via* a covalent bond and R₆ and R₇, identical or different, represent a C₁ to C₄ alkyl group; and
      **iii)** a group of the following formula (VI): 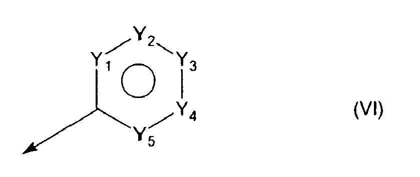
in which:
- the arrow represents the attachment point of said group of formula (VI) to A *via* a covalent bond,
- Y₁ and Y₅, identical or different, represent -CH=; -N= or
- C(R₈)= in which R₈ represents an hydrogen atom, a methyl radical or a halogen atom selected from chlorine and fluorine;
- Y₃ represents -CH=; -N=; -C(R₉)= or -C(R₁₀)=;
- Y₂ and Y₄, identical or different, represent -CH=, -N=, -C(R₉)= or -C(R₁₀)=;
- R₉ represents a hydrogen atom or a group chosen from a methyl, an amino group and an oxygen group, said groups being optionally mono- or disubstituted with a methyl radical;
- Y₂ and Y₃ can also form together a methylenedioxy group;
- R₁₀ represents a hydrogen atom or a solubilizing group;
wherein the asymmetric carbon atom (number 12a) of said derivative of formula (I) is in the *R* conformation, for the preparation of a pharmaceutical composition for the prevention and/or the treatment of parasitic diseases involving parasites having a phosphodiesterase (PDE) activity.

The compounds of the present invention are potent and selective inhibitors of cAMP and/or cGMP PDE and the inventors have actually demonstrated that they inhibit parasite growth in infected mice at doses ranging from 1 to 50 mg per kg when administered by the oral route (*per os*) or by intra-peritoneal or intravenous injections. They also inhibit the growth of plasmodium parasites in red blood cells at concentrations ranging from 1 nM to 10 µM.

The chiral tetra-hydro β-carboline derivatives of formula (I) make possible to prevent and/or treat parasitic diseases involving parasites which have developed a particular resistance to usual antiparasitic agents, when they are administered alone or in combination with usual antimalarial drugs.

According to a prefered embodiment of the invention, the chiral tetra-hydro β-carboline derivatives of formula (I) are used for the preparation of a pharmaceutical composition for the prevention and/or the treatment of malaria, leishmaniasis or trypanosomiasis; the prevention and treatment of malaria being the most prefered indication.

In the derivatives of formula (I), the solubilizing groups above-mentioned for R₁₀ can be chosen amongst the prior well known water solubilizing groups, preferably such as those of the following formulas S-1 to S-5: 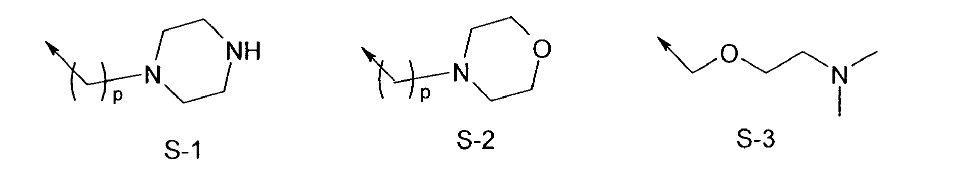 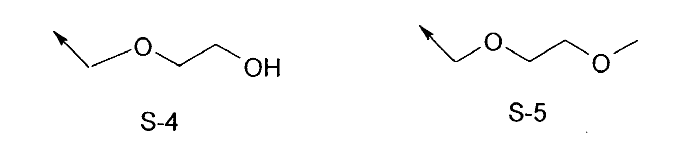 in which p is an integer equal to 0 or 1 and the arrows represent the attachment point of said groups S-1 to S-5 to the carbon atom designated for Y₂, Y₃ and Y₄ in the corresponding group of formula (VI) as above-defined.

According to a preferred embodiment of the invention, R₃ represents one of the following groups:

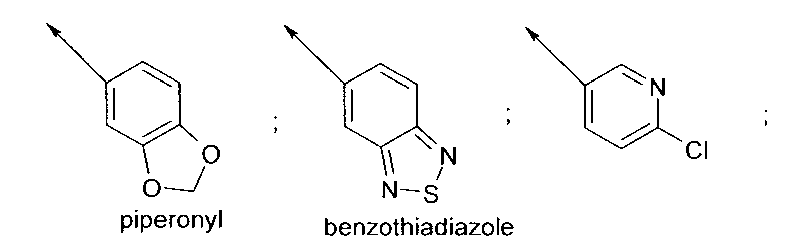

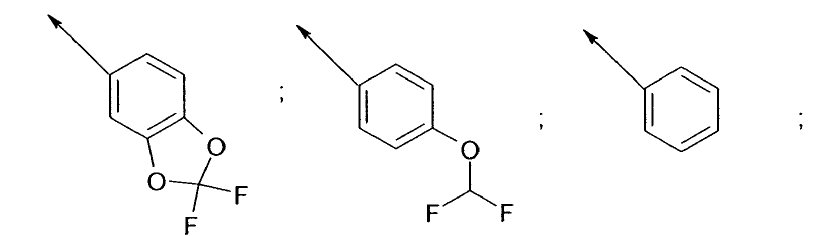

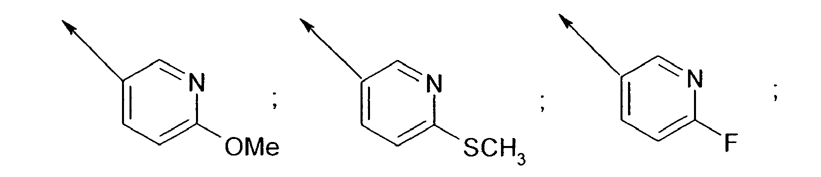

According to another preferred embodiment of the invention, R₄ is a group of formula (II-b) in which n = 1 and X₁ is a carbon or a nitrogen atom.

In the groups -NH-A-R₅ and in the groups of formulas (II-a) to (II-c) mentioned for R₄, A preferably represents -CH₂-; -CH(CH₃)-; -C(CH₃)₂-or -CH₂-CH₂.

In the groups of formula (II-a) to (II-c), R₅ preferably represents one of the following groups of formulas (IV-1) to (IV-14):

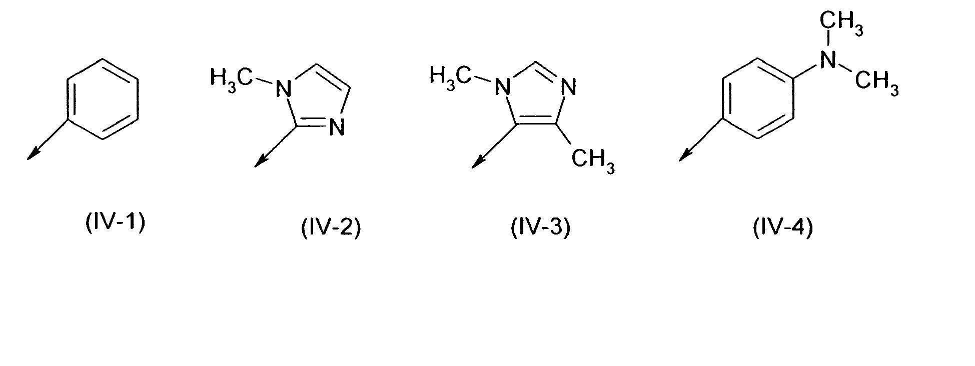

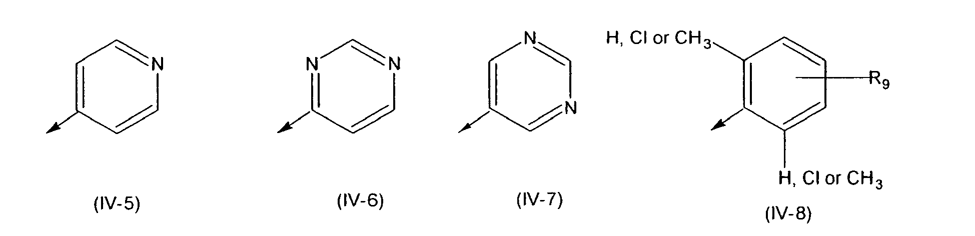

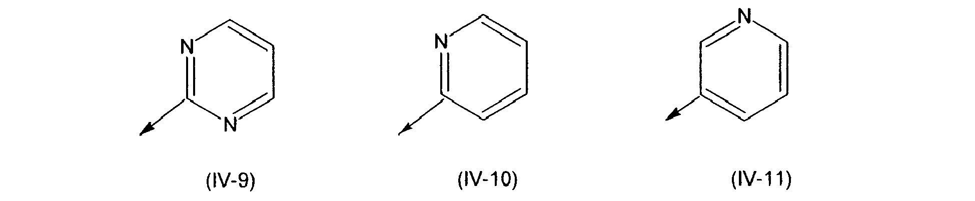

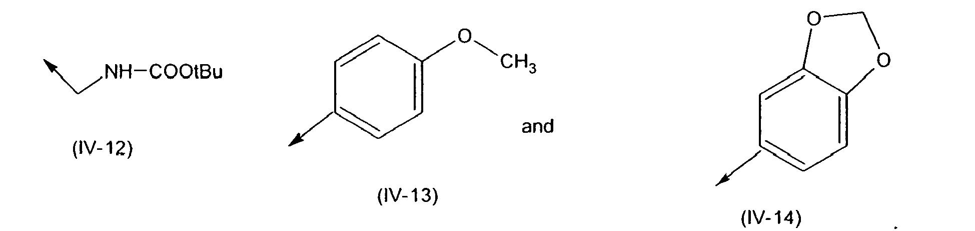

Amongst said derivatives of formula (I), those in which:
i) k = 0, R₃ represents a piperonyl, a benzothiadiazole, a 4-chloro-3-N-pyridine or a 4-fluoro-3-N-pyridine group and R₄ represents a group of formula (II-b) in which X₁ is a carbon atom or a nitrogen atom, n = 1, A is -CH₂- and R₅ is a group of formulas (IV-2), (IV-3), (IV-8), (IV-10) or (IV-12);
ii) k = 0, R₃ represents a piperonyl, a benzothiadiazole, a phenyl or a 4-chloro-3-N-pyridine group and R₄ represents a group of formula (II-b) in which X₁ is a carbon atom or a nitrogen atom, n = 0, A is -CH₂- and R₅ is a group of formula (IV-1) or (IV-2);
iii) k = 0, R₃ represents a phenyl group and R₄ represents a group of formula (II-b) in which X₁ is a carbon atom or a nitrogen atom, n = 1, A is -CH₂- and R₅ is a phenyl group of formula (IV-1);
iv) k = 0, R₃ represents a piperonyl or a benzothiadiazole group and R₄ represents a group of formula (II-c) in which X₁ is a carbon atom or a nitrogen atom, m = 3, A is -CH₂- and R₅ is a phenyl group of formula (IV-1);
v) k = 0, R₃ represents a piperonyl or a benzothiadiazole group and R₄ represents a group of formula (II-c) in which X₁ is a carbon atom or a nitrogen atom, m = 2, A is -CH₂- and R₅ is a phenyl group of formula (IV-1);
vi) k = 0, R₃ represents a piperonyl or a benzothiadiazole group and R₄ represents a group of formula (II-b) in which X₁ is a carbon atom or a nitrogen atom, n = 1, A is -CH₂- and R₅ is a phenyl group of formula (IV-1);
vii) k = 0, R₃ represents a piperonyl or a benzothiadiazole group and R₄ represents a group of formula (II-b) in which X₁ is a carbon atom or a nitrogen atom, n = 0, A is -CH₂- and R₅ is a group of formulas (IV-4), (IV-10), (IV-12), (IV-13) or (IV-14);
viii) k = 0, R₃ represents a 3',4'-(difluoromethylenedioxy)-phenyl group, R₄ represents a group of formula (II-b) in which X₁ is a carbon atom or a nitrogen atom, n = 1, A is -CH₂- and R₅ is a group of formula (IV-1);
ix) k = 0, R₃ represents a 4'-difluoromethoxyphenyl group, R₄ represents a group of formula (II-b) in which X₁ is a carbon atom or a nitrogen atom, n = 1, A is -CH₂- and R₅ is a group of formula (IV-1);
x) k = 1, R₃ represents a phenyl group, R₄ represents a group of formula (II-b) in which X₁ is a carbon atom or a nitrogen atom, n = 1, A is -CH₂- and R₅ is a group of formula (IV-1) or (IV-2);
xi) k = 0, R₃ represents a piperonyl, a benzothiadiazole, a 4-fluoro-3-N-pyridine or a 4-chloro-3-N-pyridine group, R₄ represents a group of formula (II-b) in which X₁ is a carbon atom or a nitrogen atom, n = 1, A is single bond and R₅ is a group of formula (IV-9), and pharmaceutically acceptable salts thereof, are preferred.

Among compounds of formula (I) here above listed under items i) to xi), those in which R₁ and R₂ simultaneously represent a hydrogen atom and those in which one of R₁ and R₂ represents a fluorine atom while the other of R₁ and R₂ represents a hydrogen atom are particularly preferred.

Among compounds of formula (I) here above listed under items i) to xi), those in which X₁ represents a carbon atoms are also particularly prefered.

As particular compounds of formula (I), one can cite:
- (6R,12aR)-6-Benzo[1,3]dioxol-5-yl-2-[(R)-1-(1-methyl-1*H*-imidazol-2-yl methyl)-pyrrolidin-3-yl]-2,3,6,7,12,12a-hexahydro-pyrazino[1',2':1,6]pyrido[3,4-*b*]indole-1,4-dione;
- (6S,12aR)-6-Benzo[1,3]dioxol-5-yl-2-[(R)-1-(1-methyl-1*H*-imidazol-2-yl methyl)-pyrrolidin-3-yl]-2,3,6,7,12,12a-hexahydro-pyrazino[1',2':1,6]pyrido[3,4-*b*]indole-1,4-dione;
- (6R,12aR)-2-(1-Benzyl-piperidin-4-yl)-6-phenyl-2,3,6,7,12,12a-hexahydro-pyrazino[1',2':1,6]pyrido[3,4-b]indole-1,4-dione;
- (6R,12aR)-2-((R)-1-Benzyl-pyrrolidin-3-yl)-6-(6-chloro-pyridin-3-yl)-2,3,6,7, 12,12a-hexahydro-pyrazino[1',2':1,6]pyrido[3,4-b]indole-1,4-dione;
- (6S,12aR)-2-((R)-1-Benzyl-pyrrolidin-3-yl)-6-(6-chloro-pyridin-3-yl)-2,3,6,7, 12,12a-hexahydro-pyrazino[1',2':1,6]pyrido[3,4-b]indole-1,4-dione;
- (6R,12aR)-2-(1-Benzyl-piperidin-4-yl)-6-(6-chloro-pyridin-3-yl)-2,3,6,7,12, 12a-hexahydro-pyrazino[1',2':1,6]pyrido[3,4-b]indole-1,4-dione;
- (6R,12aR)-6-Benzo[1,3]dioxol-5-yl-2-[3-(benzyl-methyl-amino)-propyl]-2,3,6, 7,12,12a-hexahydro-pyrazino[1',2':1,6]pyrido[3,4-*b*]indole-1,4-dione;
- (6S,12aR)-6-Benzo[1,3]dioxol-5-yl-2-[3-(benzyl-methyl-amino)-propyl]-2,3,6, 7,12,12a-hexahydro-pyrazino[1',2':1,6]pyrido[3,4-*b*]indole-1,4-dione;
- (6R,12aR)-6-Benzo[1,3]dioxol-5-yl-2-[2-(benzyl-methyl-amino)-ethyl]-2,3,6,7, 12,12a-hexahydro-pyrazino[1',2':1,6]pyrido[3,4-*b*]indole-1,4-dione;
- (6S,12aR)-6-Benzo[1,3]dioxol-5-yl-2-[2-(benzyl-methyl-amino)-ethyl]-2,3,6,7, 12,12a-hexahydro-pyrazino[1',2':1,6]pyrido[3,4-*b*]indole-1,4-dione;
- (6R,12aR)-6-Benzo[1,3]dioxol-5-yl-2-(1-benzyl-piperidin-4-yl)-2,3,6,7,12,12a-hexahydro-pyrazino[1',2':1,6]pyrido[3,4-*b*]indole-1,4-dione;
- (6R,12aR)-6-Benzo[1,3]dioxol-5-yl-2-(1-benzyl-piperidin-4-yl)-9-fluoro-2,3,6, 7,12,12a-hexahydro-pyrazino[1',2':1,6]pyrido[3,4-b]indole-1,4-dione;
- 6-Benzo[1,3]dioxol-5-yl-2-(1-benzyl-piperidin-4-yl)-9-fluoro-2,3,6,7,12,12a-hexahydro-pyrazino[1',2':1,6]pyrido[3,4-b]indole-1,4-dione in the form of a mixture of enantiomers (CIS : (1R,3R) and (1S,3S));
- (6R,12aR)-6-Benzo[1,3]dioxol-5-yl-2-((R)-1-benzo[1,3]dioxol-5-ylmethyl-pyrrolidin-3-yl)-2,3,6,7,12,12a-hexahydro-pyrazino[1',2':1,6]pyrido [3,4-*b*]indole -1,4-dione;
- (6R,12aR)-6-Benzo[1,3]dioxol-5-yl-2-[(R)-1-(4-methoxy-benzyl)-pyrrolidin-3-yl]-2,3,6,7,12,12a-hexahydro-pyrazino[1',2':1,6]pyrido[3,4-*b*]indole-1,4-dione;
- (6R,12aR)-6-Benzo[1,3]dioxol-5-yl-2-((R)-1-pyridin-2-ylmethyl-pyrrolidin-3-yl)-2,3,6,7,12,12a-hexahydro-pyrazino[1',2':1,6]pyrido [3,4-*b*]indole-1,4-dione;
- (6R,12aR)-6-Benzo[1,3]dioxol-5-yl-2-[(R)-1-(4-dimethyl-amino-benzyl)-pyrrolidin-3-yl]-2,3,6,7,12,12a-hexahydro-pyrazino[1',2':1,6]pyrido[3,4-b]indole-1,4-dione;
- 2-[(R)-3-((6R,12aR)-6-Benzo[1,3]dioxol-5-yl-1,4-dioxo-3,4,6,7,12,12a-hexa hydro-1*H*-pyrazino[1',2':1,6]pyrido[3,4-*b*]indol-2-yl)-pyrrolidin-1-yl]-ethyl}-carbamic acid *tert*-butyl ester;
- (6R,12aR)-6-Benzo[1,3]dioxol-5-yl-2-((R)-1-benzyl-pyrrolidin-3-yl)-2,3,6,7,12, 12a-hexahydro-pyrazino[1',2':1,6]pyrido[3,4-*b*]indole-1,4-dione;
- (6S,12aR)-6-Benzo[1,3]dioxol-5-yl-2-((R)-1-benzyl-pyrrolidin-3-yl)-2,3,6,7,12, 12a-hexahydro-pyrazino[1',2':1,6]pyrido[3,4-*b*]indole-1,4-dione;
- (6R,12aR)-6-Benzo[1,3]dioxol-5-yl-2-[1-(1-methyl-1*H*-imidazol-2-ylmethyl)-piperidin-4-yl]-2,3,6,7,12,12a-hexahydro-pyrazino[1',2':1,6]pyrido[3,4-*b*]indole-1,4-dione;
- (6R,12aR)-2-(1-Benzyl-piperidin-4-yl)-6-(4-difluoromethoxy-phenyl)-2,3,6,7, 12,12a-hexahydro-pyrazino[1',2':1,6]pyrido[3,4-b]indole-1,4-dione;
- (6R,12aR)-2-(1-Benzyl-piperidin-4-yl)-6-(2,2-difluoro-benzo[1,3]dioxol-5-yl)-2, 3,6,7,12,12a-hexahydro-pyrazino[1',2':1,6]pyrido[3,4-*b*]indole-1,4-dione;
- (6R,12aR)-6-Benzo[1,2,5]thiadiazol-5-yl-2-((R)-1-benzyl-pyrrolidin-3-yl)-2,3, 6,7,12,12a-hexahydro-pyrazino[1',2':1,6]pyrido[3,4-*b*]indole-1,4-dione;
- (6S,12aR)-6-Benzo[1,2,5]thiadiazol-5-yl-2-((R)-1-benzyl-pyrrolidin-3-yl)-2,3, 6,7,12,12a-hexahydro-pyrazino[1',2':1,6]pyrido[3,4-*b*]indole-1,4-dione;
- (6R,12aR)-6-Benzyl-2-(1-benzyl-piperidin-4-yl)-2,3,6,7,12,12a-hexahydro-pyrazino[1',2':1,6]pyrido[3,4-*b*]indole-1,4-dione;
- (6S,12aR)-6-Benzyl-2-(1-benzyl-piperidin-4-yl)-2,3,6,7,12,12a-hexahydro-pyrazino[1',2':1,6]pyrido[3,4-*b*]indole-1,4-dione;
- (6R,12aR)-6-Benzyl-2-[1-(1-methyl-1*H*-imidazol-2-ylmethyl)-piperidin-4-yl]-2, 3,6,7,12,12a-hexahydro-pyrazino[1',2':1,6]pyrido[3,4-b]indole-1,4-dione;
- (6S,12aR)-6-Benzyl-2-[1-(1-methyl-1*H*-imidazol-2-ylmethyl)-piperidin-4-yl]-2, 3,6,7,12,12a-hexahydro-pyrazino[1',2':1,6]pyrido[3,4-*b*]indole-1,4-dione; and pharmaceutically acceptable salts thereof.

The pharmaceutically acceptable salts of the chiral tetra-hydro β-carboline derivatives of formula (I) can be acid addition salts formed with pharmaceutically acceptable acids. Such a definition encompasses hydrochloride, hydrobromide, sulphate or bisulphate, phosphate or hydrogenophosphate, acetate, benzoate, succinate, fumarate, maleate, lactate, citrate, tartrate, gluconate, methanesulphonate, benzene-sulphonate and paratoluene-sulphonate salts of tetra-hydro β-carboline derivates of formula (I).

The pharmaceutical composition of the present invention may be administered by any suitable route, for example, by oral, buccal, inhalation, sublingual, nasal, percutaneous, i.e., transdermal, or parenteral (including intravenous, intramuscular, subcutaneous and intracoronary) administration. Therefore, the pharmaceutical composition of the invention can be provided in various forms, such as in the form of hard gelatin capsules, of capsules, of compressed tablets, of suspensions to be taken orally, of lozenges or of injectable solutions or in any other form appropriate to the method of administration.

According to a prefered embodiment of the invention, the pharmaceutical composition is for an oral administration.

The pharmaceutical composition according to the invention include those wherein the at least one chiral tetra-hydro β-carboline derivative of above-defined formula (I) is administered in an effective amount to achieve its intended purpose. Determination of the effective amounts is well within the capability of those skilled in the art.

A "therapeutically effective dose" refers to that amount of the chiral tetra-hydro β-carboline derivatives of formula (I) that results in achieving the desired effect. Toxicity and therapeutic efficacy of such derivatives can be easily determined by standard pharmaceutical procedures in cell cultures or experimental animals, e.g., for determining the LD₅₀ (the dose lethal to 50% of the population) end the ED₅₀ (the dose therapeutically effective in 50% of the population). The dose ratio between toxic and therapeutic effects is the therapeutic index, which is expressed as the ratio between LD₅₀ and ED₅₀. β-carboline derivatives of formula (I) which exhibit high therapeutic indices are of course preferred. The data obtained from such data can be used in formulating range of dosage for use in humans. The dosage of β-carboline derivatives of formula (I) preferably lies within a range of circulating concentrations that include the ED₅₀ with little or no toxicity. The dosage can vary within this range depending upon the dosage form employed, and the route of administration.

The exact formulation, route of administration, and dosage can be chosen by the individual physician in view of the patient's conditions. Dosage amount and interval of administration can be adjusted individually to provide plasma levels of the active moiety which are sufficient to maintain the preventive or therapeutic effects.

The amount of pharmaceutical composition administered will therefore depend on the subject being treated, on the subject's weight, the severity of the affliction and the manner of administration.

For human use, the chiral tetra-hydro β-carboline derivatives of formula (I) can be administered alone, but they are preferably administered in admixture with at least one pharmaceutically acceptable carrier, the nature of which will depend on the intended route of administration and the presentation form. Pharmaceutical composition for use according to the present invention thus can be formulated in a conventional manner using one or more physiologically acceptable carriers comprising one or more excipients and auxiliaries that facilitate processing of the chiral tetra-hydro β-carboline derivatives of formula (I) into preparations which can be used pharmaceutically. Amongst the excipients and auxiliaries which can be used in the pharmaceutical composition according to the invention, one can mention antiagglomerating agents, antioxidants, preservatives agents, dyes, vitamins, inorganic salts, taste-modifying agents, smoothing agents, coating agents, isolating agents, stabilizing agents, wetting agents, anti-caking agents, dispersing agents, emulsifying agents, aromas, penetrating agents, solubilizing agents, etc..., mixtures thereof and generally any excipient conventionally used in the pharmaceutical industry.

By way of example, when the pharmaceutical composition is administered orally, the carrier may comprise one or several excipients such as talc, lactose, starch or modified starches, cellulose or cellulose derivatives, polyethylene glycols, acrylic acid polymers, gelatin, magnesium stearate, animal or vegetal fats of natural or synthetic origin, paraffin derivatives, glycols, etc....

In addition to the at least one chiral tetra-hydro β-carboline derivative of formula (I), the pharmaceutical composition may also comprises one or more additional usually used anti-malarial drugs such as for example chloroquine, quinacrine, primaquine and artemisinine.

For general information about the formulation and administration of pharmaceutical compositions, one can obviously refer to the book "Remington's Pharmaceutical Sciences", last edition. Of course, a person skilled in the art will take care on this occasion that the excipient(s) and/or auxiliary(ies) optionally used are compatible with the intrinsic properties attached to the pharmaceutical composition in accordance with the invention.

These pharmaceutical compositions can be manufactured in a conventional manner, e.g., by conventional mixing, dissolving, granulating, dragee-making, emulsifying, encapsulating, entrapping, or lyophilizing processes. Proper formulation is dependent upon the route of administration chosen.

Amongst the chiral tetra-hydro β-carboline derivatives of the above-defined formula (I), some compounds have never been disclosed in the literature.

A further subject of the present invention is therefore the chiral tetra-hydro β-carboline derivatives of formula (I') below: 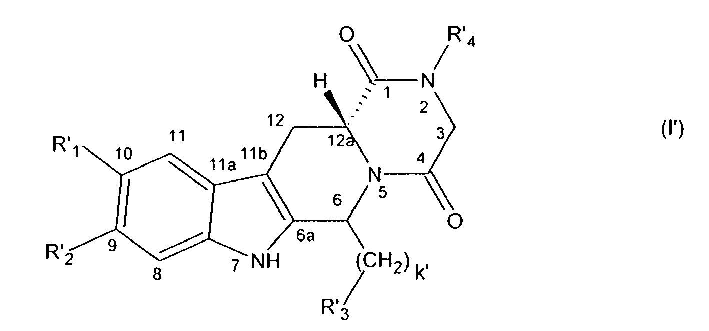 or a pharmaceutically acceptable salt thereof, in which:
- R'₁ and R'₂, identical or different, represent a hydrogen atom or a fluorine atom;
- k' is an integer equal to 0 or 1;
- R'₃ is selected from the group consisting of:
   ■ a phenyl ring optionally substituted by a methylenedioxy radical, a difluoromethylenedioxy radical attached to the phenyl ring at the 3'-4' carbon atoms, a thiadiazole fused with the phenyl ring at the 3'-4' positions, a difluoromethoxy radical or a chlorine atom in the para 4' position with regard to the attachment point of said phenyl ring to the piperidine cycle directly bearing R'₃ when k' = 0 or to the carbon atom of the -CH₂- group when k' = 1;
   ■ a 3'-*N*-pyridine ring optionally substituted by a substituent chosen from a chlorine atom, a fluorine atom, a methoxy group, and a methylthio group, said substituent being in the 4' position with regard to the attachment point of said 3'-N-pyridine ring to the piperidine cycle directly bearing R'₃ when k' = 0 or to the carbon atom of the -CH₂- group when k' = 1;
- R'₄ is a group selected in the group consisting of the following groups: -NH-A-R'₅; -NHC(O)-R'₅ and the groups of formulas (II'-a) to (II'-c) below: 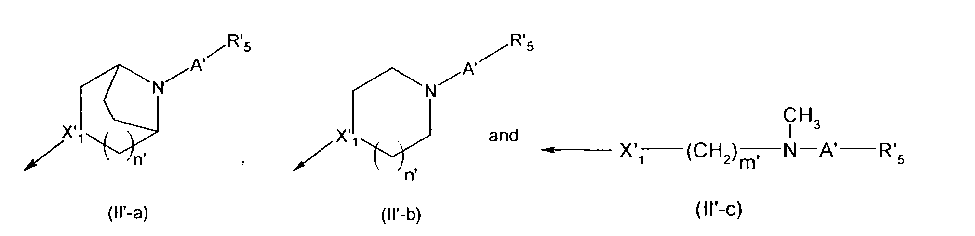
in which:
■ n' is an integer equal to 0 or 1,
■ m' is an integer equal to 0, 1 or 2,
■ the arrows represent the attachment point of groups of formulas (II'-a), (II'-b) or (II'-c) on the formula (I') derivative piperazine-dione cycle;
■ X'₁ is a carbon or a nitrogen atom;
   - A' represents a single bond or a C₁ to C₃ linear or branched alkyl chain optionally interrupted or terminated by an oxygen atom;
   - R'₅ is selected in the group consisting of:
      **i)** a group of formula (III') below: 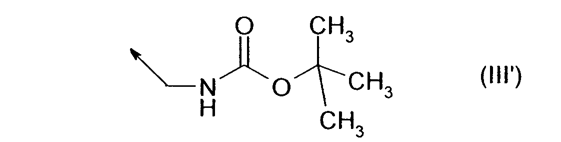 in which the arrow represents the attachment point of said group of formula (III') to A' *via* a covalent bond;
      **ii)** a group of formulas (IV') or (V') below: 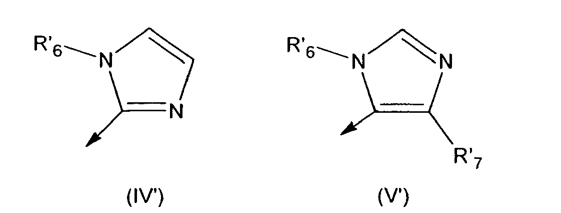 in which the arrows represent the attachment point of said group of formula (IV') and (V') to A' *via* a covalent bond and R'₆ and R'₇, identical or different, represent a C₁ to C₄ alkyl group; and
      **iii)** a group of the following formula (VI'): 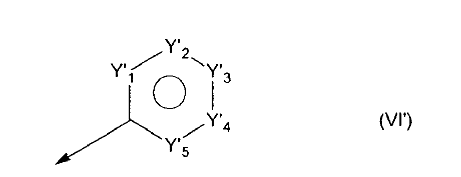
in which:
- the arrow represents the attachment point of said group of formula (VI') to A' *via* a covalent bond,
- Y'₁ and Y'₅, identical or different, represent -CH=; -N= or
- C(R'₈)= in which R'₈ represents an hydrogen atom, a methyl radical or a halogen atom selected from chlorine and fluorine;
- Y'₃ represents -CH=; -N=; -C(R'₉)= or -C(R'₁₀)=;
- Y'₂ and Y'₄, identical or different, represent -CH=, -N=, -C(R'₉)= or -C(R₁₀)=;
- R'₉ represents a hydrogen atom or a group chosen from a methyl, an amino group and an oxygen group, said groups being optionally mono- or disubstituted with a methyl radical;
- Y'₂ and Y'₃ can also form together a methylenedioxy group;
- R'₁₀ represents a hydrogen atom or a solubilizing group;
wherein the asymmetric carbon atom (number 12a) of said derivatives of formula (I') is in the R conformation
with the provisos that:
* when k = 0, and R'₁ and R'₂ simultaneously represent a hydrogen atom, and R'₃ is a piperonyl group, and R'₄ represents a group of formula (II'-b) wherein n' = 0, X₁ is a carbon or a nitrogen atom, and A' = -CH₂-, then R'₅ is different from a phenyl group, a pyridine group, a pyrimidine group, an imidazole group and a 1-methylimidazole group;
* when k = 0, and R'₁ and R'₂ simultaneously represent a hydrogen atom, and R'₃ is a piperonyl group, and R'₄ represents a group of formula (II'-b) wherein n' = 1, X₁ is a carbon or a nitrogen atom, and A' = -CH₂-, then R'₅ is different from a phenyl group.The compounds excluded from the above-defined formula (I') by these two provisos are known in the art, some of them being disclosed by Graham N. Maw et al., Bioorganic & Medicinal Chemistry Letters 13 (2003) 1425-1428.

As particular compounds of formula (I'), one can cite:
- (6R,12aR)-2-(1-Benzyl-piperidin-4-yl)-6-phenyl-2,3,6,7,12,12a-hexahydro-pyrazino[ 1',2':1,6]pyrido[3,4-b]indole-1,4-dione;
- (6R, 12aR)-2-((R)-1-Benzyl-pyrrolidin-3-yl)-6-(6-chloro-pyridin-3-yl)-2,3,6,7, 12,12a-hexahydro-pyrazino[1',2':1,6]pyrido[3,4-b]indole-1,4-dione;
- (6S,12aR)-2-((R)-1-Benzyl-pyrrolidin-3-yl)-6-(6-chloro-pyridin-3-yl)-2,3,6,7, 12,12a-hexahydro-pyrazino[1',2':1,6]pyrido[3,4-b]indole-1,4-dione;
- (6R,12aR)-2-(1-Benzyl-piperidin-4-yl)-6-(6-chloro-pyridin-3-yl)-2,3,6,7,12, 12a-hexahydro-pyrazino[1',2':1,6]pyrido[3,4-b]indole-1,4-dione;
- (6R,12aR)-6-Benzo[1,3]dioxol-5-yl-2-[3-(benzyl-methyl-amino)-propyl]-2,3,6, 7,12,12a-hexahydro-pyrazino[1',2':1,6]pyrido[3,4-*b*]indole-1,4-dione;
- (6S,12aR)-6-Benzo[1,3]dioxol-5-yl-2-[3-(benzyl-methyl-amino)-propyl]-2,3,6, 7,12,12a-hexahydro-pyrazino[1',2':1,6]pyrido[3,4-*b*]indole-1,4-dione;
- (6R,12aR)-6-Benzo[1,3]dioxol-5-yl-2-[2-(benzyl-methyl-amino)-ethyl]-2,3,6,7, 12,12a-hexahydro-pyrazino[1',2':1,6]pyrido[3,4-*b*]indole-1,4-dione;
- (6S,12aR)-6-Benzo[1,3]dioxol-5-yl-2-[2-(benzyl-methyl-amino)-ethyl]-2,3,6,7, 12,12a-hexahydro-pyrazino[1',2':1,6]pyrido[3,4-*b*]indole-1,4-dione;
- (6R, 12aR)-6-Benzo[1,3]dioxol-5-yl-2-(1-benzyl-piperidin-4-yl)-2,3,6,7,12,12a-hexahydro-pyrazino[1',2':1,6]pyrido[3,4-*b*]indole-1,4-dione;
- (6R,12aR)-6-Benzo[1,3]dioxol-5-yl-2-(1-benzyl-piperidin-4-yl)-9-fluoro-2,3,6, 7,12,12a-hexahydro-pyrazino[1',2':1,6]pyrido[3,4-b]indole-1,4-dione;
- 6-Benzo[1,3]dioxol-5-yl-2-(1-benzyl-piperidin-4-yl)-9-fluoro-2,3,6,7,12,12a-hexahydro-pyrazino[1',2':1,6]pyrido[3,4-b]indole-1,4-dione in the form of a mixture of enantiomers (CIS : (1R,3R) and (1S,3S));
- (6R,12aR)-6-Benzo[1,3]dioxol-5-yl-2-((R)-1-benzo[1,3]dioxol-5-ylmethyl-pyrrolidin-3-yl)-2,3,6,7,12,12a-hexahydro-pyrazino[1',2':1,6]pyrido [3,4-*b*]indole -1,4-dione;
- (6R,12aR)-6-Benzo[1,3]dioxol-5-yl-2-[(R)-1-(4-methoxy-benzyl)-pyrrolidin-3-yl]-2,3,6,7,12,12a-hexahydro-pyrazino[1',2':1,6]pyrido[3,4-*b*]indole-1,4-dione;
- (6R,12aR)-6-Benzo[1,3]dioxol-5-yl-2-[(R)-1-(4-dimethyl-amino-benzyl)-pyrrolidin-3-yl]-2,3,6,7,12,12a-hexahydro-pyrazino[1',2':1,6]pyrido[3,4-b]indole-1,4-dione;
- 2-[(R)-3-((6R,12aR)-6-Benzo[1,3]dioxol-5-yl-1,4-dioxo-3,4,6,7,12,12a-hexa hydro-1*H*-pyrazino[1',2':1,6]pyrido[3,4-*b*]indol-2-yl)-pyrrolidin-1-yl]-ethyl}-carbamic acid *tert*-butyl ester;
- (6R,12aR)-6-Benzo[1,3]dioxol-5-yl-2-[1-(1-methyl-1*H*-imidazol-2-ylmethyl)-piperidin-4-yl]-2,3,6,7,12,12a-hexahydro-pyrazino[1',2':1,6]pyrido[3,4-*b*]indole-1,4-dione;
- (6R,12aR)-2-(1-Benzyl-piperidin-4-yl)-6-(4-difluoromethoxy-phenyl)-2,3,6,7, 12,12a-hexahydro-pyrazino[1',2':1,6]pyrido[3,4-b]indole-1,4-dione;
- (6R,12aR)-2-(1-Benzyl-piperidin-4-yl)-6-(2,2-difluoro-benzo[1,3]dioxol-5-yl)-2, 3,6,7,12,12a-hexahydro-pyrazino[1',2':1,6]pyrido[3,4-*b*]indole-1,4-dione;
- (6R,12aR)-6-Benzo[1,2,5]thiadiazol-5-yl-2-((R)-1-benzyl-pyrrolidin-3-yl)-2,3, 6,7,12,12a-hexahydro-pyrazino[1',2':1,6]pyrido[3,4-*b*]indole-1,4-dione;
- (6S,12aR)-6-Benzo[1,2,5]thiadiazol-5-yl-2-((R)-1-benzyl-pyrrolidin-3-yl)-2,3, 6,7,12,12a-hexahydro-pyrazino[1',2':1,6]pyrido[3,4-*b*]indole-1,4-dione;
- (6R,12aR)-6-Benzyl-2-(1-benzyl-piperidin-4-yl)-2,3,6,7,12,12a-hexahydro-pyrazino[1',2':1,6]pyrido[3,4-*b*]indole-1,4-dione;
- (6S, 12aR)-6-Benzyl-2-(1-benzyl-piperidin-4-yl)-2,3,6,7,12,12a-hexahydro-pyrazino[1',2':1,6]pyrido[3,4-*b*]indole-1,4-dione;
- (6R,12aR)-6-Benzyl-2-[1-(1-methyt-1*H*-imidazol-2-ylmethyl)-piperidin-4-yl]-2, 3,6,7,12,12a-hexahydro-pyrazino[1',2':1,6]pyrido[3,4-*b*]indole-1,4-dione;
- (6S,12aR)-6-Benzyl-2-[1-(1-methyl-1*H*-imidazol-2-ylmethyl)-piperidin-4-yl]-2, 3,6,7,12,12a-hexahydro-pyrazino[1',2':1,6]pyrido[3,4-*b*]indole-1,4-dione; and pharmaceutically acceptable salts thereof.

The chiral tetra-hydro β-carboline derivatives of formula (I) according to the invention can be prepared according to the general synthesis scheme A below: 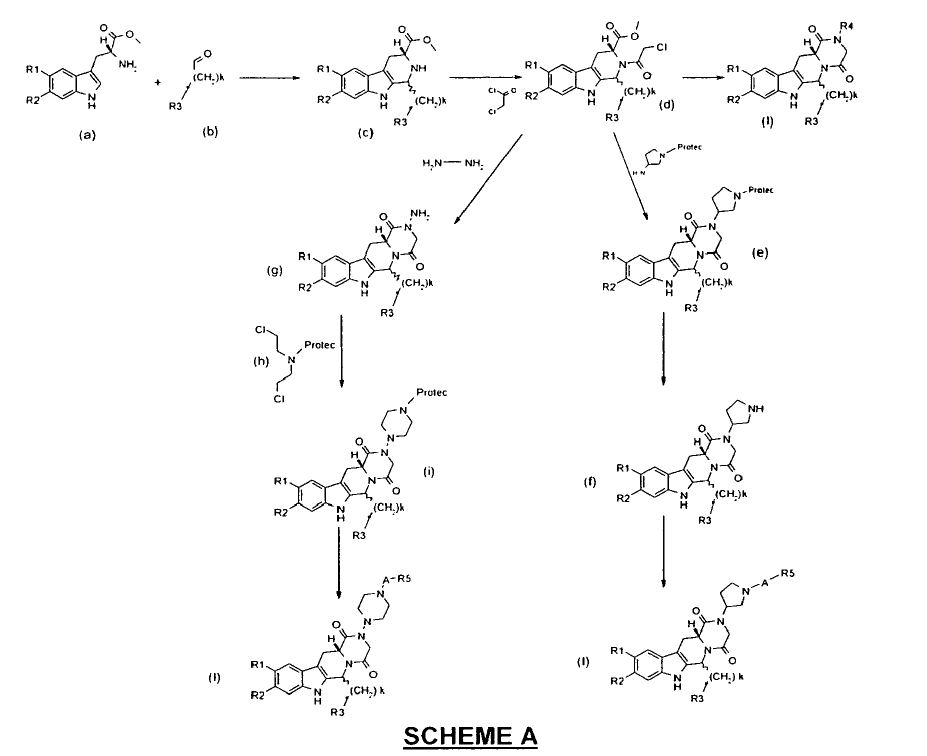 in which R₁ to R₅, A and k have the same significations than those previously indicated for compounds of formula (I) and Protec represents a protecting group. Protecting groups are well known from the one skilled in the art and are described for example in *"*Protective groups in organic synthesis", T.W. GREENE et al., 2nd edition, Wiley Interscience, 1991. As examples of these protecting groups, one can mention benzyl; benzyloxycarbonyle (Z); trifluoroacetyle (TFA); *tert*-butyloxycarbonyle (Boc); trimethylsilylethoxycarbonyle (Teoc) and fluorenylmethyloxycarbonyle (Fmoc) groups.

According to this general process, and in a first step, tryptophan (a) is heated with an aldehyde derivative (b) in the presence of an acid according to the Pictet-Spengler reaction (Pictet, A.; Spengler, T., Ber., 1911, 44, 2030) to give the corresponding compound (c). In a second step compound (c) is acylated with chloroacetyl chloride to lead to compound (d) which, in a third step, is cyclizated with an amine derivative bearing the R₄ radical to lead to the expected compound of formula (I). These reactions are classically used in organic chemistry and are well known from the one skilled in the art.

Another subject matter of the invention is also the chiral tetra-hydro β-carboline derivatives of formula (I') for a use as a drug, in particular as a drug for the prevention and/or the treatment of parasitic diseases involving parasites having a phosphodiesterase (PDE) activity. According to a very prefered embodiment of the invention the drug is for the prevention and/or the treatment of malaria.

Finally, a subject matter of the invention is also a pharmaceutical composition comprising, as an active principle, at least one compounds of formula (I'), and at least one pharmaceutically acceptable excipient.

Besides the arrangements above, the invention also comprises other arrangements which will emerge from the following description, which refers to examples of preparation of β-carboline derivatives of formula (I) according to the invention, to *in vitro* demonstration of the antiparasitic activity of compounds of formula (I) and to *in vivo* demonstration of the antiparasitic activity of compounds of formula (I).

It should be clearly understood, however, that these examples are given only by way of illustration of the subject of the invention, of which they in no way constitute a limitation.

### GENERAL SYNTHESIS PROTOCOLS USED IN THE FOLLOWING EXAMPLES

### I) General Protocol 1

General protocol 1 corresponds to a Pictet-Spengler reaction. D-tryptophan-methylester hydrochloride (D-tryptophan-OMe.HCl) and the aldehyde compound (1 eq.) were dissolved in dry dichloromethane (CH₂Cl₂) (0.05 M) containing activated molecular sieve (4A). The solution was cooled to 0°C. 3 eq. of trifluoroacetic acid in CH₂Cl₂ were added dropwise and the mixture was allowed to warm to room temperature and stirred for 24 h. The reaction mixture was filtered and partially evaporated. The crude product was diluted with ethyl acetate and washed with aqueous NaHCO₃ and water. The organic layer was dried over magnesium sulphate (MgSO₄) and evaporated to dryness. The diastereoisomers were separated by column chromatography.

### II) General Protocol 2: For acylation of β-carbolines intermediates

β-carboline intermediates (1 eq.) were suspended in chloroform (0.2M) with 2.4 eq. of triethylamine (TEA). The reaction mixture was cooled at -10°C and 2.4 eq. of chloroacetyl chloride in chloroform (0.5M) were added dropwise. The reaction mixture was stirred at -10°C until completion and quenched with water. Organic layer was washed with aqueous solution of sodium hydrogenocarbonate (NaHCO₃), and brine, dried over MgSO₄ and evaporated to dryness.

### III) General Protocol 3: For cyclisation of chloro-acetyl intermediates with amines

The chloro-acetyl intermediates (1 eq.) and amine (2 eq.) are refluxed in methanol or ethanol (0.075 M) for 12-24 h. The methanol was removed by evaporation. The residue was dissolved in ethyl acetate, washed with saturated NaHCO₃ aqueous solution, dried over MgSO₄, evaporated to dryness and purified by flash chromatography in a dichloromethane/methanol mixture (CH₂Cl₂/MeOH).

### IV) General Protocol 4: For reductive amination: Procedure 4

The intermediates **23** or **24** as described hereinafter (1 eq.) and the aldehyde (1.5 eq.) were dissolved in a mixture of dichloromethane with acetic acid (CH₂Cl₂/acetic acid) 10/1 (0.1 M). 2 eq. of (polystyrylmethyl) trimethylammonium cyanoborohydride resin were suspended and the reaction mixture was stirred at room temperature for 12-24 hours. The precipitate was dissolved with methanol, the resin was filtered, the solution evaporated to dryness. The reaction mixture was purified by thin layer chromatography (TLC) with a solvent system CH₂Cl₂/MeOH.

### V) General Protocol 5: Synthesis of methylic ester of tryptophan derivatives

Thionyl chloride (3 eq.) was added dropwise to a suspension of tryptophan derivative (1 eq.) in anhydrous methanol at 0°C. The mixture was warmed slowly to room temperature and stirred for 24 h. The thionyl chloride treatment was repeated an additional time as described above. The methanol and the thionyl chloride excess were removed under reduced pressure.

### VI) General Protocol 6: For cyclisation of acylated intermediates with amine in microwaves

Acylated intermediates were dissolved in ethanol (0.04 M) with 2.5 eq. of amine. The reaction mixture was heated by microwaves 100 W for 2-15 minutes. The reaction mixture was totally evaporated and purified by thick layer chromatography.

### EXAMPLE 1: Synthesis of (6R,12aR)-6-Benzo[1,3]dioxol-5-yl-2-((R)-1-benzyl-pyrrolidin-3-yl)-2,3,6,7,12,12a-hexahydro-pyrazino[1',2':1,6]pyrido [3,4-b] indole-1,4-dione (Compound 6).

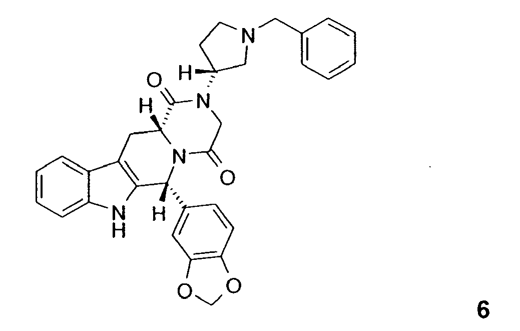

**1) First step:** Synthesis of the intermediate (1R,3R)-1-Benzo[1,3]dioxol-5-yl-2,3,4,9-tetrahydro-1H-β-carboline-3-carboxylic acid methyl ester (Compound **2**) 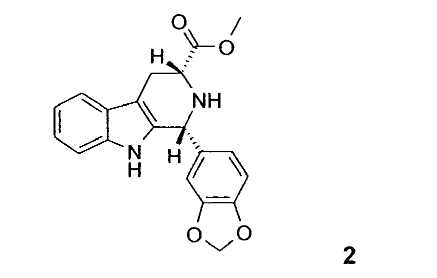 **2** was prepared according to general protocol 1 starting from D-tryptophan OMe.HCl (3 g) and obtained as 950 mg of a white powder (23%).
   **LC :** t_{R}=3.7 min;
   **MS** (ESI+) : m/z=351 (M+H)⁺;
   **¹H NMR** (CDCl₃): δ 7.45 (d, *J*=6.9 Hz, 1H), 7.39 (s, 1H), 7.16 (m, 1 H), 7.10-7.01 (m, 2H), 6.81 (dd, *J*=8.0 Hz and *J*=1.7 Hz, 1 H), 6.75 (d, *J*=1.7 Hz, 1 H), 6.73 (d, *J*=8.0 Hz, 1 H), 5.87 (s, 2H), 5.09 (s, 1 H), 3.88 (dd, *J*=11.1 Hz and *J*=4.3 Hz, 1 H), 3.75 (s, 3H), 3.14 (ddd, *J*=15.0 Hz, *J*=4.3 Hz and *J*=1.9 Hz, 1 H), 2.91 (ddd, *J*=15.0 Hz, *J*=11.0 Hz and *J*=2.5 Hz, 1H);
   **¹³C NMR** (CDCl₃), δ 173.58, 148.58, 148.23, 136.56, 135.08, 127.52, 122.39, 120.04, 118.64, 111.36, 109.26, 109.20, 108.72, 101.64, 58.8, 57.27, 52.68, 26.07,
   **Mp**=160-161°C.
**2) Second step:** Synthesis of the intermediate (1R,3R)-1-Benzo[1,3]dioxol-5-yl-2-(2-chloro-acetyl)-2,3,4,9-tetrahydro-1*H*-β-carboline-3-carboxylic acid methyl ester (Compound **5**) 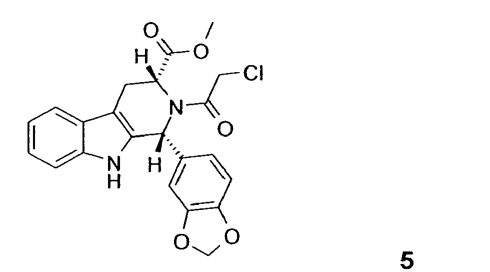 **5** was prepared according to general protocol 2 starting from intermediate **2** of step 1 (2.0 g) and obtained after precipitation in diethyl ether as a white powder (2 g, 83%).
   **LC :** t_{R}=6.2 min;
   **MS** (ESI+) : m/z = 427 (M+H)⁺;
   **¹H NMR** (CDCl₃): δ 7.87 (s, 1H), 7.61 (d, *J*=7.8 Hz, 1H), 7.15-7.32 (m, 3H), 6.84-6.90 (m, 2H), 6.65 (brs, 2H), 5.92 (s, 2H), 4.95 (brs, 1H), 4.20-4.40 (m, 2H), 3.70 (d, *J*=16.2 Hz, 1H), 3.22 (m, 4H).
**3) Third Step:** Synthesis of Compound **6**
   Compound **6** was prepared according to general protocol 3 starting from intermediate **5** as obtained here-above in step 2 (1.55 g) and (R)-(-)-1-Benzyl-3-aminopyrrolidine (2 eq.). Compound **6** was obtained after purification by flash chromatography (CH₂Cl₂/MeOH 99/1: v/v) as a white powder (936 mg, 48%).
   **LC:** t_{R}=4.9 min;
   **MS** (ESI+) : m/z = 535 (M+H)⁺;
   **¹H NMR** (CDCl₃) : δ 8.25 (s, 1H), 7.60 (d, 1H, *J*=6.6 Hz, 1H), 7.14-7.37 (m, 8H), 6.84 (dd, *J*=8.1 Hz and *J*=1.5 Hz, 1 H), 6.75 (d, *J*=1.5 Hz, 1H), 6.68 (d, *J*=8.1 Hz, 1 H), 6.24 (s, 1 H), 5.86 (d, *J*=3.6 Hz, 2H), 5.12 (brs, 1H), 4.41 (brm, 1H), 4.22 (dd, *J*=11.4 Hz and *J*=4.8 Hz, 1H), 3.99 (d, *J*=17.7 Hz, 1H), 3.78 (brs, 2H), 3.70 (dd, *J*=15.9 Hz and *J*=4.8 Hz, 1H), 3.22 (dd, *J*=16.2 Hz and *J*=11.7 Hz, 1 H), 1.79-3.09 (m, 6H).

### EXAMPLE 2: Synthesis of (6R,12aR)-6-Benzo[1,3]dioxol-5-yl-2-(1-benzyl-piperidin-4-yl)-2,3,6,7,12,12a-hexahydro-pyrazino[1',2':1,6]pyrido[3,4-b]indole-1,4-dione (Compound 10)

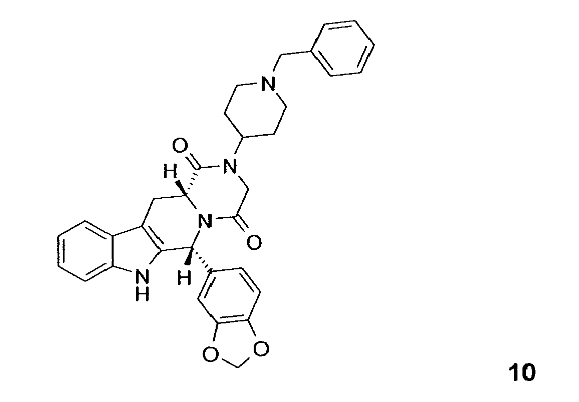

Compound **10** was prepared according to general protocol 3 starting from intermediate **5** as above-obtained at step 2 of Example 1 (100 mg) and 4-amino-N-benzylpiperidine (2 eq.) in ethanol (EtOH). Compound 10 was obtained after purification by flash chromatography (CH₂Cl₂/MeOH) as a white powder (76 mg, 59%).
**LC :** t_{R}=4.7 min;
**MS** (ESI+) : m/z = 549 (M+H)⁺;
**¹H NMR** (MeOD) : δ 7.54 (d, *J*=7.2 Hz, 1 H), 7.27-7.34 (m, 6H), 7.06 (m, 2H), 6.79 (dd, *J*=8.1 Hz and *J*=1.8 Hz, 1H), 6.75 (d, *J*=1.8 Hz, 1H), 6.67 (d, *J*=8.1 Hz, 1 H), 6.21 (s, 1 H), 5.82 (d, *J*=3.6 z, 2H), 4.37 (m, 2H), 4.06 (d, *J*=17.1 Hz, 1 H), 3.96 (d, *J*=17.1 Hz, 1 H), 3.63 (dd, *J*=15.9 Hz and *J*=4.8 Hz, 1 H), 3.53 (s, 2H), 3.13 (dd, *J*=15.0 Hz and *J*=11.4 Hz, 1H), 2.97 (m, 2H), 2.09 (m, 2H), 1.68-1.88 (m, 3H), 1.54 (m, 1 H).

### EXAMPLE 3: Synthesis of (6S,12aR)-6-Benzo[1,3]dioxol-5-yl-2-((R)-1-benzyl-pyrrolidin-3-yl)-2,3,6,7,12,12a-hexahydro-pyrazino[1',2':1,6]pyrido [3,4-b]indole-1,4-dione (Compound 14)

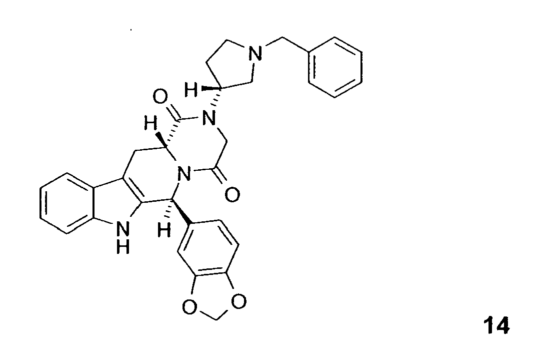

**1) First Step:** Synthesis of the intermediate (1S,3R)-1-Benzo[1,3]dioxol-5-yl-2,3,4,9-tetrahydro-1H-β-carboline-3-carboxylic acid methyl ester (Compound 3) 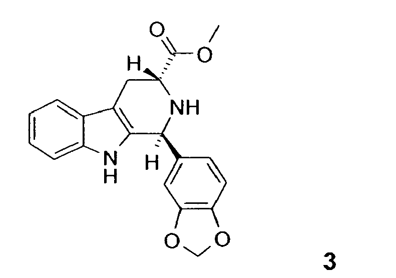 **3** was prepared according to general protocol 1 starting from D-tryptophan OMe.HCl (3 g) and obtained as 870 mg of a white powder (21 %).
   **LC :** t_{R}=3.9 min;
   **MS** (ESI+) : m/z=351 (M+H)⁺;
   **¹H NMR** (CDCl₃), δ 7.52 (s, 1H), 7.47 (d, *J*=6.8 Hz, 1 H), 7.17 (m, 1H), 7.11-7.02 (m, 2H), 6.67 (s, 3H), 5.85 (s, 2H), 5.25 (s, 1 H), 3.90 (t, *J*=6.2 Hz, 1H), 3.63 (s, 3H), 3.18 (dd, *J*=15.5 Hz and *J*=5.5 Hz, 1 H), 3.04 (dd, *J*=15.3 Hz and *J*=6.6 Hz, 1 H);
   **¹³C NMR** (CDCl₃), δ 174.4, 148.46, 147.90, 136.60, 136.15, 133.52, 127.34, 122.43, 122.25, 119.93, 118.68, 111.37, 109.18, 108.56, 101.59, 55.06, 53.88, 52.91, 52.58, 24.93;
   **Mp**=187-189°C
**2) Second Step:** Synthesis of the intermediate (1S,3R)-1-Benzo[1,3]dioxol-5-yl-2-(2-chloro-acetyl)-2,3,4,9-tetrahydro-1H-β-carboline-3-carboxylic acid methyl ester (Compound **4**) 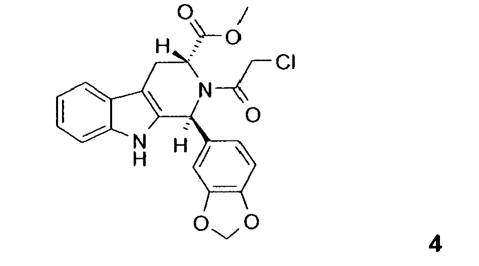 **4** was prepared according to general protocol 2 starting from intermediate **3** as obtained here-above at step 1 (996 mg, 1 eq.). Compound **4** was obtained as a yellow powder (1.06 g, 96%), which was directly engaged in the cyclisation step.
   **LC :** t_{R}=6.3 min;
   **MS** (ESI+) : m/z=427 (M+H)⁺.
**3) Third Step:** Synthesis of Compound **14**
   Compound **14** was prepared according to general protocol 3 starting from intermediate **4** as here-above obtained at step 2 (1.55 g) and (R)-(-)-1-Benzyl-3-aminopyrrolidine (2 eq.). Compound **14** was obtained after purification by flash chromatography (CH₂Cl₂/MeOH 99/1: v/v) as a white powder (936 mg, 48%).
   **LC:** t_{R}=4.8 min;
   **MS** (ESI+) : m/z = 535 (M+H)⁺;
   **¹H NMR** (CDCl₃) : δ 7,88 (s, 1 H), 7.52 (d, *J*=7,6 Hz, 1 H), 7.32-7.13 (m, 8H), 6.96 (s, 1 H), 6.81 (s, 1H), 6.71 (s, 2H), 5.93 (s, 2H), 5.22 (brs, 1H), 4.33 (dd, *J*=11.7 Hz and *J*=4.2 Hz, 1H), 4,29 (d, *J*=17.6 Hz, 1H), 4,12 (d, *J*=18,0 Hz, 1H), 3.57 (s, 2H), 3.50 (dd, *J*=15.1 Hz and *J*=3.8 Hz, 1 H), 2,93 (m, 2H), 2,73 (m, 1 H), 2,47 (dd, *J*=10,5 Hz and *J*=7,6 Hz, 1 H), 2,21 (m, 2H), 1.75 (m, 1 H).

### EXAMPLE 4: Synthesis of (6R,12aR)-6-Benzo[1,3]dioxol-5-yl-2-[2-(benzyl-methyl-amino)-ethyl]-2,3,6,7,12,12a-hexahydro-pyrazino[1',2':1,6]pyrido [3,4-b]indole-1,4-dione (Compound 15)

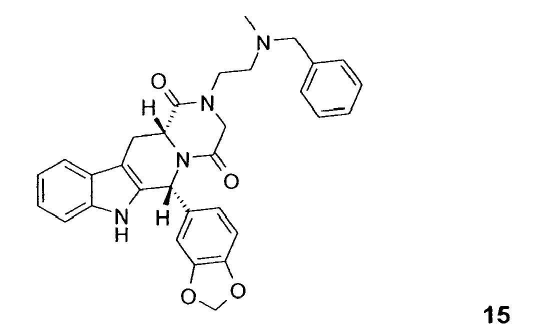

**1) First Step:** Synthesis of a reactive amine: N1-Benzyl-N1-methyl-ethane-1,2-diamine, 2HCl (Compound **20)** 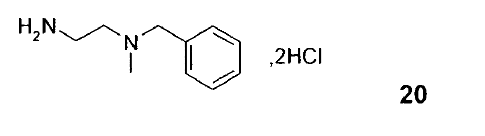
   **a)** Synthesis of [2-(Benzyl-methyl-amino)-ethyl]-carbamic acid *tert*-butyl ester (Compound **19)** 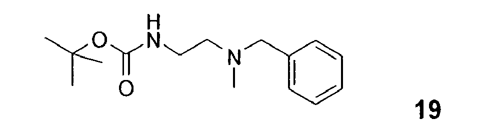 N-Methyl-benzylamine (114 µL, 1 eq.), 2-(Boc-amino)ethylbromide (200 mg, 1 eq.), potassium iodide (74 mg, 0.5 eq.) and cesium carbonate (580 mg, 2 eq.) were dissolved in acetone and stirred at room temperature for 70 h. The reaction mixture was diluted with ethyl acetate, washed with water, dried over MgSO₄, and evaporated to dryness to give 160 mg of yellow oil (68 %).
      **LC :** t_{R}=3.6 min;
      **MS** (ESI+) : m/z = 265 [M+H]⁺;
      **¹H NMR** (CD₂Cl₂) : δ 7,38-7,26 (m, 5H), 5,05 (m, 1 H), 3,53 (s, 2H), 3,23 (q, *J*=5,8 Hz, 2H), 2,51 (t, *J*=6,0 Hz, 2H), 2,21 (s, 3H), 1,47 (s, 9H).
   **b)** Synthesis of (Compound 20)
      Intermediate **19** (160 mg, 600 µmol) was dissolved in dioxane (3 mL), 1.5 mL of HCl 4N in dioxane was added, The reaction mixture was stirred at room temperature for 20h. Dioxane was removed by evaporation to give 125 mg of translucent oil (88%) to lead to compound **20**.
      **LC** : t_{R}=1.1 min;
      **MS** (ESI+) : m/z = 165 [M+H]⁺.
**2) Second Step**: Preparation of Compound **15**

Compound **15** was prepared according to general protocol 3 starting from intermediate **5** as obtained here-above at step 2 of Example 1 (100 mg), Compound 20 as prepared above at Step 1) b) (2 eq.) and TEA (3 eq.) in EtOH. Compound **15** was obtained after purification by flash chromatography (CH₂Cl₂/MeOH) as a yellow powder (50 mg, 41%).
**LC :** t_{R}=4.6 min;
**MS** (ESI+) : m/z = 523 (M+H)⁺;
**¹H NMR** (MeOD) : δ 7.54 (d, *J*=7.2 Hz, 1H), 7.20-7.30 (m, 6H), 7.07 (m, 2H), 6.83 (m, 2H), 6.65 (d, *J*=7.8 Hz, 1H), 6.24 (s, 1H), 5.81 (d, *J*=5.4 Hz, 2H), 4.37 (dd, *J*=11.4 Hz and *J*=3.6 Hz, 1H), 4.20 (dd, *J*=17.1 Hz and J=1.5 Hz, 1H), 4.01 (d, *J*=17.4 Hz, 1H), 3.47-3.69 (m, 5H), 3.12 (dd, *J*=15.9 Hz and *J*=11.4 Hz, 1 H), 2.62 (m, 2H), 2.23 (s, 3H).

### EXAMPLE 5: Synthesis of (6S,12aR)-6-Benzo[1,3]dioxol-5-yl-2-[2-(benzyl-methyl-amino)-ethyl]-2,3,6,7,12,12a-hexahydro-pyrazino[1',2':1,6]pyrido [3,4-b]indole-1,4-dione (Compound 16)

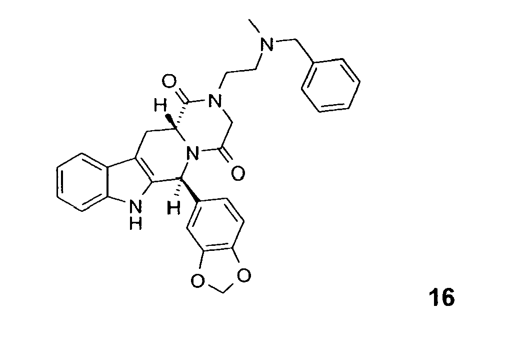

Compound **16** was prepared according to general protocol 3 starting from intermediate **4** as obtained here-above at step 2 of Example 3 (56 mg), Compound **20** as obtained above at Step 1) b) of Example 4 (4 eq.) and TEA (12 eq.) in MeOH (48 h). Compound 16 was obtained after purification by flash chromatography (CH₂Cl₂/MeOH) as a white powder (30 mg, 41%).
**LC :** t_{R}=4.9 min;
**MS** (ESI-) : m/z = 523 (M-H);
**¹H NMR** (MeOD) : 6 7.51 (d, *J*=7.8 Hz, 1H), 7.33 (d, *J*=7.8 Hz, 1 H), 7.21-7.25 (m, 2H), 7.05-7.25 (m, 5H), 6.99 (s, 1 H), 6.85 (d, *J*=1.5 Hz, 1H), 6.80 (d, *J*=8.1 Hz, 1 H), 6.70 (dd, *J*=8.1 Hz and *J*=1.5 Hz, 1H), 5.94 (d, *J*=6.6 Hz, 2H), 4.15-4.34 (m, 3H), 3.55 (m, 4H), 3.34 (m, 1 H), 2.90 (ddd, *J*=15.3 Hz, *J*=11.7 Hz and *J*=1.2 Hz, 1 H), 2.57 (m, 2H), 2.33 (s, 3H).

### EXAMPLE 6: Synthesis of (6R,12aR)-6-Benzo[1,3]dioxol-5-yl-2-[3-(benzyl-methyl-amino)-propyl]-2,3,6,7,12,12a-hexahydro-pyrazino[1',2':1,6]pyrido [3,4-b]indole-1,4-dione (Compound 17)

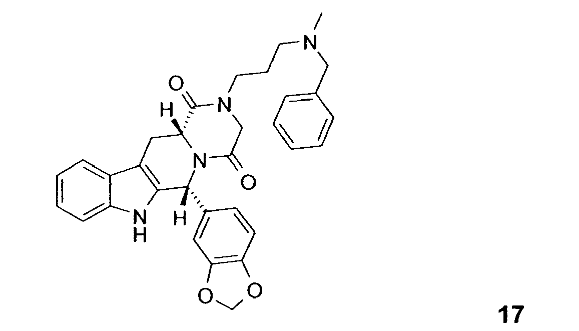

**1) First Step:** Synthesis of a reactive amine: N1-Benzyl-N1-methyl-propane-1,3-diamine, 2HCl (Compound **22)** 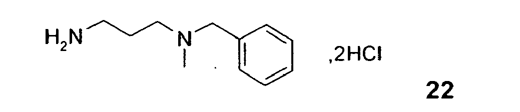
   **a)** Synthesis of [3-(Benzyl-methyl-amino)-propyl]-carbamic acid *tert*-butyl ester (Compound **21**) 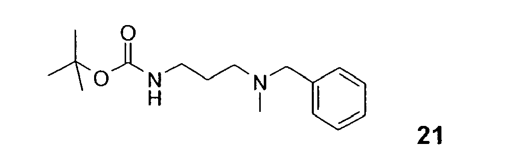 N-Methyl-benzylamine (108 µL, 1 eq.), 2-(Boc-amino)propylbromide (200 mg, 1 eq.), potassium iodide (69 mg, 0.5 eq.) and cesium carbonate (547 mg, 2 eq.) were dissolved in acetone and stirred at room temperature for 70 h. The reaction mixture was diluted with ethyl acetate, washed 3 times with saturated NaHCO₃ aqueous solution, dried over MgSO₄, and evaporated to dryness to give 210 mg of yellow oil (90 %).
      **LC** : t_{R}=3.4 min;
      **MS** (ESI+) : m/z = 279 [M+H]⁺;
      **¹H NMR** (CD₂Cl₂) : δ 7.34-7.25 (m, 5H), 5.62 (m, 1H), 3.51 (s, 2H), 3.18 (q, *J*=6.1 Hz, 2H), 2.47 (t, *J*=6.3 Hz, 2H), 2.18 (s, 3H), 1.69 (qt, J=6.4 Hz, 2H), 1.47 (s, 9H).
   **b)** Synthesis of Compound 22
      Intermediate 21 as obtained above at step a) (210 mg) was dissolved in dioxane (3 mL), 1.5 mL of HCl 4N in dioxane was added, The reaction mixture was stirred at room temperature for 20h. Dioxane was removed by evaporation to give 190 mg of translucent oil (90%).
      **LC** : t_{R}=1.1 min;
      **MS** (ESI+) : m/z = 179 [M+H]⁺.
**2) Second Step:** Synthesis of Compound **17**

Compound **17** was prepared according to general protocol 3 starting from intermediate **5** as obtained here-above at step 2 of Example 1 (100 mg), Compound **22** as above obtained at Step 1) b) (2 eq.) and TEA (3 eq.) in EtOH (5 days). Compound **17** was obtained after purification by flash chromatography (CH₂Cl₂/MeOH) as a white powder (30 mg, 24%).
**LC:** t_{R}=4.7 min;
**MS** (ESI+): m/z = 537 (M+H)⁺;
**¹H NMR** (MeOD) : δ 7.54 (d, *J*=6.9 Hz, 1H), 7.21-7.33 (m, 6H), 7.07 (m, 2H), 6.81 (m, 2H), 6.68 (d, *J*=8.1 Hz, 1 H), 6.23 (s, 1H), 5.83 (s, 2H), 4.36 (dd, *J*=11.7 Hz and *J*=3.9 Hz, 1H), 4.22 (dd, *J*=17.1 Hz and *J*=1.2 Hz, 1H), 3.99 (d, *J*=17.1 Hz, 1 H), 3.62 (dd, *J*=15.6 Hz and *J*=4.8 Hz, 1 H), 3.52 (m, 4H), 3.12 (dd, *J*=14.7 Hz and *J*=11.4 Hz, 1H), 2.44 (td, *J*=6.9 Hz and *J*=3.0 Hz, 2H), 2.20 (s, 3H), 1.85 (qt, *J*=7.5 Hz, 2H).

### EXAMPLE 7: Synthesis of (6S,12aR)-6-Benzo[1,3]dioxol-5-yl-2-[3-(benzyl-methyl-amino)-propyl]-2,3,6,7,12,12a-hexahydro-pyrazino[1',2':1,6]pyrido [3,4-b]indole-1,4-dione (Compound 18)

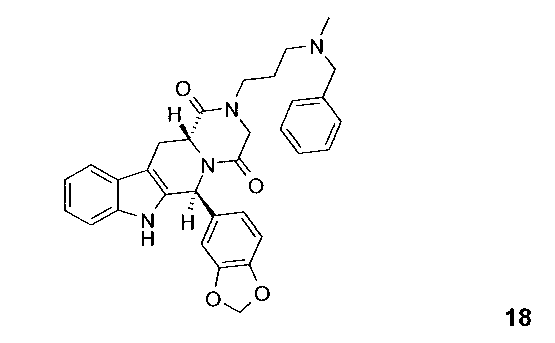

Compound **18** was prepared according to general protocol 3 starting from intermediate **4** as obtained her-above at step 2 of Example 3 (86 mg), Compound **22** as above obtained at step 1) b) of Example 6 (4 eq.) and TEA (12 eq.) in MeOH (48 h). Compound **18** was obtained after purification by flash chromatography (CH₂Cl₂/MeOH) as a yellow powder (70 mg, 65%).
**LC :** t_{R}=4.7 min;
**MS** (ESI-) : m/z = 537 (M-H);
**¹H NMR** (MeOD) : δ 7.51 (d, *J*=7.5 Hz, 1 H), 7.24-7.34 (m, 5H), 7.05-7.18 (m, 3H), 6.95 (s, 1 H), 6.80 (d, *J*=1.5 Hz, 1 H), 6.76 (d, *J*=8.1 Hz, 1 H), 6.66 (dd, *J*=8.1 Hz and *J*=1.5 Hz,1H), 5.93 (d, *J*=2.4 Hz, 2H), 4.19-4.32 (m, 2H), 4.08 (d, *J*=17.4 Hz, 1H), 3.48-3.60 (m, 4H), 3.36 (m, 1H), 2.86 (ddd, *J*=15.0 Hz, *J*=11.7 Hz and *J*=1.2 Hz, 1H), 2.41 (m, 2H), 2.24 (s, 3H), 1.84 (qt, J=7.2 Hz, 2H).

### EXAMPLE 8: Synthesis of (6R,12aR)-6-Benzo[1,3]dioxol-5-yl-2-[(R)-1-(4-dimethylamino-benzyl)-pyrrolidin-3-yl]-2,3,6,7,12,12a-hexahydro-pyrazino[1',2':1,6]pyrido[3,4-b]indole-1,4-dione (Compound 26)

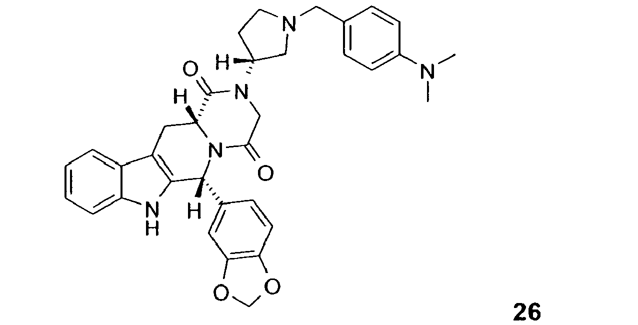

**1) First Step:** Synthesis of the intermediate (6R,12aR)-6-Benzo[1,3]dioxol-5-yl-2-(R)-pyrrolidin-3-yl-2,3,6,7,12,12a-hexahydro-pyrazino[1',2':1,6]pyrido[3,4-*b*]indole-1,4-dione (Compound **23)** 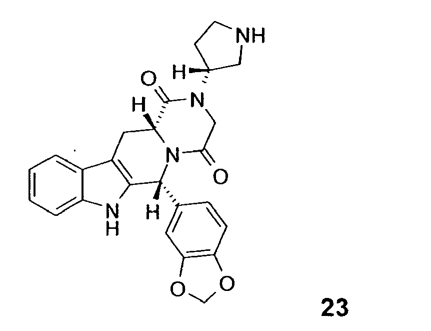 Compound **6** as above obtained at the end of Example 1 (720 mg, 1 eq.) was dissolved in ethyl acetate (0.07 M), and palladium dihydroxide (Pd(OH)₂) on carbon (1 eq. w/w) was suspended. The reaction mixture was stirred at room temperature under H₂ atmosphere for 24-48 h. The reaction mixture was filtered on celite, evaporated to dryness and precipitated in diethyl ether to obtain compound **23** as a white powder (412 mg, 69%).
   **LC :** t_{R}=4.0 min;
   **MS** (ESI+) : m/z = 445 (M+H)⁺;
   **¹H NMR** (DMSO-*d₆*) : δ 11.12 (s, 1H), 8.31 (s, 1H), 7.54 (d, J=7.8 Hz, 1H), 7.30 (d, *J*=7.8 Hz, 1H), 7.08-6.97 (m, 2H), 6.83-6.72 (m, 3H), 6.17 (s, 1 H), 5.92 (s, 1 H), 4.92 (m, 1 H), 4.43 (dd, *J*=11.1 Hz and 4.8 Hz, 1 H), 4.17 (d, *J*=16.6 Hz, 1H), 4.00 (d, *J*=16.8 Hz, 1 H), 3.47 (dd, *J*=15.8 Hz and *J*=4.9 Hz, 1H), 3.14-2.88 (m, 5H), 2.08-2.03 (m, 1H), 1.75-1.66 (m, 1 H).
**2)** **Second Step**: Synthesis of Compoud **26**

**26** was prepared according to general protocol 4 starting from intermediate **23** as above obtained at step 1 (45 mg) and p-dimethylaminobenzaldehyde (1.5 eq.). Compound **26** was obtained after purification by TLC (CH₂Cl₂/MeOH) as a white powder (16 mg, 27%).
**LC:** t_{R}=5.14 min;
**MS** (ESI+) : m/z = 578 (M+H)⁺;
**¹H NMR** (MeOD) : δ 7.52 (d, *J*=6.9 Hz, 1 H), 7.28 (d, *J*=7.5 Hz, 1 H), 7.20 (d, *J*=8.7 Hz, 1 H), 7.06 (m, 2H), 6.66-6.79 (m, 5H), 6.24 (s, 1H), 5.85 (s, 2H), 5.04 (brs, 1H), 4.34 (dd, *J*=11.7 Hz and *J*=5.4 Hz, 1H), 4.31 (d, J=17.1 Hz, 1H), 4.09 (dd, *J*=17.1 Hz and *J*=1.2 Hz, 1H), 3.66 (d, *J*=12.3 Hz, 1 H), 3.58 (dd, *J*=15.6 Hz and *J*=4.8 Hz, 1 H), 3.46 (d, *J*=12.6 Hz, 1 H), 3.12 (dd, *J*=15.9 Hz and *J*=11.7 Hz, 1 H), 2.97 (m, 1 H), 2.90 (s, 6H), 2.75 (dd, *J*=10.8 Hz and *J*= 4.2 Hz, 1 H), 2.54 (dd, *J*=10.5 Hz and *J*=7.8 Hz, 1 H), 2.25-2.38 (m, 2H), 1.19-1.72 (m, 1H).

### EXAMPLE 9: Synthesis of (6R,12aR)-6-Benzo[1,3]dioxol-5-yl-2-((R)-1-pyridin-2-ylmethyl-pyrrolidin-3-yl)-2,3,6,7,12,12a-hexahydro-pyrazino [1',2':1,6]pyrido[3,4-b]indole-1,4-dione (Compound 27)

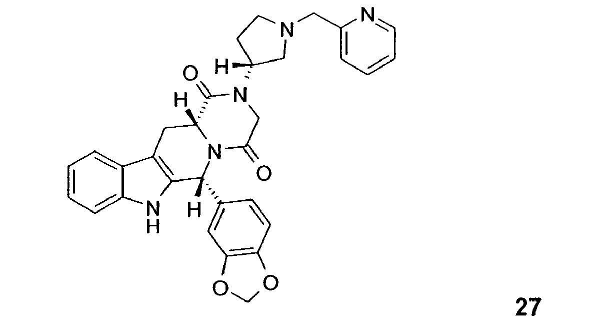

**27** was prepared according to general protocol 4 starting from intermediate **23** as above obtained at step 1 of Example 8 (45 mg) and pyridine-2-carboxaldehyde (1.5 eq.). Compound **27** was obtained after purification by TLC (CH₂Cl₂/MeOH) as a white powder (27 mg, 50%).
**LC:** t_{R} =4.58 min;
**MS** (ESI+) : m/z = 536 (M+H)⁺;
**¹H NMR** (MeOD) : δ 8.53 (d, *J*= 4.8 Hz, 1H), 7.86 (td, *J*=7.8 Hz and *J*=1.8 Hz, 1 H), 7.55 (m, 2H), 7.36 (dd, *J*=6.6 Hz and *J*=5.1 Hz, 1 H), 7.29 (d, *J*=7.5 Hz, 1 H), 7.06 (m, 2H), 6.78-6.81 (m, 2H), 6.68 (d, *J*=8.7 Hz, 1 H), 6.25 (s, 1 H), 5.85 (s, 2H), 5.01 (brs, 1 H), 4.39 (dd, *J*=11.7 Hz and *J*=5.1 Hz, 1H), 4.3 (d, *J*=17.1 Hz, 1H), 4.19 (d, *J*= 17.7 Hz, 1H), 3.92-4.03 (m, 2H), 3.61 (dd, *J*=15.6 Hz and *J*=4.8 Hz, 1H), 3.07-3.25 (m, 3H), 2.78-2.84 (m, 1H), 2.54-2.62 (m, 1H), 2.32-2.43 (m, 1 H), 1.89-1.84 (m, 1 H).

### EXAMPLE 10: Synthesis of (6R,12aR)-6-Benzo[1,3]dioxol-5-yl-2-[(R)-1-(1-methyl-1H-imidazol-2-ylmethyl)-pyrrolidin-3-yl]-2,3,6,7,12,12a-hexahydro-pyrazino[1',2':1,6]pyrido[3,4-b]indole-1,4-dione (Compound 28)

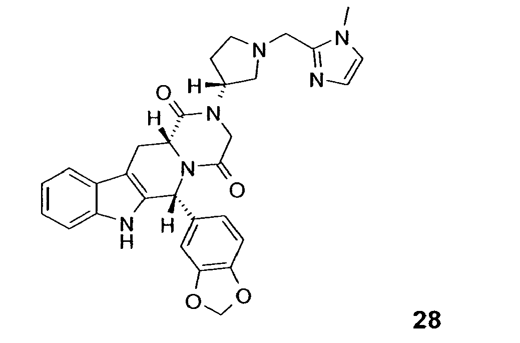

**28** was prepared according to general protocol 4 starting from intermediate **23** as above obtained at step **1** of Example 8 (45 mg) and 1-methyl-2-imidazolecarboxaldehyde (1.5 eq.). Compound **28** was obtained after purification by TLC (CH₂Cl₂/MeOH) as a white powder (26 mg, 48%).
**LC:** t_{R} =4.2 min;
**MS** (ESI+) : m/z = 539 (M+H)⁺;
**¹H NMR** (MeOD) : δ 7.53 (d, *J*=6.9 Hz, 1 H), 7.29 (d, *J*=7.5 Hz, 1 H), 7.01-7.12 (m, 3H), 6.90 (d, *J*=1.2 Hz, 1H), 6.75-6.79 (m, 2H), 6.67-6.69 (m, 1H), 6.25 (s, 1H), 5.85 (s, 2H), 5.15 (brs, 1H), 4.37 (dd, *J*=11.4 Hz and *J*=4.5 Hz, 1H), 4.26 (d, *J*=17.1 Hz, 1H), 4.10 (d, *J*=17.1 Hz, 1H), 3.78 (s, 3H), 3.73 (d, *J*=2.4 Hz, 2H), 3.59 (dd, *J*=15.6 Hz and *J*=5.1 Hz, 1H), 3.12 (dd, *J*=15.6 Hz and *J*=11.7 Hz, 1H), 2.93 (m, 1H), 2.80 (dd, *J*=10.8 Hz and *J*=2.1 Hz, 1 H), 2.50 (dd, *J*=10.5 Hz and *J*=7.5 Hz, 1 H), 2.28 (m, 2H), 1.62-1.67 (m, 1H).

### EXAMPLE 11: Synthesis of (6S,12aR)-6-Benzo[1,3]dioxol-5-yl-2-[(R)-1-(1-methyl-1H-imidazol-2-ylmethyl)-pyrrolidin-3-yl]-2,3,6,7,12,12a-hexahydro-pyrazino[1',2':1,6]pyrido[3,4-b]indole-1,4-dione (Compound 29)

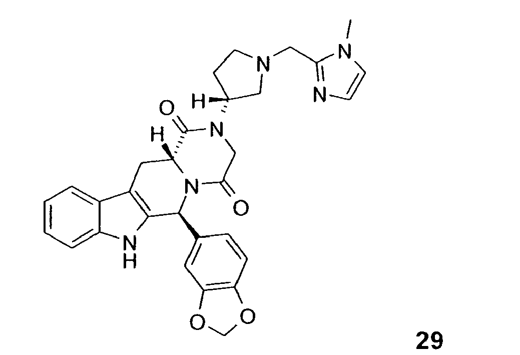

**1) First Step:** Synthesis of the intermediate (6S,12aR)-6-Benzo[1,3]dioxol-5-yl-2-(R)-pyrrolidin-3-yl-2,3,6,7,12,12a-hexahydro-pyrazino[1',2':1,6]pyrido[3,4-b]indole-1,4-dione (Compound **24)** 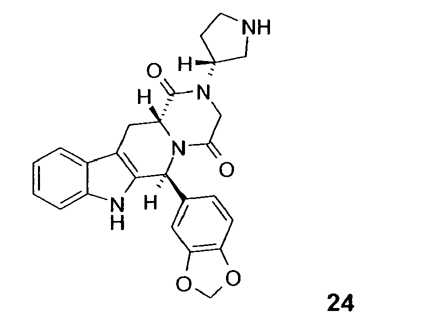 Compound **14** as obtained above at the end of Example 3 (235 mg) was dissolved in ethyl acetate (0.07 M), and Pd(OH)₂ on carbon (1 eq. w/w) was suspended. The reaction mixture was stirred at room temperature under H₂ atmosphere for 24-48 h. The reaction mixture was filtered on celite, evaporated to dryness and precipitated in diethyl ether to obtain compound **24** as a white powder.
   **LC :** t_{R}=4.0 min;
   **MS** (ESI+) : m/z = 445 (M+H)⁺.
**2)** **Second Step**: Synthesis of compound **29**
   **29** was prepared according to general protocol 4 starting from intermediate **24** as above obtained at step 1 (45 mg) and 1-methyl-2-imidazolecarboxaldehyde (1.5 eq.). Compound **29** was obtained after purification by TLC (CH₂Cl₂/MeOH) as a white powder (15 mg, 28%).
   **LC:** t_{R} =4.36 min;
   **MS** (ESI+) : m/z = 539 (M+H)⁺;
   **¹H NMR** (DMSO-*d₆*) : δ 11,07 (s, 1H), 7.50 (d, *J*=8,1 Hz, 1H), 7.31 (d, *J*=8,1 Hz, 1H), 7.08 (m, 3H), 6.87 (d, *J*=8,1 Hz, 1 H), 6.76 (m, 3H), 6.60 (d, *J*=8,1 Hz, 1H), 6.01 (m, 2H), 4,98 (brs, 1 H), 4,27 (d, *J*=17,4 Hz, 1 H), 4.07 (m, 2H), 3.66 (m, 4H), 3.54 (d, *J*=13,5 Hz, 1H), 3.21 (dd, *J*=15.3 Hz and *J*=4,2 Hz, 1H), 2,97 (dd, *J*=14,4 Hz and *J*=12,9 Hz, 1H), 2,78 (m, 2H), 2.38 (m, 1 H), 2.17-2,03 (m, 2H), 1,80 (m, 1 H).

### EXAMPLE 12: Synthesis of (6R,12aR)-6-Benzo[1,3]dioxol-5-yl-2-[(R)-1-(4-methoxy-benzyl)-pyrrolidin-3-yl]-2,3,6,7,12,12a-hexahydro-pyrazino[1',2':1,6]pyrido[3,4-b]indole-1,4-dione (Compoud 31)

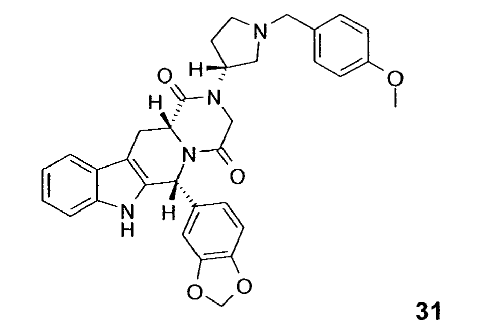

**31** was prepared according to general protocol 4 starting from intermediate **23** as above obtained at step 1 of Example 8 (50 mg) and para-anisaldehyde (2.0 eq.). Compound **31** was obtained after purification by TLC (CH₂Cl₂/MeOH) as a white powder (20 mg, 31%).
**LC:** t_{R}=5.0 min;
**MS** (ESI+) : m/z = 565 (M+H)⁺;
**¹H NMR** (MeOD) : δ 7.52 (d, *J*=7.2 Hz, 1H), 7.29 (d, *J*=8.7 Hz, 3H), 7.06 (m, 2H), 6.89 (d, *J*=8.7 Hz, 2H), 6.66-6.79 (m, 3H), 6.24 (s, 1H), 5.85 (s, 2H), 5.01 (m, 1 H), 4.34 (dd, *J*=11.1 Hz and *J*=4.8 Hz, 1H), 4.30 (d, J=17.1 Hz, 1 H), 4.10 (d, *J*=17.1 Hz, 1 H), 3.77 (s, 3H), 3.73 (d, *J*=12.6 Hz, 1 H), 3.59 (dd, *J*=15.9 Hz and *J*=4.8 Hz, 1H), 3.56 (d, *J*=12.6 Hz, 1H), 3.12 (dd, *J*=15.6 Hz and *J*=11.7 Hz, 1H), 3.03 (td, *J*=8.4 Hz and *J*=2.7 Hz, 1H), 2.83 (dd, *J*=11.1 Hz and *J*=3.3 Hz, 1H), 2.59 (dd, *J*=10.8 Hz and *J*=7.8 Hz, 1 H), 2.24-2.45 (m, 2H), 1.68-1.80 (m, 1 H).

### EXAMPLE 13: Synthesis of (6R,12aR)-6-Benzo[1,3]dioxol-5-yl-2-((R)-1-benzo[1,3]dioxol-5-ylmethyl-pyrrolidin-3-yl)-2,3,6,7,12,12a-hexahydro-pyrazino[1',2':1,6]pyrido[3,4-b]indole-1,4-dione (Compound 33)

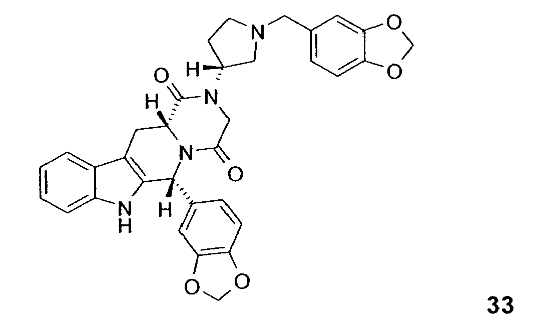

**33** was prepared according to general protocol 4 starting from intermediate **23** as above obtained at step 1 of Example 8 (45 mg) and Piperonal (1.2 eq.). Compound **33** was obtained after purification by TLC (CH₂Cl₂/MeOH) as a white powder (18 mg, 31%).
**LC :** t_{R}=4.8 min;
**MS** (ESI+) : m/z = 579 (M+H)⁺;
**¹H NMR** (MeOD) : δ 5.53 (d, *J*=6.9 Hz, 1H), 7.29 (d, *J*=7.5 Hz, 1H), 7.06 (m, 2H), 6.90 (d, *J*=1.2 Hz, 1H), 6.74-6.84 (m, 4H), 6.68 (m, 1 H), 6.25 (s, 1H), 5.92 (s, 2H), 5.85 (s, 2H), 5.05 (m, 1H), 4.37 (dd, *J*=11.7 Hz and *J*=4.2 Hz, 1 H), 4.31 (d, *J*=16.8 Hz, 1 H), 4.14 (d, *J*=15.9 Hz, 1H), 3.69 (d, *J*=12.6 Hz, 1H), 3.51-3.66 (m, 3H), 3.00-3.17 (m, 2H), 2.83 (dd, *J*=10.8 Hz and *J*=3.3 Hz, 1H), 2.58 (dd, *J*=10.8 Hz and *J*=7.8 Hz, 1H), 2.27-2.40 (m, 2H), 1.70-1.76 (m, 1 H).

### EXAMPLE 15: Synthesis of {2-[(R)-3-((6R,12aR)-6-Benzo[1,3]dioxol-5-yl-1,4-dioxo-3,4,6,7,12,12a-hexahydro-1H-pyrazino[1',2':1,6]pyrido[3,4-b] indol-2-yl)-pyrrolidin-1-yl]-ethyl)-carbamic acid tert-butyl ester Compound 35)

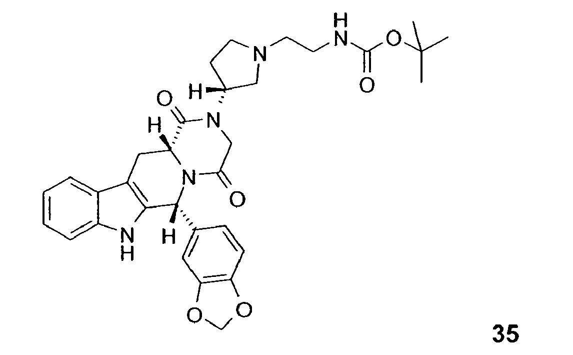

Compound **23** as above obtained at step 1 of Example 8 (50 mg), 2-(Boc-amino)ethyl bromide (25 mg, 1 eq.) and triethylamine (1.1 eq.) were dissolved in dioxane (1 ml) and stirred at room temperature. 46 hours later, 0.5 eq. of 2-(Boc-amino)ethyl bromide, 0.5 eq. of TEA and 0.5 ml of dioxane were added. 96 hours later, the reaction mixture was heated to 70°C for 20 hours. The dioxane was removed by evaporation; the residue was dissolved in ethyl acetate, washed with saturated NaHCO₃ aqueous solution, dried over MgSO₄ and evaporated to dryness. Compound **35** was purified by TLC (CH₂Cl₂/MeOH) to give 29 mg of a white powder (44%).
**LC :** t_{R} = 4.94 min;
**MS** (ESI+) : m/z = 588 (M+H)⁺;
**¹H NMR** (MeOD) : δ 7.53 (d, *J*=7.2 Hz, 1 H), 7.29 (d, *J*=7.2 Hz, 1 H), 7.07 (m, 2H,), 6.68-6.80 (m, 3H), 6.27 (s, 1 H), 5.86 (s, 2H), 5.10 (m, 1H), 4.41 (dd, *J*=11.4 Hz and *J*=4.5 Hz, 1H), 4.31 (d, *J*=17.4 Hz, 1H), 4.18 (d, J=17.1 Hz, 1H), 3.61 (dd, *J*=15.6 Hz and *J*=4.8 Hz, 1H), 2.87-3.23 (m, 5H), 2.50-2.63 (m, 3H), 2.23-2.32 (m, 2H), 1.65-1.71 (m, 1 H), 1.44 (s, 9H).

### EXAMPLE 16: Synthesis of (6R,12aR)-6-Benzo[1,2,5]thiadiazol-5-yl-2-((R)-1-benzyl-pyrrolidin-3-yl)-2,3,6,7,12,12a-hexahydro-pyrazino[1',2':1,6] pyrido[3,4-b]indole-1,4-dione (Compound 70c)

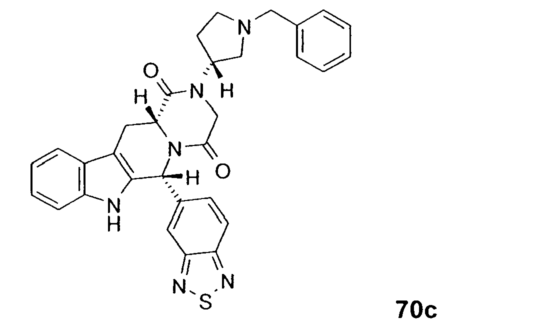

**1) First Step:** Synthesis of (1R,3R)-1-Benzo[1,2,5]thiadiazol-5-yl-3-methoxycarbonyl-2,3,4,9-tetrahydro-1H-β-carbolin-2-ium trifluoroacetate (Compound 64c) 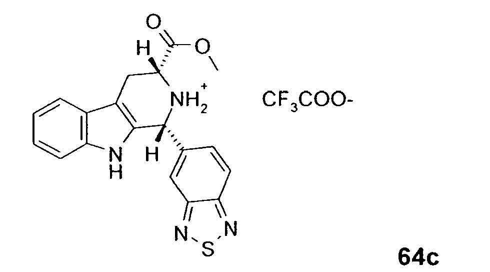 64c was prepared according to general protocol 1 starting from D-tryptophan-OMe.HCl (1241 mg) and 2,1,3-benzothiadiazole-5-carbaldehyde (800 mg). The reaction mixture was stirred overnight. After filtration and concentration, the cis isomer was precipitated in EtOAc to provide the compound 64c as a yellow solid (1180 mg, 66%).
   **LC :** t_{R}= 3.91 min;
   **MS** (ESI+) : m/z= 365 (M+H)⁺;
   **¹H NMR** (DMSO-*d₆*): δ 10.85 (s, 1 H), 8.31 (s, 1 H), 8.20 (d, J=9.0 Hz, 1H), 7.78 (dd, *J*=9.3 Hz and 1.5 Hz, 1H), 7.58 (d, *J*=7.5 Hz, 1H), 7.24 (d, *J*=7.5 Hz, 1 H), 7.14-7.04 (m, 2H), 6.21 (s, 1H), 4.85 (m, 1 H),) 3.86 (s, 3H), 3.42 (m, 2H);
   Mp=237-238°C.
**2)** **Second** Step: Synthesis of (1R,3R)-1-Benzo[1,2,5]thiadiazol-5-yl-2-(2-chloro-acetyl)-2,3,4,9-tetrahydro-1H-β-carboline-3-carboxylic acid methyl ester (Compound **67c**) 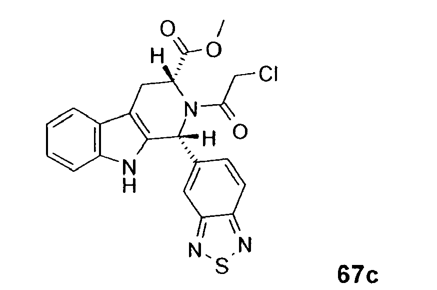 **67c** was prepared according to general protocol 2 starting from compound **64c** obtained at step 1 above (200 mg, 1 eq.). Compound **67c** was obtained as a yellow powder (205 mg, 84%) which was directly engaged in the cyclisation step.
   **LC :** t_{R}= 6.2 min;
   **MS** (ESI+) : m/z=439 (M-H)
3) Third Step: Synthesis of Compound **70c**

Compound **70c** was prepared according to general protocol 4 starting from compound **67c** as obtained above at step 2 (185 mg, 0.42 mmol) and (R)-(-)-1-Benzyl-3-aminopyrrolidine (145 µl, 2 eq.) in MeOH. Compound **70c** was obtained after purification by TLC (CH₂Cl₂/MeOH 95:5 v/v) as a yellow powder (120 mg, 44%).
**LC:** t_{R}= 5.03 min;
**MS** (ESI+) : m/z= 549 (M+H)⁺;
**¹H NMR** (DMSO-*d₆*): δ 11.2 (s, 1H), ) 8.01 (s, 1 H), 7.97 (d, J=9.30 Hz, 1 H), 7.66 (dd, *J*=9.0 Hz and 1.5 Hz, 1 H), 7.56 (d, *J*=7.20 Hz, 1 H), 7.38-7.35 (m, 5H), 7.28 (d, *J*=7.80 Hz, 1H), 7.05 (td, *J*=7.50 Hz and 0.9 Hz, 1 H), 7.02 (td, *J*=7.80 Hz and 1.2 Hz, 1 H), 6.39 (s, 1H), 5.07 (m, 1H), 4.52 (dd, J=11.4 Hz and 4.2 Hz, 1 H), 4.17 (s, 2H), 3.55 (dd, *J*=15.9 Hz and 4.5 Hz, 2H), 3.13 (dd, *J*=15.3 Hz and 11.7 Hz, 2H), 3.12-3.01 (m, 1H), 2.96-2.80 (m, 1H), 2.75 (m, 1H), 2.23 (m, 2H), 2.02-1.90 (m, 1 H).

### EXAMPLE 17: Synthesis of (6S,12aR)-6-Benzo[1,2,5]thiadiazol-5-yl-2-((R)-1-benzyl-pyrrolidin-3-yl)-2,3,6,7,12,12a-hexahydro-pyrazino[1',2':1,6] pyrido[3,4-b]indole-1,4-dione (Compound 70t)

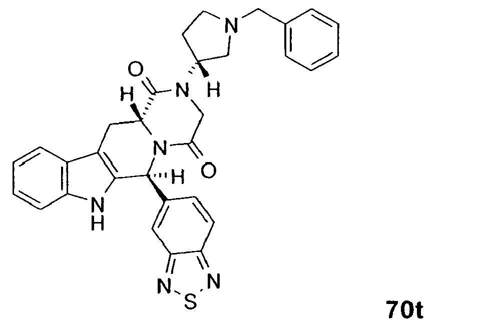

**1) First Step:** Synthesis of (1S,3R)-1-Benzo[1,2,5]thiadiazol-5-yl-2,3,4,9-tetrahydro-1*H*-β-carboline-3-carboxylic acid methyl ester (Compound **64t**) 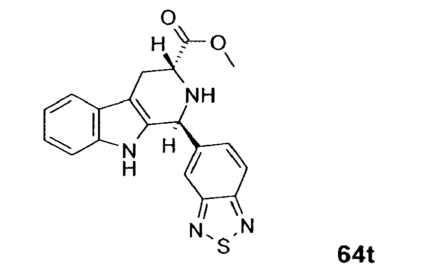 **64t** was prepared according to general protocol 1 starting from D-tryptophan-OMe.HCl (1241 mg) and 2,1,3-benzothiadiazole-5-carbaldehyde (800 mg). The reaction mixture was stirred overnight. After filtration and concentration, the cis isomer **64c** was separated by precipitation in EtOAc and the residue was purified by column chromatography (CH₂Cl₂/MeOH) to provide the *trans* compound **64t** as a orange solid (160 mg, 10%)
   **LC :** t_{R}= 4.22 min;
   **MS** (ESI+) : m/z= 365 (M+H)⁺;
   **¹H NMR** (DMSO-*d₆*): δ 10.65 (s, 1 H), 8.03 (d, *J*=9.3 Hz, 1 H), 7.80 (s, 1H), 7.70 (dd, *J*=9.3 Hz and *J*=1.8 Hz, 1H), 7.48 (d, *J*=7.5 Hz, 1H), 7.22 (d, *J*=7.5 Hz, 1H), 7.06-6.95 (m, 2H), 5.57 (s, 1H), 3.89 (m, 1H), 3.62 (s, 3H), 3.07 (m, 2H);
   **Mp**=156-160°C.
**2) Second Step:** Synthesis of (1S,3R)-1-Benzo[1,2,5]thiadiazol-5-yl-2-(2-chloro-acetyl)-2,3,4,9-tetrahydro-1H-β-carboline-3-carboxylic acid methyl ester (Compound **67t**) 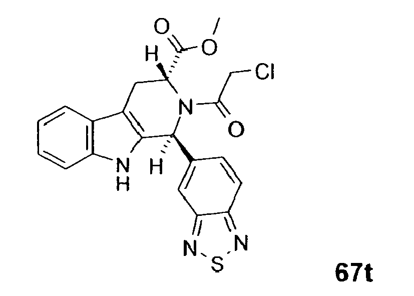 **67t** was prepared according to general protocol 2 starting from compound **64t** as obtained above at step 1 (160 mg, 1 eq.) and obtained as a yellow oil (190 mg, 97%) which was directly engaged in the cyclisation step.
   **LC :** t_{R}= 6.2 min;
   **MS** (ESI+) : m/z=441 (M+H)⁺.
**3) Third Step:** Synthesis of Compound 70t

Compound **70t** was prepared according to general protocol 4 starting from compound **67t** as obtained above at step 2 (190 mg, 0.43 mmol) and (R)-(-)-1-Benzyl-3-aminopyrrolidine (148 µl, 2 eq.) in MeOH. Compound **70t** was obtained after purification by TLC (CH₂Cl₂/MeOH 95:5 v/v) as a yellow powder (147 g, 60%).
**LC:** t_{R}= 5.05 min;
**MS** (ESI+) : m/z= 549 (M+H)⁺;
**¹H NMR** (DMSO-*d₆*): 6 11.15 (s, 1H), 8.10 (d, *J*=9.6 Hz, 1H), 7.58 (dd, *J*=9.3 Hz and 1.5 Hz, 1H), 7.64 (s, 1H), 7.56 (d, *J*=7.5 Hz, 1H), 7.36 (d, *J*=8.1 Hz, 1H), 7.33-7.22 (m, 5H), 7.14 (td, *J*=7.20 Hz and 1.2 Hz, 1H), 7.08 (s, 1 H), 7.05 (td, *J*=7.50 Hz and 0.9 Hz, 1 H), 5.03-4.95 (m, 1 H), 4.39 (d, *J*=17.7 Hz, 1H), 4.15 (d, *J*=17.4 Hz, 1H), 4.14 (dd, *J*=12.3 Hz and 4.5 Hz, 1H), 3.58 (d, *J*=12.9 Hz, 1 H), 3.51 (d, *J*=12.9 Hz, 1 H), 3.41 (dd, *J*=15.6 Hz and 4.2 Hz, 1H), 3.06 (dd, *J*=15.0 Hz and 12.3 Hz) 2.86-2.82 (m, 1 H), 2.72 (dd, *J*=10.2 Hz and 2.7 Hz, 1H), 2.35 (dd, *J*= 10.2 Hz and 7.8 Hz, 1H), 2.20-2.01 (m, 2H), 1.91-1.74 (m, 1H).

### EXAMPLE 18: Synthesis of (6R,12aR)-2-(1-Benzyl-piperidin-4-yl)-6-(2,2-difluoro-benzo[1,3]dioxol-5-yl)-2,3,6,7,12,12a-hexahydro-pyrazino-[1',2':1,6]pyrido[3,4-b]indole-1,4-dione (Compound 77)

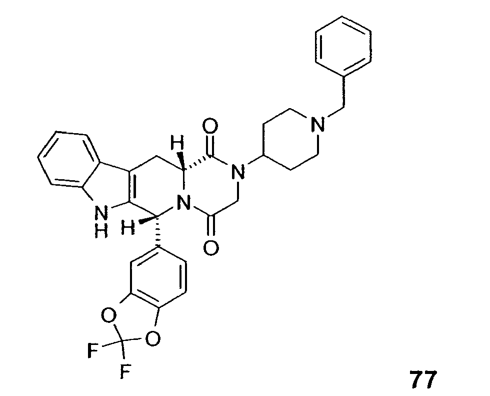

**1) First Step:** Synthesis of (1R,3R)-1-(2,2-Difluoro-benzo[1,3]dioxol-5-yl)-2,3,4,9-tetrahydro-1*H*-β-carboline-3-carboxylic acid methyl ester (Compound 73c) 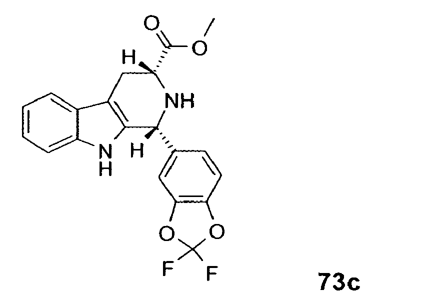 **73c** was prepared according to general protocol 1 starting from D-tryptophan-OMe.HCl (1.03 g) and 2,2-difluoro-5-formylbenzodioxole (930 mg). The reaction mixture was stirred for 17 hours. Compound **73c** was obtained after purification by column chromatography, as a white powder (862 mg, 56%).
   **LC :** t_{R}=4.6 min;
   **MS** (ESI+) : m/z=387 (M+H)⁺;
   **¹H NMR** (DMSO-*d₆*), δ 10.45 (s, 1H), 7.45 (m, 2H), 7.36 (d, J=1.5 Hz, 1H), 7.25 (Dd, *J*=1.5 Hz and *J*=8.3 Hz, 1H), 7.21 (d, *J*=7.5 Hz, 1H), 7.02 (td, *J*=1.5 Hz and *J*=15 Hz, 1H), 6.95 (td, *J*= 1.1 Hz and *J*= 14.5 Hz, 1H), 5.42 (s, 1H), 4.09 (m, 1 H), 3.75 (s, 3H), 3.10 (m, 1 H), 2.93 (m, 1H);
   **Mp** = 124-126°C.
**2) Second Step:** Synthesis of (1R,3R)-2-(2-Chloro-acetyl)-1-(2,2-difluoro-benzo[1,3]dioxol-5-yl)-2,3,4,9-tetrahydro-1*H*-β-carboline-3-carboxylic acid methyl ester (Compound 75) 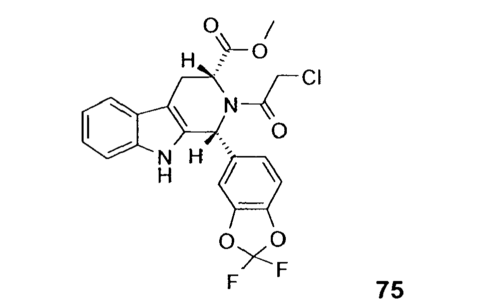 75 was prepared according to general protocol 2 starting from compound 73c as obtained above at step 1 (662 mg, 1 eq.). Compound 75 was obtained as an orange powder (783 mg, 100%) which was directly engaged in the cyclisation step.
   **LC :** t_{R}=7.1 min;
   **MS** (ESI+) : m/z=383 (M+H)⁺.
3) Third Step: Synthesis of Compound 77

Compound 77 was prepared according to general protocol 4 starting from compound 75 as obtained above at step 2 (100 mg, 1 eq.), triethylamine (4 eq.) and 4-amino-N-benzylpiperidine (1.5 eq.) in EtOH. Compound **77** was obtained after purification by TLC (CH₂Cl₂/MeOH) as a white powder (51 mg, 34%).
**LC :** t_{R}=5.5 min;
**MS** (ESI+) : m/z= 585 (M+H)⁺;
**¹H NMR** (DMSO-*d₆*) ppm: δ 11.03 (s, 1H), 7.54 (d, *J*= 7.7 Hz, 1H), 7.33-7.24 (m, 8H), 7.15 (dd, *J*= 9.9 Hz and 1.5 Hz, 1H), 7.08-6.97 (m, 2H), 6.19 (s, 1 H), 4.44 (dd, *J*= 11.7 Hz and 4.3 Hz, 1 H), 4.26 (m, 1 H), 4.03 (d, *J*= 17.0 Hz, 1H), 3.92 (d, *J*= 16.8 Hz, 1H), 3.47 (m, 3H), 3.04 (dd, *J*= 15.4 Hz and 11.5 Hz, 1 H), 2.88 (m, 2H), 2.03 (m, 2H), 1.82-1.46 (m, 4H).

### EXAMPLE 19: Synthesis of (6R,12aR)-2-((R)-1-Benzyl-pyrrolidin-3-yl)-6-(6-chloro-pyridin-3-yl)-2,3,6,7,12,12a-hexahydro-pyrazino[1',2':1,6] pyrido[3,4-b]indole-1,4-dione (Compound 84c)

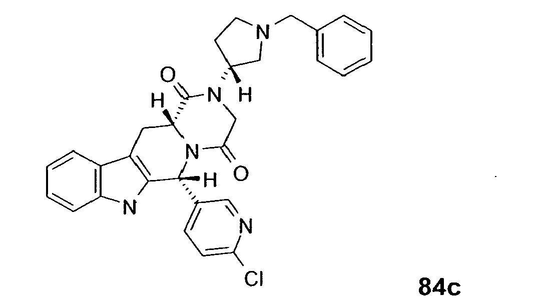

**1) First Step:** Synthesis of (1R,3R)-1-(6-Chloro-pyridin-3-yl)-2,3,4,9-tetrahydro-1*H*-β-carboline-3-carboxylic acid methyl ester (Compound **81c)** 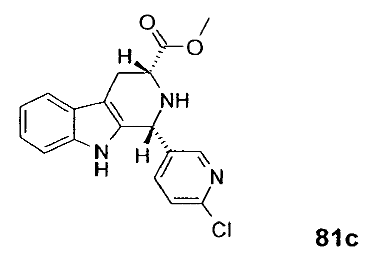 **81c** was prepared according to general protocol 1 starting from D-tryptophan-OMe.HCl (1.02 g) and 6-chloropyridine-3-carboxaldehyde (708 mg), after stirring for 67 hours and purification by column chromatography. Compound 81c was obtained as a white powder (937 mg, 69%).
   **LC:** t_{R}=3.8 min;
   **MS** (ESI+) : m/z=342 (M+H)⁺;
   **¹H NMR** (DMSO-*d₆*): δ 10.45 (s, 1H), 8.42 (d, *J*=2.1 Hz, 1H), 7.72 (dd, *J*=8.3 Hz and *J*= 2.5 Hz, 1 H), 7.50 (d, *J*=8.5 Hz, 1H), 7.44 (d, *J*=7.0 Hz, 1H), 7.13 (m, 1 H), 6.98 (m, 2H), 5.3 (d, *J*=5.5 Hz, 1H), 3.89 (m, 1 H), 3.71 (s, 3H), 3.21 (t, *J*=5.5 Hz, 1 H), 3.05 (dd, *J*=14.9 Hz and *J*=3.1 Hz, 1 H), 2.85 (m, 1H);
   **Mp** = 190-192°C.
**2) Second Step:** Synthesis of (1R,3R)-2-(2-Chloro-acetyl)-1-(6-chloro-pyridin-3-yl)-2,3,4,9-tetrahydro-1*H*-β-carboline-3-carboxylic acid methyl ester (Compound **83c)** 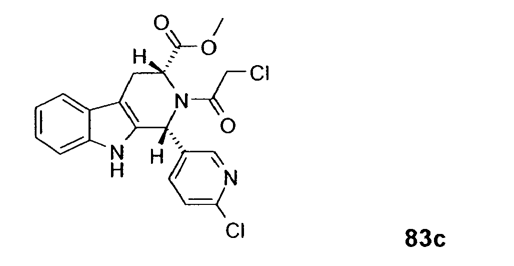 **83c** was prepared according to general protocol 2 starting from compound **81c** as obtained above at step 1 (681 mg, 1 eq.). Compound **83c** was obtained as a white powder (667 mg, 80%) which was directly engaged in the cyclisation step.
   **LC :** t_{R}= 6.0 min;
   **MS** (ESI+) : m/z=420 (M+H)⁺.
3) Third Step: Synthesis of Compound 84c

Compound **84c** was prepared according to general protocol 4 starting from compound **83c** as obtained above at step 2 (0.44 mmol), triethylamine (123 µl, 2 eq.) and (R)-(-)-1-Benzyl-3-aminopyrrolidine (152 µl, 2 eq.) in MeOH. Compound **84c** was obtained after purification by TLC (CH₂Cl₂/MeOH 95:5 v/v) as a white powder (100 mg, 43%).
**LC:** t_{R}= 4.69 min;
**MS** (ESI+) : m/z= 526 (M+H)⁺;
**¹H NMR** (MeOD): δ 8.4 (d, *J*=2.4 Hz, 1H), 7.63 (dd, *J*=2.4 Hz and 8.4 Hz, 1H), 7.55 (d, *J*=7.20 Hz, 1H), 7.37-7.22 (m, 7H), 7.10 (td, *J*=7.50 Hz and 1.2 Hz, 1H), 7.04 (td, *J*=7.20 Hz and 1.2 Hz, 1H), 6.27 (s, 1H), 5.12 (m, 1H), 4.40 (dd, *J*=11.4 Hz and 4.8 Hz, 1H), 4.32 (d, *J*=17.7 Hz, 1H), 4.12 (dd, *J*=17.4 Hz and 1.5 Hz, 1H), 3.70 (d, *J*=12.9 Hz, 1H), 3.67 (dd, *J*=15.9 Hz and 4.5 Hz, 1 H), 3.54 (d, *J*=12.6 Hz, 1H), 3.15 (dd, *J*=15.9 Hz and 11.4 Hz, 1H), 2.94 (m, 1 H), 2.75 (dd, *J*=10.5 Hz and 3.3 Hz, 1 H), 2.48 (dd, *J*=10.5 Hz and 7.8 Hz, 1H), 2.34-2.20 (m, 2H), 1.74-1.65 (m, 1H).

### EXAMPLE 20: Synthesis of (6S,12aR)-2-((R)-1-Benzyl-pyrrolidin-3-yl)-6-(6-chloro-pyridin-3-yl)-2,3,6,7,12,12a-hexahydro-pyrazino[1',2':1,6] pyrido[3,4-b]indole-1,4-dione (Compound 84t)

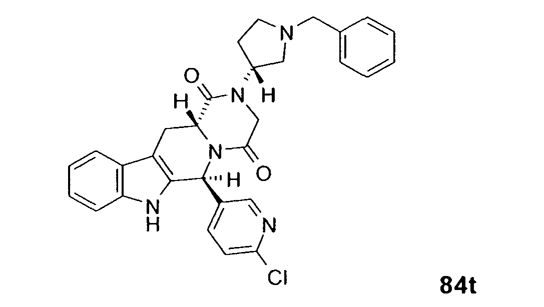

**1) First Step:** Synthesis of (1S,3R)-1-(6-Chloro-pyridin-3-yl)-2,3,4,9-tetrahydro-1*H*-β-carboline-3-carboxylic acid methyl ester (Compound **81t)** 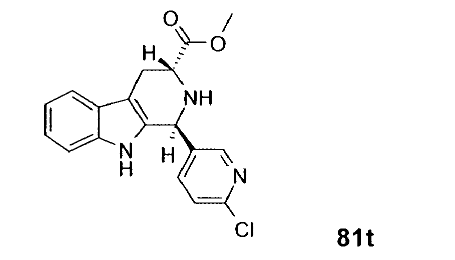 **81t was** prepared according to general protocol 1 starting from D-tryptophan-OMe.HCl (1.02 g) and 6-chloropyridine-3-carboxaldehyde (708 mg), after stirring for 67 hours and purification by column chromatography. Compound **81t was** obtained as a white powder (262 mg, 19%).
   **LC:** t_{R}=3.9 min;
   **MS** (ESI+): m/z=342 (M+H)⁺;
   **¹H NMR** (DMSO-*d₆*), δ: 10.50 (s, 1 H), 8.35 (d, *J*=2.3 Hz, 1H), 7.67 (dd, *J*=2.4 Hz and *J*=8.2 Hz, 1H), 7.44 (m, 2H), 7.22 (d, *J*=7.9 Hz, 1H), 7.02 (td, *J*=1.4 Hz and *J*=8.2 Hz, 1H), 6.95 (td, *J*=1.1 Hz and *J*=7.5 Hz, 1H), 5.41 (s, 1 H), 3.82 (m, 1 H), 3.62 (s, 3H), 3.50 (s, 1 H), 3.06 (dd, *J*=5.5 Hz and *J*=15.1 Hz, 1H), 2.92 (dd, *J*=5.4 Hz and *J*=15.1 Hz, 1 H);
   **Mp =** 180-183°C.
**2) Second Step:** Synthesis of (1S,3R)-2-(2-Chloro-acetyl)-1-(6-chloro-pyridin-3-yl)-2,3,4,9-tetrahydro-1H-β-carboline-3-carboxylic acid methyl ester (Compound **83t**) 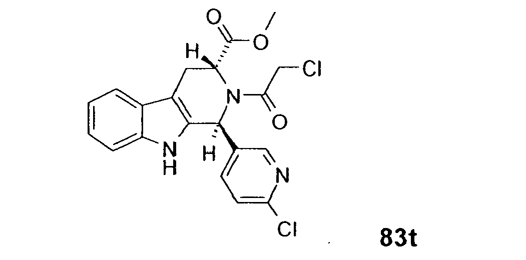 **83t** was prepared according to general protocol 2 starting from compound **81t** as obtained above at step 1 (150 mg, 1 eq.). Compound **83t** was obtained as a white powder (147 mg, 80%) which was directly engaged in the cyclisation step.
   **LC :** t_{R}= 5.92 min;
   **MS** (ESI+) : m/z=418 (M+H)⁺.
3) Third step: Synthesis of Compound **84t**

Compound **84t** was prepared according to general protocol 4 starting from compound **83t** as obtained above at step 2 (0.44 mmol), triethylamine (185 µl, 3 eq.) and (R)-(-)-1-Benzyl-3-aminopyrrolidine (227 µl, 3 eq.) in MeOH and obtained after purification by TLC (CH₂Cl₂/MeOH 95:5 v/v) as a white powder (120 mg, 52%).
**LC:** t_{R}= 4.77 min;
**MS** (ESI+): m/z= 526 (M+H)+;
**¹H NMR** (MeOD): δ 8.34 (d, *J*=2.4 Hz, 1H) 7.65 (dd, *J*=2.7 Hz and 8.4 Hz, 1 H), 7.52 (d, *J*=7.8 Hz, 1 H), 7.41 (d, *J*=8.4 Hz, 1H), 7.36-7.24 (m, 6H), 7.15 (td, *J*=6.9 Hz and 1.2 Hz, 1H), 7.07 (td, *J*=6.9 Hz and 0.9 Hz, 1H), 7.03 (s, 1H), 5.12-5.04 (m, 1H), 4.36 (d, *J*=17.7 Hz, 1H), 4.27 (d, *J*=18.0 Hz, 1 H), 4.23 (dd, *J*=11.7 Hz and 4.2 Hz, 1H), 3.64 (d, *J*=12.6 Hz, 1H), 3.58 (d, *J*=12.6 Hz, 1H), 3.42 (dd, *J*=15.3 Hz and 4.2 Hz, 1 H), 3.04 (dd, *J*=15.3 Hz and 12.0 Hz, 1 H), 3.02-2.87 (m, 1H), 2.77 (dd, *J*=10.5 Hz and 3.3 Hz, 1H), 2.50 (dd, *J*=10.5 Hz and 7.5 Hz, 1H), 2.33 (m, 1H), 2.21-2.14 (m, 1H), 1.98-1.87 (m, 1 H).

### EXAMPLE 21: Synthesis of (6R,12aR)-6-Benzyl-2-(1-benzyl-piperidin-4-yl)-2,3,6,7,12,12a-hexahydro-pyrazino[1',2':1,6]pyrido[3,4-b]indole-1,4-dione (Compound 89c)

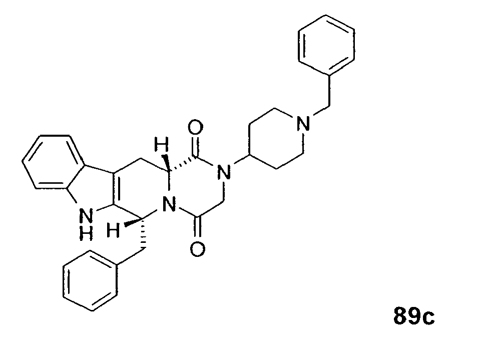

**1) First Step:** Synthesis of (1R,3R)-1-Benzyl-2,3,4,9-tetrahydro-1H-β-carboline-3-carboxylic acid methyl ester (Compound **85c**) 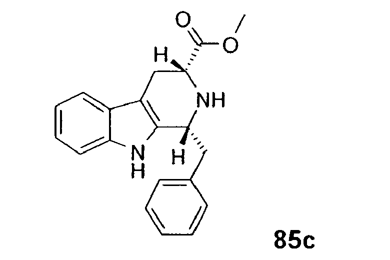 **85c** was prepared according to general protocol 1 starting from D-tryptophan-OMe.HCl (500 mg) and phenylacetaldehyde (274 µl, 1.25 eq.), after stirring for 72 hours and purification by TLC (chloroform/ether). Compound **85c** was obtained as a white powder (120 mg, 19%).
   **LC:** t_{R}=3.9 min;
   **MS** (ESI+): m/z= 321 (M+H)⁺;
   **¹H NMR** (CDCl₃) : δ 7.50 (d, *J*=8.0 Hz, 1H), 7.37 (m, 5H), 7.22-7.08 (m, 4H), 4.55 (t, *J*=6.8 Hz, 1 H), 3.83 (s, 3H), 3.79 (dd, *J*=8.4 Hz and *J*=4.2 Hz, 1H), 3.25 (dd, *J*=13.4 Hz and *J*=6.3 Hz, 1H), 3.16 (dd, *J*=15.0 Hz and *J*=12.3 Hz, 1H), 3.06 (dd, *J*=13.4 Hz and *J*=7.7 Hz, 1H), 2.87 (m, 1H);
   **¹³C NMR** (CDCl₃), δ 173.39, 137.58, 135.68, 134.97, 129.48, 129.09, 127.28, 126.71, 121.88, 119.60, 118.07, 110.86, 108.38, 56.47, 54.12, 52.31, 41.67, 25.86.
**2) Second Step:** Synthesis of (1R,3R)-1-Benzyl-2-(2-chloro-acetyl)-2,3,4,9-tetrahydro-1H-β-carboline-3-carboxylic acid methyl ester (Compound **87c)** 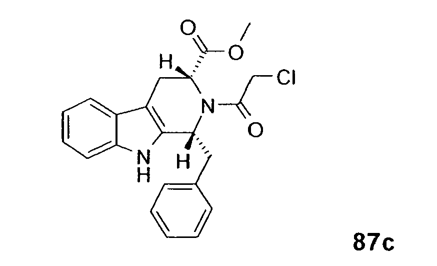 **87c** was prepared according to general protocol 2 starting from compound **85c** as obtained above at step 1 (120 mg, 1 eq.). Compound **87c** was obtained as a yellow powder (148 mg, 100%) which was directly engaged in the cyclisation step.
   **LC :** t_{R}=6.7 min;
   **MS** (ESI+) : m/z= 397 (M+H)⁺.
3) Thirs Step: Synthesis of Compound 89c
   Compound **89c** was prepared according to general protocol 6 starting from compound **87c** as obtained above at step 2 (148 mg, 1 eq.) and 4-amino-N-benzylpiperidine (2.5 eq.) in EtOH. Compound **89c** was obtained after purification by TLC (chloroform/ether) as a white powder (95 mg, 49%).
   **LC:** t_{R}= 4.9 min;
   **MS** (ESI+) : m/z= 519 [M+H]⁺;
   **¹H RMN** (CDCl₃), δ: 8.06 (s, 1H), 7.51 (d, *J*=7.5 Hz, 1H), 7.33 (m, 6H), 7.23-7.08 (m, 5H), 6.73 (d, *J*=7.0 Hz, 1H), 5.61 (t, *J*=5.1 Hz, 1H), 4.50 (m, 1H), 4.16 (d, *J*=16.9 Hz, 1 H), 3.96 (dd, *J*=11.5 Hz and *J*=4,9 Hz, 1 H), 3.86 (d, *J*=16.7 Hz, 1H), 3.63 (s, 2H), 3.29 (dd, *J*=15.7 Hz and *J*=5,0 Hz, 1H), 3.18 (d, *J*=5.13 Hz, 2H), 3.09 (m, 2H), 2.25 (q, *J*=11,8 Hz, 2H), 2.05 (dd, *J*=15.5 Hz and *J*=11.4 Hz, 1 H), 1.96-1.70 (m, 4H).

### EXAMPLE 22: Synthesis of (6S,12aR)-6-Benzyl-2-(1-benzyl-piperidin-4-yl)-2,3,6,7,12,12a-hexahydro-pyrazino[1',2':1,6]pyrido[3,4-b]indole-1,4-dione (Compound 89t)

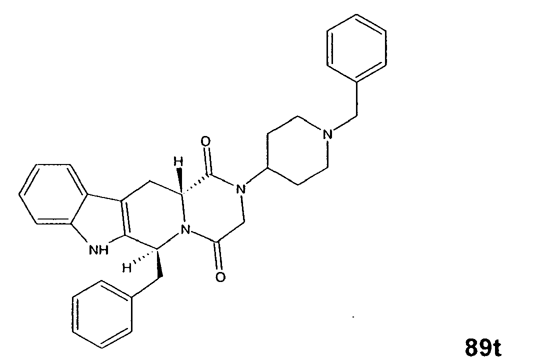

**1) First Step:** Synthesis of (1S,3R)-1-Benzyl-2,3,4,9-tetrahydro-1H-β-carboline-3-carboxylic acid methyl ester (Compound **85t)** 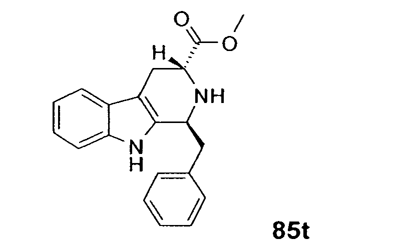 **85t** was prepared according to general protocol 1 starting from D-tryptophan-OMe.HCl (500 mg) and phenylacetaldehyde (274 µl, 1.25 eq.), after stirring for 72 hours and purification by TLC (chloroform/ether). Compound **85t** was obtained as a white powder (70 mg, 11%).
   **LC:** t_{R}=4.2 min;
   **MS** (ESI+): m/z= 321 (M+H)⁺;
   **¹H NMR** (CDCl₃): δ 7.64-7.30 (m, 6H), 7.15 (m, 4H), 4.24 (t, *J*=5,7 Hz, 1 H), 3.83 (m, 1H), 3.81 (s, 3H), 3,36 (m, 4H);
   **¹³C NMR** (CDCl₃), δ 169.77, 136.05, 135.81, 129.74, 129.10, 127.67, 125.79, 122.66, 119.86, 118.28, 111.22, 106.04, 53.20, 52.67, 52.51, 39.68, 22.77.
**2) Second Step:** Synthesis of (1S,3R)-1-Benzyl-2-(2-chloro-acetyl)-2,3,4,9-tetrahydro-1H-β-carboline-3-carboxylic acid methyl ester (Compound **87t**) 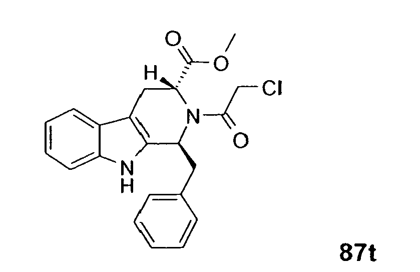 **87t** was prepared according to general protocol 2 starting from compound **85t** as above obtained at step 1 (70 mg, 1 eq.). Compound **87t** was obtained as a yellow powder (85 mg, 99%) which was directly engaged in the cyclisation step.
   **LC :** t_{R}=6.5 min;
   **MS** (ESI+) : m/z= 397 (M+H)⁺.
**3)** **Third Step**: Synthesis of Compound **89t**
   Compound **89t** was prepared according to general protocol 6 starting from compound **87t** as above obtained at step 2 (95 mg, 1 eq.) and 4-amino-N-benzylpiperidine (2.5 eq.) in EtOH. and obtained after purification by TLC (chloroform/ether) as a white powder (66 mg, 61 %).
   **LC:** t_{R}= 4.7 min;
   **MS** (ESI+) : m/z= 519 [M+H]⁺;
   **¹H RMN** (CDCl₃): δ 7.47 (m, 2H), 7,37-7,07 (m, 12H), 6.04 (dd, *J*=8.5 Hz and *J*=5.7 Hz, 1H), 4.54 (m, 1H), 4.34 (dd, *J*=11.7 Hz and *J*=3.9 Hz, 1H), 4.03 (d, *J*=17.6 Hz, 1H), 3.95 (d, *J*=17.7 Hz, 1H), 3.59 (s, 2H), 3.46 (dd, *J*=15.4 Hz and *J*=4.0 Hz, 1H), 3.26 (dd, *J*=13.0 Hz and *J*=5.6 Hz, 1H), 3.15-3.03 (m, 3H), 2.84 (dd, *J*=14.4 Hz and *J*=3.3 Hz, 1 H), 2.21 (m, 2H), 1.88 (m, 2H), 1.69 (m, 2H).

### EXAMPLE 23: Synthesis of (6R,12aR)-6-Benzyl-2-[1-(1-methyl-1H-imidazol-2-ylmethyl)-piperidin-4-yl]-2,3,6,7,12,12a-hexahydro-pyrazino-[1',2':1,6]pyrido[3,4-b]indole-1,4-dione (Compound 91c)

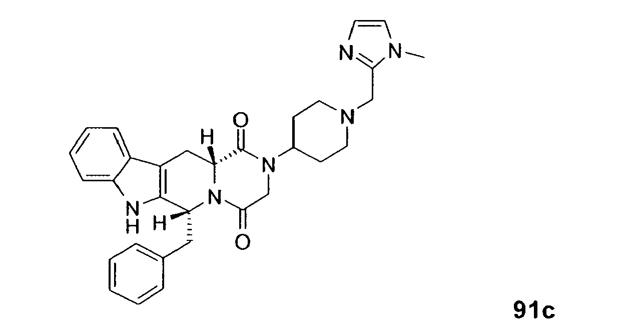

**1) First Step:** Synthesis of a reactive amine: 1-(1-Methyl-1*H*-imidazol-2-ylmethyl)-piperidin-4-yl-ammonium trichloride (compound **59**) 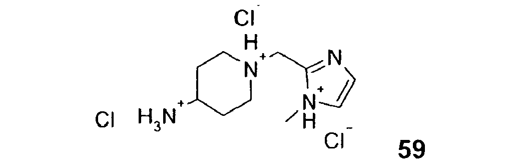
   **a)** Synthesis of [1-(1-Methyl-1*H*-imidazol-2-ylmethyl)-piperidin-4-yl]-carbamic acid *tert*-butyl ester (Compound **61)** 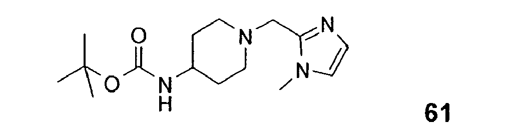 2-(chloromethyl)-1-methyl-1H-imidazole hydrochloride (800 mg, 1 eq.) and 4-(N-Boc-amino)piperidine (959 mg, 1 eq.) were dissolved in CH₂Cl₂ (48 mL) and Diisopropyl Ethyl Amine (DIEA) (2.5 mL, 3 eq.) was added. The reaction mixture was stirred at room temperature for 48 h. Solvent was evaporated, the residue dissolved in CH₂Cl₂ and washed with saturated NaHCO₃ aqueous solution and water, dried over MgSO₄, and evaporated to dryness to give 1.25 g of a yellow powder (89 %)
      **LC :** t_{R}=2.6 min;
      **MS** (ESI+) : m/z = 295 (M+H)⁺;
      **¹H NMR** (DMSO-*d₆*) : δ 7.05 (s, 1H), 6.73 (m, 2H), 3.61 (s, 3H), 3.45 (s, 2H), 3.18 (m, 1H), 2.68 (d, *J*=11.8 Hz, 2H), 1.95 (t, *J*=11.6 Hz, 2H), 1.64 (d, *J*=10.7 Hz, 2H), 1.36 (s, 9H), 1.29 (m, 2H).
   **b)** Synthesis of Compound **59**
      Intermediate **61** as obtained above at step a) (940 mg) was dissolved in a mixture of methanol (16 mL) and HCl in dioxane 4N (16 mL). The reaction mixture was stirred at 40°C for 5h. After evaporation, compound **59** was obtained as a white powder (957 mg, 100%).
      **LC :** t_{R}=0.6 min;
      **MS** (ESI+) : m/z = 195 (M+H)⁺
**2) Second Step**: Synthesis of Compound 91c
   Compound **91c** was prepared according to general protocol 3 starting from a mixture of compounds **87c** and **87t** as respectively above obtained at each Step 2 of Examples 21 and 22 (110 mg, 1 eq.) and Compound **59** as above prepared at step 1) b) (2.2 eq.) in MeOH. Compound **91c** was obtained after purification by TLC (CH₂Cl₂/MeOH) as a white powder (20 mg, 14%).
   **LC:** t_{R}= 4.4 min;
   **MS** (ESI+) : m/z= 523 [M+H]⁺;
   **¹H RMN** (MeOD), δ: 7.36 (m, 2H), 7.10 (m, 4H), 7.01 (m, 3H), 6.91 (s, 1 H), 6.47 (d, *J*=7.7 Hz, 2H), 5.64 (m, 1 H), 4.32 (m, 1 H), 4.07 (m, 3H), 3.80 (s, 3H), 3.66 (s, 2H), 3.50 (dd, *J*=13.1 Hz and *J*=5.2 Hz, 1 H), 3.08 (m, 4H), 2.23 (m, 2H), 2.02-1.67 (m, 4H), 1.38 (dd, *J*=11.45 Hz and *J*=11.6 Hz, 1 H).

### EXAMPLE 24: Synthesis of (6S,12aR)-6-Benzyl-2-[1-(1-methyl-1H-imidazol-2-ylmethyl)-piperidin-4-yl]-2,3,6,7,12,12a-hexahydro-pyrazino-[1',2':1,6]pyrido[3,4-b]indole-1,4-dione (Compound 91t)

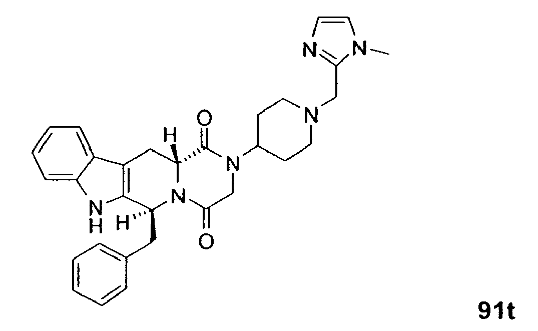

Compound **91t** was prepared according to general protocol 3 starting from a mixture of compounds **87c** and **87t** as respectively above obtained at each Step 2 of Examples 21 and 22 (110 mg, 1 eq.) and Compound **59** as above obtained at Step 1) b) of Example 23 (2.2 eq.) in MeOH. Compound **91t** was obtained after purification by TLC (CH₂Cl₂/MeOH) as a white powder (30 mg, 21 %).
**LC:** t_{R}= 4.3 min;
**MS** (ESI+) : m/z= 523 [M+H]⁺;
**¹H RMN** (CDCl₃): δ 7.63 (dd, *J*=10.6 Hz and *J*=1.7 Hz, 2H), 7,39 (m, 2H), 7.23-7.11 (m, 6H), 7.03 (t, *J*=7.1 Hz, 1H), 6.01 (t, *J*=6.8 Hz, 1H), 4.42 (m, 1 H), 4.30 (s, 2H), 4.13-3.89 (m, 6H), 3.41-3.19 (m, 3H), 3.59 (s, 2H), 2.87 (m, 3H), 2.11 (m, 2H), 1.84 (m, 2H).

### EXAMPLE 25: Synthesis of (6R,12aR)-2-(1-Benzyl-piperidin-4-yl)-6-phenyl-2,3,6,7,12,12a-hexahydro-pyrazino[1',2':1,6]pyrido[3,4-b]indole-1,4-dione (Compound 90)

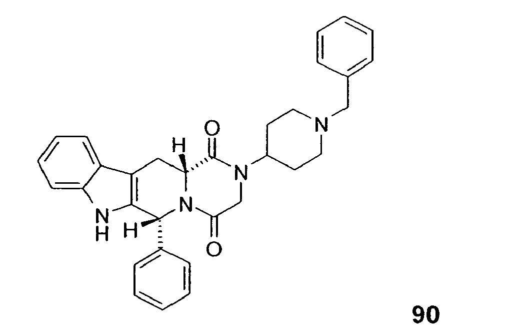

**1) First Step:** Synthesis of (1R,3R)-1-Phenyl-2,3,4,9-tetrahydro-1*H*-β-carboline-3-carboxylic acid methyl ester (Compound **86c)** 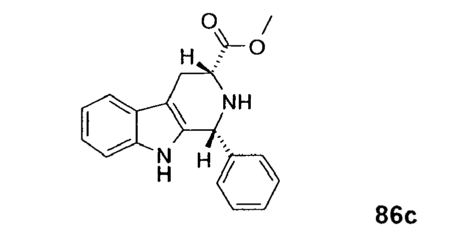 **86c** was prepared according to general protocol 1 starting from D-tryptophan-OMe.HCl (1.02 g) and 4-benzaldehyde (507 µl), after stirring for 48 hours and purification by column chromatography. Compound **86c** was obtained as a white powder (716 mg, 58%).
   **LC :** t_{R}=3.6 min;
   **MS** (ESI+) : m/z=307 (M+H)⁺;
   **¹H NMR** (DMSO-*d₆*), δ 10.34 (s, 1H), 7.42 (d, *J*=8.0 Hz, 1 H), 7.36 (m, 5H), 7.19 (d, *J*=7.3 Hz, 1H), 6.92 (m, 2H), 5.21 (d, *J*= 5.1 Hz, 1H), 3.84 (m, 1H), 3.70 (s, 3H), 3.01 (dd, *J*= 2.3 Hz and *J*= 13.3 Hz, 1H), 2.79 (m, 1 H), 2.69 (t, *J*= 4.9 Hz, 1 H);
   **Mp** = 223-225°C.
**2) Second Step:** Synthesis of (1R,3R)-2-(2-Chloro-acetyl)-1-phenyl-2,3,4,9-tetrahydro-1*H*-β-carboline-3-carboxylic acid methyl ester (Compound **88)** 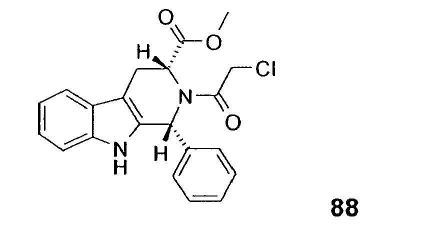 **88** was prepared according to general protocol 2 starting from compound 86c as obtained above at step 1 (516 mg, 1 eq.). Compound **88** was obtained as a white powder (552 mg, 92%) which was directly engaged in the cyclisation step.
   **LC:** t_{R}=6.6 min;
   **MS** (ESI+) : m/z=383 (M+H)⁺.
**3)** **Third Step**: Synthesis of Compound **90**
   Compound **90** was prepared according to general protocol 4 starting from compound **88** as obtained above at step 2 (100 mg, 1 eq.) and 4-amino-N-benzylpiperidine (2.2 eq.) in MeOH. Compound 90 was obtained after purification by TLC (CH₂Cl₂/MeOH) as a white powder (74 mg, 56%).
   **LC :** t_{R}= 4.7 min;
   **MS** (ESI+) : m/z= 505 [M+H]⁺;
   **¹H NMR** (DMSO-*d₆*), δ (ppm) : 11.14 (s, 1H), 7.53 (d, *J*= 7.5 Hz ; 1 H), 7.22 (m, 10H), 7.15 (m, 1 H), 6.96 (m, 2H), 6.25 (s, 1H), 4.43 (dd, J₁= 4.6 Hz, J₂= 11.5 Hz; 1H), 4.23 (m, 1 H), 4.04 (d, *J*=16.6 Hz, 1H), 3,87 (d, J=16.7 Hz, 1 H), 3,46 (m, 3H), 2.86 (m, 3H), 2.02 (m, 2H), 1.65 (m, 2H), 1.56 (m, 1H), 1.45 (m, 1 H).

### EXAMPLE 26: Synthesis of 6-Benzo[1,3]dioxol-5-yl-2-(1-benzyl-piperidin-4-yl)-9-fluoro-2,3,6,7,12,12a-hexahydro-pyrazino[1',2':1,6]pyrido[3,4-b]-indole-1,4-dione (CIS : (1R,3R) and (1S,3S), mixture of enantiomers) (Compound 95)

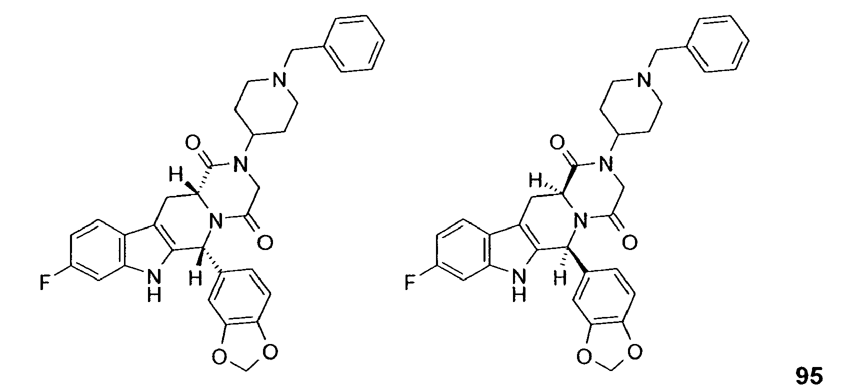

**1) First Step:** Synthesis of 2-(6-Fluoro-1H-indol-3-yl)-1-methoxycarbonylethyl-ammonium chlohydrate (Compound **92)** 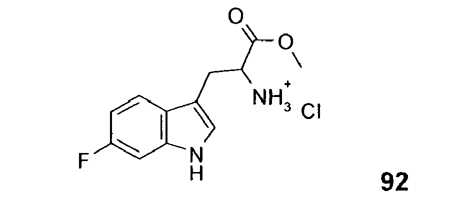 Compound **92** was prepared according to general protocol 5 starting from 6-fluoro-DL-tryptophan (4.67 g, 250 mmol) and obtained as a white solid (5.7 g, 100 %).
   **LC :** t_{R}= 3.2 min;
   **MS** (ESI+) : m/z= 237 (M+H)⁺;
   **¹H NMR** (DMSO-*d₆*): δ 11,24 (s, 1H), 7,51 (dd, *J*=8,7 Hz and *J*=5,4 Hz, 1H), 7,26 (d, *J*=2,3 Hz, 1H), 7,15 (dd, *J*=10,1 Hz and *J*=2,3 Hz, 1 H), 6,87 (ddd, *J*=11,0 Hz, *J*=8,7 Hz and *J*=2,4 Hz, 1 H), 4,21 (t, *J*=6,4 Hz, 1 H), 3,64 (s, 3H), 3,30 (m, 2H).
**2) Second Step:** Synthesis of 1-Benzo[1,3]dioxol-5-yl-7-fluoro-2,3,4,9-tetrahydro-1*H*-β-carboline-3-carboxylic acid methyl ester (CIS : mixture of enantiomers (1R,3R) and (1S,3S)) (Coumpound **93)** 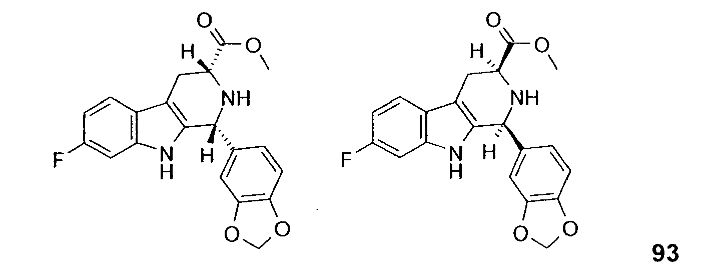 **93** was prepared according to general protocol 1 starting from compound **92** as obtained above at step 1 (1.4 g) and piperonal (940 mg), after stirring for 120 hours and purification by column chromatography. Compound **93** was obtained as a white powder (590 mg, 32%).
   **LC :** t_{R}= 4.0 min;
   **MS** (ESI+) : m/z= 369 (M+H)⁺;
   **¹H NMR** (DMSO-*d₆*): 6 10.42 (s, 1H), 7.42 (dd, *J*=8.5 Hz and J=5.5 Hz, 1 H), 6.97-6.77 (m, 5H), 6.00 (s, 2H), 5.11 (d, *J*=5.4 Hz, 1 H), 3.84 (dt, *J*=11.1 Hz and *J*=4.1 Hz, 1H), 3.70 (s, 3H), 3.00 (dd, *J*=15.1 Hz and J=2.88 Hz, 1 H), 2.85-2.69 (m, 2H).
**3) Third Step:** Synthesis of 1-Benzo[1,3]dioxol-5-yl-2-(2-chloro-acetyl)-7-fluoro-2,3,4,9-tetrahydro-1H-β-carboline-3-carboxylic acid methyl ester (CIS : (1R,3R) and (1S,3S), mixture of enantiomers) (Compound **94)** 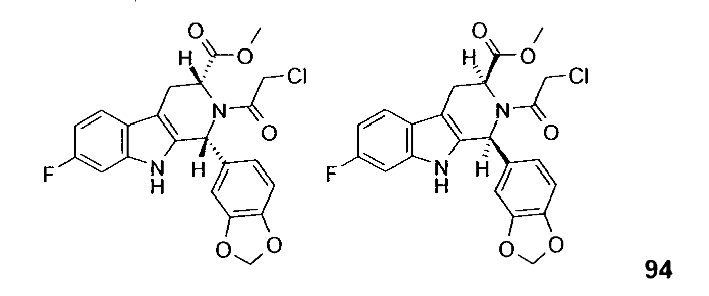 **94** was prepared according to general protocol 2 starting from compound **93** as obtained above at step 2 (378 mg) and obtained as a yellow powder (392 mg, 86%) which was directly engaged in the cyclisation step.
   **LC:** t_{R}= 6.4 min;
   **MS** (ESI+) : m/z=445 (M+H)⁺.
**4) Forth Step**: Synthesis of Compound **95**
   **95** was prepared according to general protocol 6 starting from **94** as obtained above at step 3 (53 mg) and 1-Benzyl-piperidin-4-ylamine (57 µl) in 2 minutes. Compound **95** was obtained as a white powder (27 mg, 43%).
   **LC :** t_{R} = 4.8 min;
   **MS** (ESI+) : m/z = 567 (M+H)⁺;
   **¹H NMR** (CDCl₃) : δ 7,62 (m, 1 H), 7,47 (m, 5H), 6,91 (m, 2H), 6,76 (m, 2H), 6,48 (d, *J*=8,4 Hz, 1H), 6,25 (s, 1 H), 5,85 (d, *J*=1,4 Hz, 1 H), 5,84 (d, *J*=1,4 Hz, 1 H), 4,61 (m, 1H), 4,31 (dd, *J*=11,6 Hz and *J*=4,8Hz, 1 H), 4,29 (m, 1 H), 4,07 (m, 2H), 3,90 (d, *J*=16,9 Hz, 1 H), 3,68 (m, 1 H), 3,60 (dd, *J*=15,7 Hz and *J*=4,4 Hz, 1 H), 3,43 (m, 1 H), 3,12 (dd, *J*=15,9 Hz and *J*=12,1 Hz, 1 H), 2,68 (m, 4H), 1,98 (d, *J*=11,9 Hz, 1 H), 1,70 (m, 1 H).

### EXAMPLE 27: Synthesis of (6R,12aR)-2-(1-Benzyl-piperidin-4-yl)-6-(4-difluoromethoxy-phenyl)-2,3,6,7,12,12a-hexahydro-pyrazino[1',2':1,6] pyrido[3,4-b]indole-1,4-dione (Compound 96)

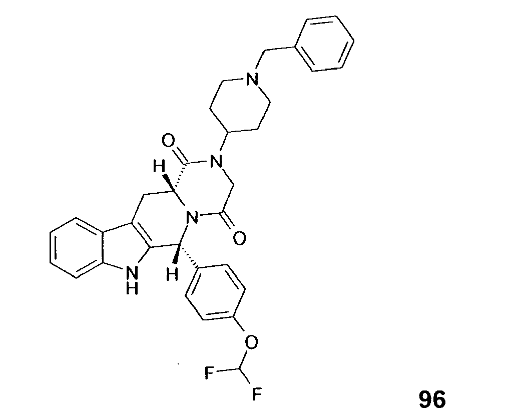

**1) First Step:** Synthesis of (1R,3R)-1-(4-Difluoromethoxy-phenyl)-2,3,4,9-tetrahydro-1*H*-β-carboline-3-carboxylic acid methyl ester (Compound **74c)** 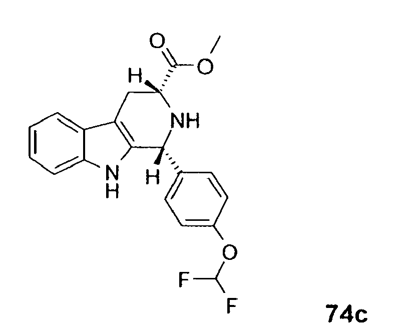 **74c** was prepared according to general protocol 1 starting from D-tryptophan-OMe.HCl (1.02 g) and 4-(difluoromethoxy)benzaldehyde (862 mg), after stirring for 72 hours and purification by column chromatography. Compound **74c** was obtained as a white powder (845 mg, 57%).
   **LC:** tR=4.3 min;
   **MS** (ESI+) : m/z=373 (M+H)+;
   **1 H NMR** (DMSO-*d6*), δ: 10.36 (s, 1 H), 7.41 (d, *J*=8.4 Hz, 2H), 7.23 (t, JH-F= 74 Hz, 1H), 7.21 (m, 1H), 7.15 (d, *J*=8.4 Hz, 2H), 6.98 (m, 2H), 5.23 (s, 1 H), 3.89 (m, 1 H), 3.72 (s, 3H), 3.02 (dd, *J*=2.6 Hz and *J*= 13.8 Hz, 1 H), 2.83 (dd, *J*= 13 Hz and *J*=14.6 Hz, 1 H);
   **Mp** = 90-93°C.
**2) Second Step:** Synthesis of (1 R,3R)-2-(2-Chloro-acetyl)-1-(4-difluoromethoxy-phenyl)-2,3,4,9-tetrahydro-1*H*-β-carboline-3-carboxylic acid methyl ester (Compound 76) 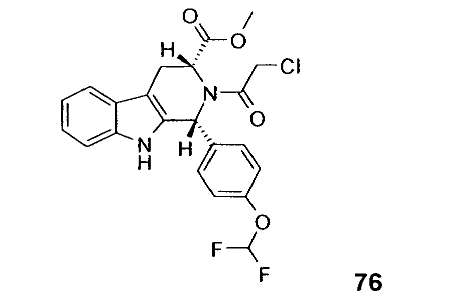 **76** was prepared according to general protocol 2 starting from compound **74c** as obtained above at step1 (625 mg, 1 eq.) and obtained as an orange powder (711 mg, 94%) which was directly engaged in the cyclisation step.
   **LC :** t_{R}=6.8 min;
   **MS** (ESI+) : m/z=449 (M+H)⁺.
**3)** **Third Step**: Synthesis of Compound **96**
   Compound **96** was prepared according to general protocol 6 starting from compound **76** as obtained above at step 2 (50 mg, 1 eq.) and 4-amino-N-benzylpiperidine (2.5 eq.) in EtOH. Compound **96** was obtained after purification by TLC (CH₂Cl₂/MeOH) as a white powder (28 mg, 45%)
   **LC:** t_{R}= 5.09 min;
   **MS** (ESI+): m/z= 571 [M+H]⁺;
   **¹H RMN** (DMSO-d₆), δ: 11.11 (s, 1 H), 7.56 (d, *J*=7.56 Hz, 2H), 7.37-7.27 (m, 7H), 7.12-6.88 (m, 5H), 6.22 (s, 1 H), 4.45 (dd, *J*= 11.49 Hz and *J*=4.44 Hz, 1 H), 4.27 (m, 1 H), 4.06 (d, *J*=16.56 Hz, 1H), 3.96 (d, *J*= 16.56 Hz, 1 H), 3.53-3.47 (m, 3H), 3.03 (m, 3H), 1.99 (m, 2H), 1.72 (m, 2H), 1.57 (m, 1 H), 1.48 (m, 1 H).

### EXAMPLE 28: Synthesis of (6R,12aR)-2-(1-Benzyl-piperidin-4-yl)-6-(6-chloro-pyridin-3-yl)-2,3,6,7,12,12a-hexahydro-pyrazino[1',2':1,6]pyrido [3,4-b]indole-1,4-dione (Compound 97)

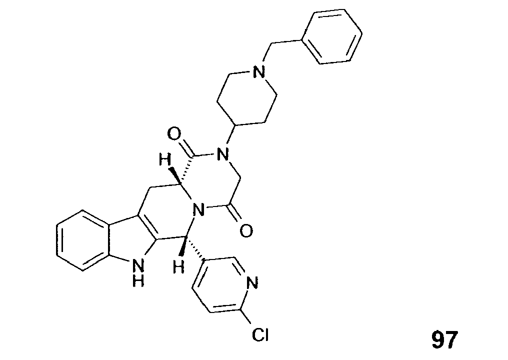

Compound **97** was prepared according to general protocol 6 starting from compound **83c** as obtained at step 2 of above Example 19 (50 mg, 1 eq.) and 4-amino-N-benzylpiperidine (2.5 eq.) in ethanol. Compound **97** was obtained after purification by TLC (dichloromethane/methanol) as a white powder (35 mg, 54%)
**LC:** t_{R}= 4.62 min;
**MS** (ESI+): m/z= 540 [M+H]⁺;
**¹H RMN** (DMSO-d₆), δ: 11.19 (s, 1 H), 8.44 (d, *J*=2.25 Hz, 1 H), 7.67-7.58 (m, 2H), 7.38-7.24 (m, 7H), 7.09-6.98 (m, 2H), 6.08 (s, 1H), 4.45 (dd, *J*= 11.19 Hz and *J*=4.2, 1H), 4.26 (m, 1H), 4.09 (d, *J*=9.81 Hz, 1H), 4.00 (d, *J*=9.81 Hz, 1H), 3.53-3.47 (m, 3H), 3.06 (m, 1H), 2.87 (m, 2H), 2.07 (m, 2H), 1.83-1.44 (m, 4H).

### EXAMPLE 29: Synthesis of (6R,12aR)-6-Benzo[1,3]dioxol-5-yl-2-(1-benzyl-piperidin-4-yl)-9-fluoro-2,3,6,7,12,12a-hexahydro-pyrazino [1',2':1,6]pyrido[3,4-b]indole-1,4-dione (Compound 98)

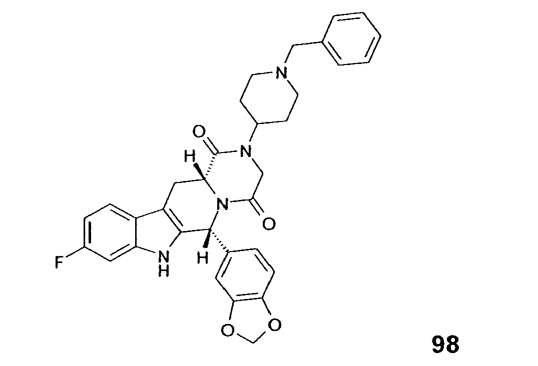

Compound **98** was prepared according to general protocol 6 starting from compound **94** as obtained at step 3 of above Example 26 (50 mg, 1 eq.) and 4-amino-N-benzylpiperidine (2.5 eq.) in ethanol. Compound **98** was obtained after purification by TLC (dichloromethane/methanol) as a white powder (27 mg, 42%)
**LC:** t_{R}= 4.86 min;
**MS** (ESI+): m/z= 567 [M+H]⁺;
**¹H RMN** (CDCl₃), δ: 7.62 (m, 1H), 7.42-7.34 (m, 5H), 6.94-6.84 (m, 2H), 6.76 (m, 2H), 6.47 (d, *J*= 8.46 Hz, 1H), 6.25 (s, 1H), 5.84 (td, *J*= 7.1 Hz and *J*=1.3 Hz, 2H), 4.60 (m, 1 H), 4.33 (m, 2H), 4.04 (d, *J*= 15.52 Hz, 1 H), 3.93 (d, *J*= 15.52 Hz, 1 H), 3.56 (m, 1H), 3.42 (m, 1 H), 3.16 (m, 1H), 2.82-2.40 (m, 3H), 2.09 (m, 1 H), 1.68-1.88 (m, 3H), 1.54 (m, 1 H).

### EXAMPLE 30: In vitro demonstration of the antiparasitic activity of compounds of formula (I)

### 1) Material and methods:

### Parasite cultures

Two clones of *P. falciparum* are used: (a) the 3D7 clone of NF54 which is known to be sensitive to all anti-malarials, (b) the K1 strain originating from Thailand that is resistant to chloroquine and pyrimethamine, but sensitive to mefloquine. The cultures are naturally asynchronous (65-75% ring stage) and are maintained in continuous log phase growth in RPMI-1640 medium (as developed by Moore *et. al.* at Roswell Park Memorial Institute, sold by Sigma Aldrich) supplemented with 5% washed human A+ erythrocytes, 25 mM Hepes, 32 nM NaHCO₃, and AlbuMAXII (lipid-rich bovine serum albumin) sold GIBCO, Grand Island, NY (CM). All cultures and assays were conducted at 37°C under an atmosphere of 5% CO₂ and 5% O₂, with a balance of N₂.

### Drug sensitivity assays

Stock drug solutions were prepared using compounds of formula (I) in 100% DMSO (dimethylsulfoxide) at 20 mg/ml unless otherwise suggested by the supplier. The compounds were further diluted to the appropriate concentration using complete medium RPMI-1640 supplemented with 15nM cold hypoxanthine and AlbuMAXII.

Assays were performed in sterile 96-well microtitre plates; each plate contained 100 µl of parasite culture (0.5% parasitemia, 2.5% hematocrit). Each compound was tested in triplicate and parasite growth compared to control and blank (uninfected erythrocytes) wells. After 24h of incubation at 37°C, 3.7 Bq of [³H]hypoxanthine was added to each well. Cultures were incubated for a further 24 h before they were harvested onto glass-fiber filter mats. The radioactivity was counted using a Wallac Microbeta® 1450 scintillation counter. The results were recorded as counts per minute (CPM) per well at each compound concentration, control and blank wells. Percentage inhibition was calculated from comparison to blank and control wells, and IC₅₀ values calculated using Microsoft *XL*Fit ® line fitting software (IDBS, UK).

### Primary screen

The preliminary screen used the 3D7 strain. The compounds were tested at 6 concentrations (30, 10, 3, 1, 0.3, and 0.1 µg/ml). If the compound did not affect parasite growth at 10 µg/ml it was classified as inactive, between 10 and 1 µg/ml, the compound was designated as partially active, and if <1 µg/ml, the compound was classified as active and was further evaluated by three-fold serial dilutions in a repeat test.

### Secondary screen

Both the 3D7 clone and the K1 line were used. The compounds were diluted three-fold over at 12 different concentrations with an appropriate starting concentration based on the preliminary screen. The IC₅₀ was determined by a sigmoidal dose response analysis using Microsoft *XL*Fit^{™} (IDBS, UK). For each assay, the IC₅₀ and IC₉₀ values for each parasite line were determined against the known anti-malarials chloroquine and artesunate, plus other standard compounds appropriate for the assay.

### Activity of Standards

| | | **IC₅₀** | **IC₅₀** |
|---|---|---|---|
| Chloroquine | 3D7 | 4 - 1 ng/ml | K1 130 - 210 ng/ml |
| Artesunate | 3D7 | 8 - 2 ng/ml | K1 8 - 2 ng/ml |

### 2) Results:

Results obtained for each the tested compounds are presented in Table 1 below:

**TABLE 1**

| **Compound** | **IC₅₀ 3D7(µg/ml)** | **IC₅₀ K1 (µg/ml)** |
|---|---|---|
| **6** (Synthesis example 1) | 0.100 | 0.120 |
| **10** (Synthesis example 2) | 0.270 | 0.010 |
| **14** (Synthesis example 3) | 0.290 | 1.030 |
| **15** (Synthesis example 4) | 0.600 | 0.400 |
| **16** (Synthesis example 5) | 0.300 | 0.600 |
| **17** (Synthesis example 6) | 0.980 | 0.190 |
| **18** (Synthesis example 7) | 1.34 | 0.28 |
| **26** (Synthesis example 8) | 0.006 | 0.140 |
| **27** (Synthesis example 9) | 0.080 | 0.200 |
| **28** (Synthesis example 10) | 0.090 | 0.070 |
| **29** (Synthesis example 11) | 0.500 | 0.090 |
| **31** (Synthesis example 12) | 0.230 | 0.130 |
| **33** (Synthesis example 13) | 0.170 | 0.160 |
| **35** (Synthesis example 15) | 0.070 | 0.015 |
| **70c** (Synthesis example 16) | 0.130 | 0.150 |
| **70t** (Synthesis example 17) | 0.700 | 0.500 |
| **84c** (Synthesis example 19) | 0.070 | 0.110 |
| **84t** (Synthesis example 20) | 0.090 | 1.030 |
| **89t** (Synthesis example 22) | 4.170 | 2.470 |
| **90** (Synthesis example 25) | 0.480 | 0.300 |
| **91t** (Synthesis example 24) | 4.130 | 4.070 |

These results show that all the tested compounds of formula (I) have an antiparasitic activity against *P. falciparum*.

### EXAMPLE 31: In vivo demonstration of the antiparasitic activity of compounds of formula (I)

### 1) Material and methods:

### Parasite and animal strains

*Plasmodium* strains used:

*P. berghei* ANKA strain, which is chloroquine sensitive (Killick-Kendrick, 1978).

Female mice (TFW or BALB/c, specific pathogen free), 18-20 g, or

### P. chabaudi

### General experimental procedure

Infection: Blood was taken from donor mice and diluted in RPMI-1640 medium to a parasitemia of 1% (the equivalent of 1x10⁷ infected erythrocytes/ml) whilst kept on ice. An inoculum of 0.2 ml was administered to each mouse by the intravenous route. At 2-4 hours post-inoculation dosing commences.

Evaluation endpoint: On day 5 blood smears of all animals (2 slides/mouse) were prepared, fixed with methanol and stained with 10% Giemsa's stain. Parasitemia was determined microscopically by counting 1000 red blood cells under oil immersion at 1000x magnification. Results were reported as % infected erythrocytes and compared to the chloroquine control group and the untreated control group.

### Primary screen

The chloroquine sensitive *Plasmodium berghei* ANKA strain was used in the primary evaluation.

### Treatment regimen:

Each of the tested compounds of formula (I) according to the invention was prepared as a stock solution of 5 mg/ml in 10% DMSO/PBS. Insoluble compounds of formula (I) were milled with stainless steel balls for up to 24 hours to reduce particle size. Fresh dilutions were prepared each day. The route of administration of tested compounds is intra peritoneal (i.p.). Compounds of formula (I) were given in a 0.2 ml bolus every day for 4 days at 50 mg/kg, i.p. unless *in vitro* toxicity indicates a narrow therapeutic index and a lower dose was required. The control drug, chloroquine was administered per os (p.o.) every day for 4 days at 10 mg/kg, giving >90% parasite clearance on day 4.

### Activity criteria:

| | |
|---|---|
| Inactive: | parasitemia < 25% reduction |
| Moderate activity: | parasitemia 25-75% reduction |
| Active: | parasitemia > 75% reduction |

### Secondary screen

The chloroquine sensitive *Plasmodium berghei* ANKA strain was used as in the primary evaluation.

### Treatment regimen:

Compounds considered active were repeated over a dose range to determine ED₅₀/ED₉₀ values. Compounds were formulated at 10 mg/kg or at 50 mg/kg in a standard suspension vehicle (0.5% carboxymethylcellulose, 0.5% benzyl alcohol, 0.4% Tween® 80 in 0.9% NaCl) for both oral and i.p. administration.

Dosing was over a 4 day period as above, and evaluation was also as above.

### Activity Criteria:

Depends upon compound class and therefore control drug used.

### 2) Results:

The corresponding results are tabulated in Table 2 below:

**TABLE 2**

| **Compound** | **Activity at the dose 10 mg/kg (%)** | **Activity at the dose 50 mg/kg (%)** |
|---|---|---|
| **6** (Synthesis example 1) | - | 43 |
| **10** (Synthesis example 2) | 15 | 31 |
| **26** (Synthesis example 8) | 15 | 17 |
| **27** (Synthesis example 9) | - | 10 |
| **28** (Synthesis example 10) | 24 | 32 |
| **29** (Synthesis example 11) | - | 19 |
| **84c** (Synthesis example 19) | - | 9.1 |

## Claims

1. The use, as an active principle, of at least one chiral tetra-hydro β-carboline derivative of general formula (I) below: 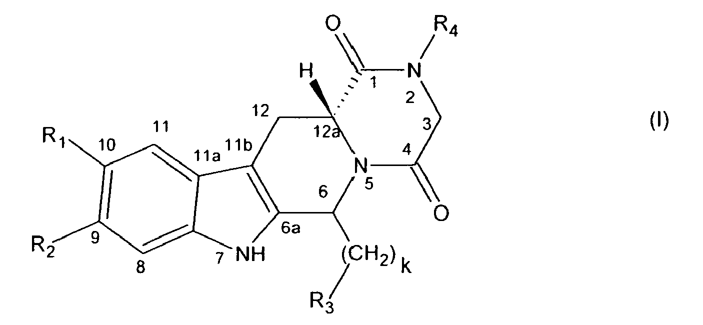 or a pharmaceutically acceptable salt thereof, in which:
- R₁ and R₂, identical or different, represent a hydrogen atom or a fluorine atom;
- k is an integer equal to 0 or 1;
- R₃ is selected from the group consisting of:
■ a phenyl ring optionally substituted by a methylenedioxy radical, a difluoromethylenedioxy radical attached to the phenyl ring at the 3'-4' carbon atoms, a thiadiazole fused with the phenyl ring at the 3'-4' positions, a difluoromethoxy radical or a chlorine atom in the para 4' position with regard to the attachment point of said phenyl ring to the piperidine cycle directly bearing R₃ when k = 0 or to the carbon atom of the -CH₂- group when k = 1;
■ a 3'-N-pyridine ring optionally substituted by a substituent chosen from a chlorine atom, a fluorine atom, a methoxy group, and a methylthio group, said substituent being in the 4' position with regard to the attachment point of said 3'-N-pyridine ring to the piperidine cycle directly bearing R3 when k = 0 or to the carbon atom of the -CH2- group when k = 1;
- R₄ is a group selected in the group consisting of the following groups: -NH-A-R₅; -NHC(O)-R₅ and the groups of formulas (II-a) to (II-c) below: 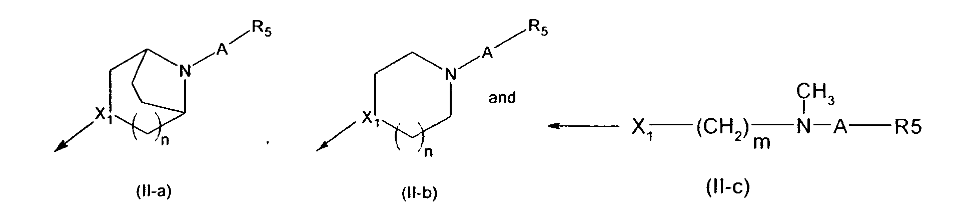
in which:
■ n is an integer equal to 0 or 1,
■ m is an integer equal to 0, 1 or 2,
■ the arrows represent the attachment point of groups of formulas (II-a), (II-b) or (II-c) on the formula (I) derivative piperazine-dione cycle;
■ X₁ is a carbon or a nitrogen atom;
- A represents a single bond or a C₁ to C₃ linear or branched alkyl chain optionally interrupted or terminated by an oxygen atom;
- R₅ is selected in the group consisting of:
**i)** a group of formula (III) below: 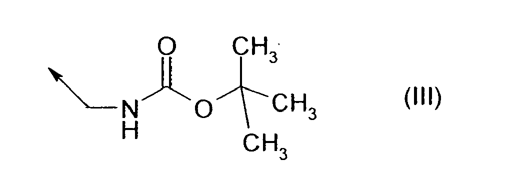 in which the arrow represents the attachment point of said group of formula (III) to A *via* a covalent bond;
**ii)** a group of formulas (IV) or (V) below: 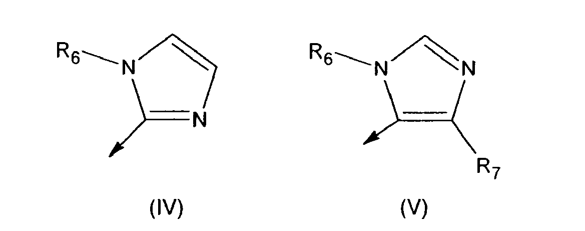 in which the arrows represent the attachment point of said group of formulas (IV) and (V) to A *via* a covalent bond and R₆ and R₇, identical or different, represent a C₁ to C₄ alkyl group; and
**iii)** a group of the following formula (VI): 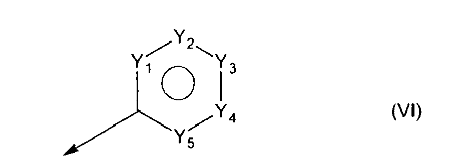
in which:
- the arrow represents the attachment point of said group of formula (VI) to A *via* a covalent bond,
- Y₁ and Y₅, identical or different, represent -CH=; -N= or
- C(R₈)= in which R₈ represents an hydrogen atom, a methyl radical or a halogen atom selected from chlorine and fluorine;
- Y₃ represents -CH=; -N=; -C(R₉)= or -C(R₁₀)=;
- Y₂ and Y₄, identical or different, represent -CH=, -N=,
- C(R₉)= or -C(R₁₀)=;
- R₉ represents a hydrogen atom or a group chosen from a methyl, an amino group and an oxygen group, said groups being optionally mono- or disubstituted with a methyl radical;
- Y₂ and Y₃ can also form together a methylenedioxy group;
- R₁₀ represents a hydrogen atom or a solubilizing group;
wherein the asymmetric carbon atom of said derivative of formula (I) is in the R conformation, for the preparation of a pharmaceutical composition for the prevention and/or the treatment of parasitic diseases involving parasites having a phosphodiesterase (PDE) activity.

2. Use according to claim 1, wherein said pharmaceutical composition is for the prevention and/or the treatment of malaria, leishmaniasis or trypanosomiasis.

3. Use according to claim 1 or 2, wherein in the derivatives of formula (I), said solubilizing groups mentioned for R₁₀ are chosen amongst the solubilizing groups of the following formulas S-1 to S-5: 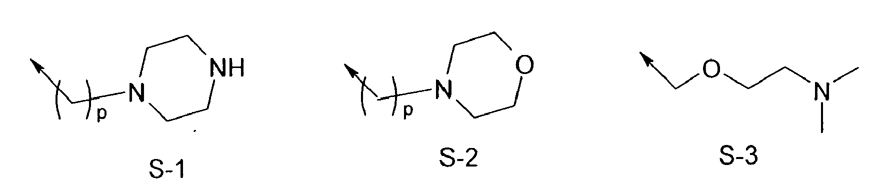 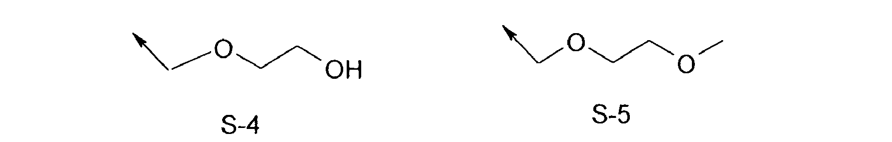 in which p is an integer equals to 0 or 1 and the arrows represent the attachment point of said groups S-1 to S-5 to the carbon atom designated for Y₂, Y₃ and Y₄ in the corresponding group of formula (VI) as defined in claim 1.

4. Use according to any one of claims 1 to 3, wherein in R₃ represents one of the following groups:

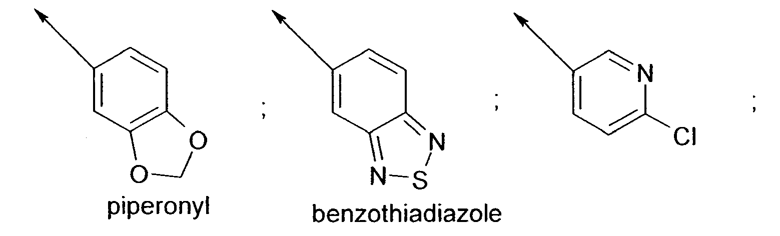

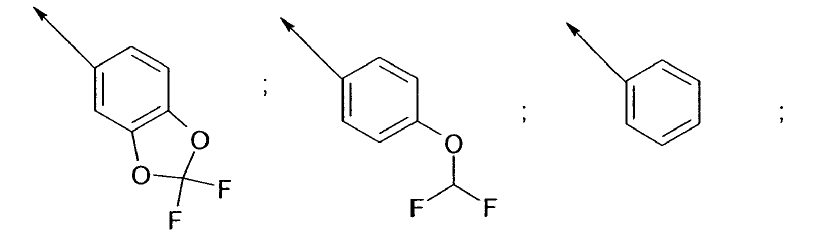

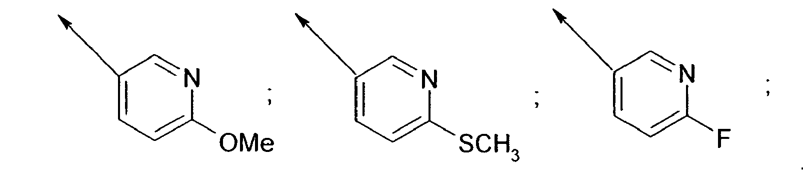

5. Use according to any one of the preceding claims, wherein R₄ is a group of formula (II-b) in which n = 1 and X₁ is a carbon or a nitrogen atom.

6. Use according to any one of the preceding claims, wherein in the groups -NH-A-R₅ and in the groups of formulas (II-a) to (II-c) mentioned for R₄, A preferably represents -CH₂-; -CH(CH₃)-; -C(CH₃)₂- or -CH₂-CH₂.

7. Use according to any one of the preceding claims, wherein in the groups of formula (II-a) to (II-c), R₅ represents one of the following groups of formulas (IV-1) to (IV-14):

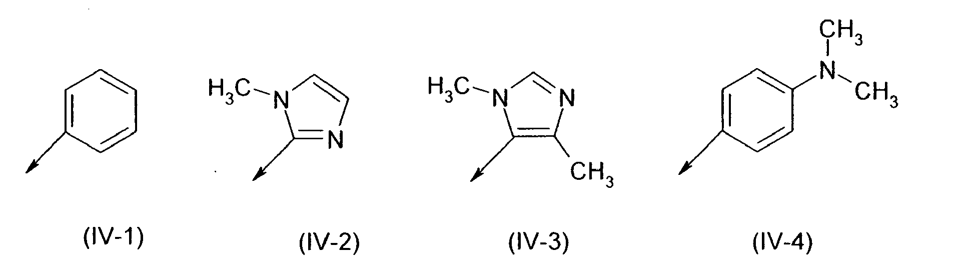

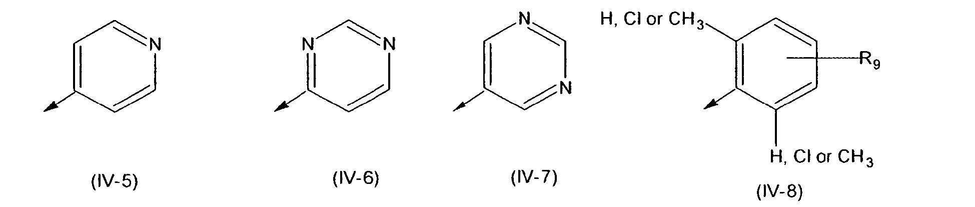

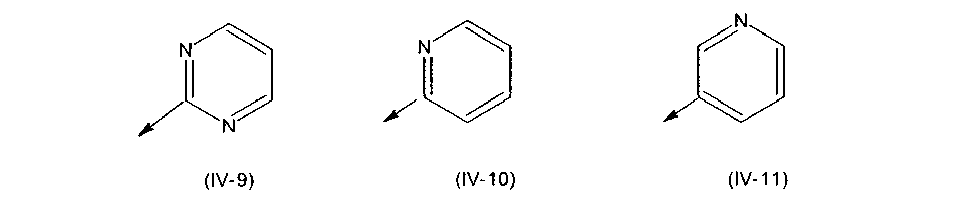

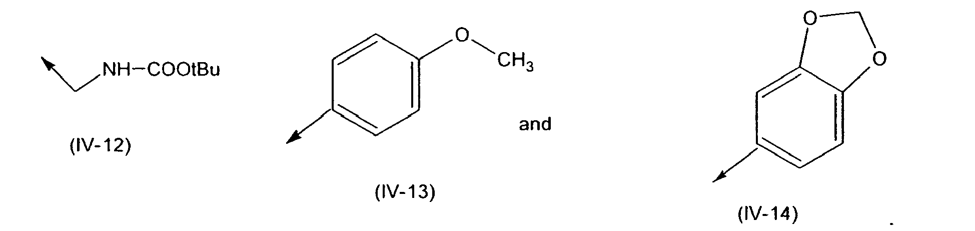

8. Use according to any one of the preceding claims, wherein said at least one derivative of formula (I) is selected in the group consisting of:
i) k = 0, R₃ represents a piperonyl, a benzothiadiazole, a 4-chloro-3-N-pyridine or a 4-fluoro-3-N-pyridine group and R₄ represents a group of formula (II-b) in which X₁ is a carbon atom or a nitrogen atom, n = 1, A is -CH₂- and R₅ is a group of formulas (IV-2), (IV-3), (IV-8), (IV-10) or (IV-12);
ii) k = 0, R₃ represents a piperonyl, a benzothiadiazole, a phenyl or a 4-chloro-3-N-pyridine group and R₄ represents a group of formula (II-b) in which X₁ is a carbon atom or a nitrogen atom, n = 0, A is -CH₂- and R₅ is a group of formula (IV-1) or (IV-2);
iii) k = 0, R₃ represents a phenyl group and R₄ represents a group of formula (II-b) in which X₁ is a carbon atom or a nitrogen atom, n = 1, A is -CH₂- and R₅ is a phenyl group of formula (IV-1);
iv) k = 0, R₃ represents a piperonyl or a benzothiadiazole group and R₄ represents a group of formula (II-c) in which X₁ is a carbon atom or a nitrogen atom, m = 3, A is -CH₂- and R₅ is a phenyl group of formula (IV-1);
v) k = 0, R₃ represents a piperonyl or a benzothiadiazole group and R₄ represents a group of formula (II-c) in which X₁ is a carbon atom or a nitrogen atom, m = 2, A is -CH₂- and R₅ is a phenyl group of formula (IV-1);
vi) k = 0, R₃ represents a piperonyl or a benzothiadiazole group and R₄ represents a group of formula (II-b) in which X₁ is a carbon atom or a nitrogen atom, n = 1, A is -CH₂- and R₅ is a phenyl group of formula (IV-1);
vii) k = 0, R₃ represents a piperonyl or a benzothiadiazole group and R₄ represents a group of formula (II-b) in which X₁ is a carbon atom or a nitrogen atom, n = 0, A is -CH₂- and R₅ is a group of formulas (IV-4), (IV-10), (IV-12), (IV-13) or (IV-14);
viii) k = 0, R₃ represents a 3',4'-(difluoromethylenedioxy)-phenyl group, R₄ represents a group of formula (II-b) in which X₁ is a carbon atom or a nitrogen atom, n = 1, A is -CH₂- and R₅ is a group of formula (IV-1);
ix) k = 0, R₃ represents a 4'-difluoromethoxyphenyl group, R₄ represents a group of formula (II-b) in which X₁ is a carbon atom or a nitrogen atom, n = 1, A is -CH₂- and R₅ is a group of formula (IV-1);
x) k = 1, R₃ represents a phenyl group, R₄ represents a group of formula (II-b) in which X₁ is a carbon atom or a nitrogen atom, n = 1, A is -CH₂- and R₅ is a group of formula (IV-1) or (IV-2);
xi) ) k = 0, R₃ represents a piperonyl, a benzothiadiazole a 4-fluoro-3-N-pyridine or a 4-chloro-3-N-pyridine group, R₄ represents a group of formula (II-b) in which X₁ is a carbon atom or a nitrogen atom, n = 1, A is single bond and R₅ is a group of formula (IV-9), and pharmaceutically acceptable salts thereof, are preferred.

9. Use according to claim 8, wherein said at least one derivative of formula (I) is selected in the group consisting of compounds in which R₁ and R₂ simultaneously represent a hydrogen atom or in which one of R₁ and R₂ represents a fluorine atom while the other of R₁ and R₂ represents a hydrogen atom.

10. Use according to claim 8 or 9, wherein said at least one derivative of formula (I) is selected in the group consisting of compounds in which X₁ represents a carbon atom.

11. Use according to any one of claims 8 to 10, wherein said at least one derivative of formula (I) is selected in the group consisting of:
- (6R,12aR)-6-Benzo[1,3]dioxol-5-yl-2-[(R)-1-(1-methyl-1*H*-imidazol-2-yl methyl)-pyrrolidin-3-yl]-2,3,6,7,12,12a-hexahydro-pyrazino[1',2':1,6]pyrido[3,4-*b*]indole-1,4-dione;
- (6S,12aR)-6-Benzo[1,3]dioxol-5-yl-2-[(R)-1-(1-methyl-1*H*-imidazol-2-yl methyl)-pyrrolidin-3-yl]-2,3,6,7,12,12a-hexahydro-pyrazino[1',2':1,6]pyrido[3,4-*b*]indole-1,4-dione;
- (6R,12aR)-2-(1-Benzyl-piperidin-4-yl)-6-phenyl-2,3,6,7,12,12a-hexahydro-pyrazino[1',2':1,6]pyrido[3,4-b]indole-1,4-dione;
- (6R,12aR)-2-((R)-1-Benzyl-pyrrolidin-3-yl)-6-(6-chloro-pyridin-3-yl)-2,3,6,7, 12,12a-hexahydro-pyrazino[1',2':1,6]pyrido[3,4-b]indole-1,4-dione;
- (6S,12aR)-2-((R)-1-Benzyl-pyrrolidin-3-yl)-6-(6-chloro-pyridin-3-yl)-2,3,6,7, 12,12a-hexahydro-pyrazino[1',2':1,6]pyrido[3,4-b]indole-1,4-dione;
- (6R,12aR)-2-(1-Benzyl-piperidin-4-yl)-6-(6-chloro-pyridin-3-yl)-2,3,6,7,12, 12a-hexahydro-pyrazino[1',2':1,6]pyrido[3,4-b]indole-1,4-dione;
- (6R,12aR)-6-Benzo[1,3]dioxol-5-yl-2-[3-(benzyl-methyl-amino)-propyl]-2,3,6, 7,12,12a-hexahydro-pyrazino[1',2':1,6]pyrido[3,4-*b*]indole-1,4-dione;
- (6S,12aR)-6-Benzo[1,3]dioxol-5-yl-2-[3-(benzyl-methyl-amino)-propyl]-2,3,6, 7,12,12a-hexahydro-pyrazino[1',2':1,6]pyrido[3,4-*b*]indole-1,4-dione;
- (6R,12aR)-6-Benzo[1,3]dioxol-5-yl-2-[2-(benzyl-methyl-amino)-ethyl]-2,3,6,7, 12,12a-hexahydro-pyrazino[1',2':1,6]pyrido[3,4-*b*]indole-1,4-dione;
- (6S,12aR)-6-Benzo[1,3]dioxol-5-yl-2-[2-(benzyl-methyl-amino)-ethyl]-2,3,6,7, 12,12a-hexahydro-pyrazino[1',2':1,6]pyrido[3,4-*b*]indole-1,4-dione;
- (6R,12aR)-6-Benzo[1,3]dioxol-5-yl-2-(1-benzyl-piperidin-4-yl)-2,3,6,7,12,12a-hexahydro-pyrazino[1',2':1,6]pyrido[3,4-*b*]indole-1,4-dione;
- (6R,12aR)-6-Benzo[1,3]dioxol-5-yl-2-(1-benzyl-piperidin-4-yl)-9-fluoro-2,3,6, 7,12,12a-hexahydro-pyrazino[1',2':1,6]pyrido[3,4-b]indole-1,4-dione;
- 6-Benzo[1,3]dioxol-5-yl-2-(1-benzyl-piperidin-4-yl)-9-fluoro-2,3,6,7,12,12a-hexahydro-pyrazino[1',2':1,6]pyrido[3,4-b]indole-1,4-dione in the form of a mixture of enantiomers (CIS : (1R,3R) and (1S,3S));
- (6R,12aR)-6-Benzo[1,3]dioxol-5-yl-2-((R)-1-benzo[1,3]dioxol-5-ylmethyl-pyrrolidin-3-yl)-2,3,6,7,12,12a-hexahydro-pyrazino[1',2':1,6]pyrido[3,4-*b*] indole-1,4-dione;
- (6R,12aR)-6-Benzo[1,3]dioxol-5-yl-2-[(R)-1-(4-methoxy-benzyl)-pyrrolidin-3-yl]-2,3,6,7,12,12a-hexahydro-pyrazino[1',2':1,6]pyrido[3,4-b]indole-1,4-dione;
- (6R,12aR)-6-Benzo[1,3]dioxol-5-yl-2-((R)-1-pyridin-2-ylmethyl-pyrrolidin-3-yl)-2,3,6,7,12,12a-hexahydro-pyrazino[1',2':1,6]pyrido [3,4-*b*]indole-1,4-dione;
- (6R,12aR)-6-Benzo[1,3]dioxol-5-yl-2-[(R)-1-(4-dimethyl-amino-benzyl)-pyrrolidin-3-yl]-2,3,6,7,12,12a-hexahydro-pyrazino[1',2':1,6]pyrido[3,4-b]indole-1,4-dione;
- 2-[(R)-3-((6R,12aR)-6-Benzo[1,3]dioxol-5-yl-1,4-dioxo-3,4,6,7,12,12a-hexa-hydro-1*H*-pyrazino[1',2':1,6]pyrido[3,4-*b*]indol-2-yl)-pyrrolidin-1-yl]-ethyl}-carbamic acid *tert*-butyl ester;
- (6R,12aR)-6-Benzo[1,3]dioxol-5-yl-2-((R)-1-benzyl-pyrrolidin-3-yl)-2,3,6,7,12, 12a-hexahydro-pyrazino[1',2':1,6]pyrido[3,4-*b*]indole-1,4-dione;
- (6S,12aR)-6-Benzo[1,3]dioxol-5-yl-2-((R)-1-benzyl-pyrrolidin-3-yl)-2,3,6,7,12, 12a-hexahydro-pyrazino[1',2':1,6]pyrido[3,4-*b*]indole-1,4-dione;
- (6R,12aR)-6-Benzo[1,3]dioxol-5-yl-2-[1-(1-methyl-1*H*-imidazol-2-ylmethyl)-piperidin-4-yl]-2,3,6,7,12,12a-hexahydro-pyrazino [1',2':1,6]pyrido[3,4-*b*]indole-1,4-dione;
- (6R,12aR)-2-(1-Benzyl-piperidin-4-yl)-6-(4-difluoromethoxy-phenyl)-2,3,6,7, 12,12a-hexahydro-pyrazino[1',2':1,6]pyrido[3,4-b]indole-1,4-dione;
- (6R,12aR)-2-(1-Benzyl-piperidin-4-yl)-6-(2,2-difluoro-benzo [1,3]dioxol-5-yl)-2,3,6,7,12,12a-hexahydro-pyrazino[1',2':1,6]pyrido[3,4-b]indole-1,4-dione;
- (6R,12aR)-6-Benzo[1,2,5]thiadiazol-5-yl-2-((R)-1-benzyl-pyrrolidin-3-yl)-2,3, 6,7,12,12a-hexahydro-pyrazino[1',2':1,6]pyrido[3,4-b]indole-1,4-dione;
- (6S,12aR)-6-Benzo[1,2,5]thiadiazol-5-yl-2-((R)-1-benzyl-pyrrolidin-3-yl)-2,3, 6,7,12,12a-hexahydro-pyrazino[1',2':1,6]pyrido[3,4-*b*]indole-1,4-dione;
- (6R,12aR)-6-Benzyl-2-(1-benzyl-piperidin-4-yl)-2,3,6,7,12,12a-hexahydro-pyrazino[1',2':1,6]pyrido[3,4-*b*]indole-1,4-dione;
- (6S,12aR)-6-Benzyl-2-(1-benzyl-piperidin-4-yl)-2,3,6,7,12,12a-hexahydro-pyrazino[1',2':1,6]pyrido[3,4-*b*]indole-1,4-dione-,
- (6R,12aR)-6-Benzyl-2-[1-(1-methyl-1*H*-imidazol-2-ylmethyl)-piperidin-4-yl]-2, 3,6,7,12,12a-hexahydro-pyrazino[1',2':1,6]pyrido[3,4-b]indole-1,4-dione;
- (6S,12aR)-6-Benzyl-2-[1-(1-methyl-1*H*-imidazol-2-ylmethyl)-piperidin-4-yl]-2, 3,6,7,12,12a-hexahydro-pyrazino[1',2':1,6]pyrido[3,4-b]indole-1,4-dione; and pharmaceutically acceptable salts thereof.

12. Use according to any one of the preceding claims, wherein said pharmaceutically acceptable salts of derivatives of formula (I) are acid addition salts formed with pharmaceutically acceptable acids.

13. Use according to claim 12, wherein said pharmaceutically acceptable salts of derivatives of formula (I) are hydrochloride, hydrobromide, sulphate or bisulphate, phosphate or hydrogenophosphate, acetate, benzoate, succinate, fumarate, maleate, lactate, citrate, tartrate, gluconate, methanesulphonate, benzene-sulphonate or paratoluene-sulphonate salts.

14. Use according to any one of the preceding claims, wherein said pharmaceutical composition is for an oral administration.

15. Use according to any one of the preceding claims, wherein said pharmaceutical composition comprises one or more additional anti-malarial drugs.

16. Chiral tetra-hydro β-carboline derivatives of formula (I') below and pharmaceutical acceptable salts thereof: 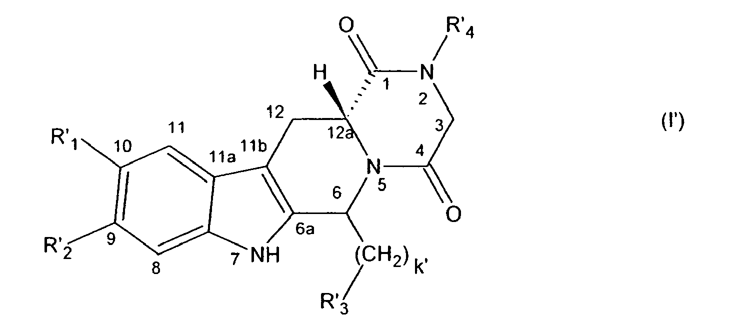 or a pharmaceutically acceptable salt thereof, in which:
- R'₁ and R'₂, identical or different, represent a hydrogen atom or a fluorine atom;
- k' is an integer equal to 0 or 1;
- R'₃ is selected from the group consisting of:
■ a phenyl ring optionally substituted by a methylenedioxy radical, a difluoromethylenedioxy radical attached to the phenyl ring at the 3'-4' carbon atoms, a thiadiazole fused with the phenyl ring at the 3'-4' positions, a difluoromethoxy radical or a chlorine atom in the para 4' position with regard to the attachment point of said phenyl ring to the piperidine cycle directly bearing R'₃ when k' = 0 or to the carbon atom of the -CH₂- group when k' = 1;
■ a 3'-N-pyridine ring optionally substituted by a substituent chosen from a chlorine atom, a fluorine atom, a methoxy group, and a methylthio group, said substituent being in the 4' position with regard to the attachment point of said 3'-N-pyridine ring to the piperidine cycle directly bearing R'₃ when k' = 0 or to the carbon atom of the -CH₂- group when k' = 1;
- R'₄ is a group selected in the group consisting of the following groups: -NH-A-R'₅; -NHC(O)-R'₅ and the groups of formulas (II'-a) to (II'-c) below: 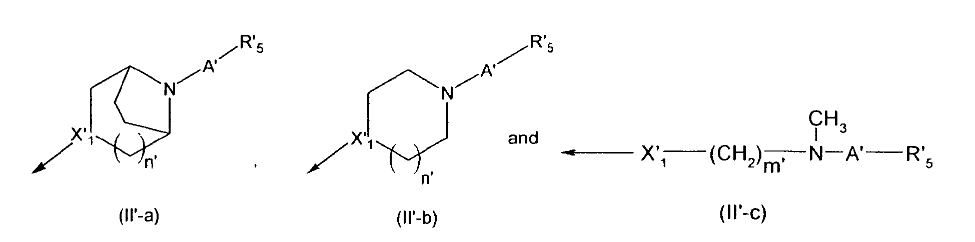
in which:
■ n' is an integer equal to 0 or 1,
■ m' is an integer equal to 0, 1 or 2,
■ the arrows represent the attachment point of groups of formulas (II'-a), (II'-b) or (II'-c) on the formula (I') derivative piperazine-dione cycle;
■ X'₁ is a carbon or a nitrogen atom;
- A' represents a single bond or a C₁ to C₃ linear or branched alkyl chain optionally interrupted or terminated by an oxygen atom;
- R'₅ is selected in the group consisting of:
i) a group of formula (III') below: 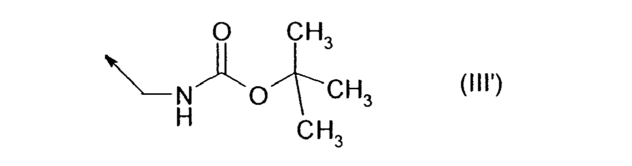 in which the arrow represents the attachment point of said group of formula (III') to A' *via* a covalent bond;
**ii)** a group of formulas (IV') or (V') below: 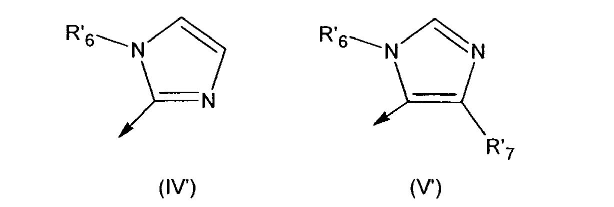 in which the arrows represent the attachment point of said group of formula (IV') and (V') to A' *via* a covalent bond and R'₆ and R'₇, identical or different, represent a C₁ to C₄ alkyl group; and
**iii)** a group of the following formula (VI'): 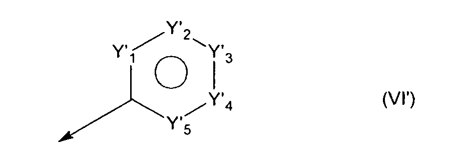
in which:
- the arrow represents the attachment point of said group of formula (VI') to A' *via* a covalent bond,
- Y'₁ and Y'₅, identical or different, represent -CH=; -N= or
- C(R'₈)= in which R'₈ represents an hydrogen atom, a methyl radical or a halogen atom selected from chlorine and fluorine;
- Y'₃ represents -CH=; -N=; -C(R'₉)= or -C(R'₁₀)=;
- Y'₂ and Y'₄, identical or different, represent -CH=, -N=,
- C(R'₉)= or -C(R₁₀)=;
- R'₉ represents a hydrogen atom or a group chosen from a methyl, an amino group and an oxygen group, said groups being optionally mono- or disubstituted with a methyl radical;
- Y'₂ and Y'₃ can also form together a methylenedioxy group;
- R'₁₀ represents a hydrogen atom or a solubilizing group;
wherein the asymmetric carbon atom of said derivative of formula (I) is in the R conformation;
with the provisos that:
* when k = 0, and R'₁ and R'₂ simultaneously represent a hydrogen atom, and R'₃ is a piperonyl group, and R'₄ represents a group of formula (II'-b) wherein n' = 0, X₁ is a carbon or a nitrogen atom, and A' = -CH₂-, then R'₅ is different from a phenyl group, a pyridine group, a pyrimidine group, an imidazole group and a 1-methylimidazole group;
* when k = 0, and R'₁ and R'₂ simultaneously represent a hydrogen atom, and R'₃ is a piperonyl group, and R'₄ represents a group of formula (II'-b) wherein n' = 1, X₁ is a carbon or a nitrogen atom, and A' = -CH₂-, then R'₅ is different from a phenyl group.

17. Chiral tetra-hydro β-carboline derivatives of formula (I') according to claim 16, wherein said derivatives are selected from the group consisting of:
- (6R,12aR)-2-(1-Benzyl-piperidin-4-yl)-6-phenyl-2,3,6,7,12,12a-hexahydro-pyrazino[1',2'-1,6]pyrido[3,4-b]indole-1,4-dione;
- (6R,12aR)-2-((R)-1-Benzyl-pyrrolidin-3-yl)-6-(6-chloro-pyridin-3-yl)-2,3,6,7, 12,12a-hexahydro-pyrazino[1',2':1,6]pyrido[3,4-b]indole-1,4-dione;
- (6S,12aR)-2-((R)-1-Benzyl-pyrrolidin-3-yl)-6-(6-chloro-pyridin-3-yl)-2,3,6,7, 12,12a-hexahydro-pyrazino[1',2':1,6]pyrido[3,4-b]indole-1,4-dione;
- (6R,12aR)-2-(1-Benzyl-piperidin-4-yl)-6-(6-chloro-pyridin-3-yl)-2,3,6,7,12, 12a-hexahydro-pyrazino[1',2':1,6]pyrido[3,4-b]indole-1,4-dione;
- (6R,12aR)-6-Benzo[1,3]dioxol-5-yl-2-[3-(benzyl-methyl-amino)-propyl]-2,3,6, 7,12,12a-hexahydro-pyrazino[1',2':1,6]pyrido[3,4-*b*]indole-1,4-dione;
- (6S,12aR)-6-Benzo[1,3]dioxol-5-yl-2-[3-(benzyl-methyl-amino)-propyl]-2,3,6, 7,12,12a-hexahydro-pyrazino[1',2':1,6]pyrido[3,4-*b*]indole-1,4-dione;
- (6R,12aR)-6-Benzo[1,3]dioxol-5-yl-2-[2-(benzyl-methyl-amino)-ethyl]-2,3,6,7, 12,12a-hexahydro-pyrazino[1',2':1,6]pyrido[3,4-*b*]indole-1,4-dione;
- (6S,12aR)-6-Benzo[1,3]dioxol-5-yl-2-[2-(benzyl-methyl-amino)-ethyl]-2,3,6,7, 12,12a-hexahydro-pyrazino[1',2':1,6]pyrido[3,4-*b*]indole-1,4-dione;
- (6R,12aR)-6-Benzo[1,3]dioxol-5-yl-2-(1-benzyl-piperidin-4-yl)-2,3,6,7,12,12a-hexahydro-pyrazino[1',2':1,6]pyrido[3,4-*b*]indole-1,4-dione;
- (6R,12aR)-6-Benzo[1,3]dioxol-5-yl-2-(1-benzyl-piperidin-4-yl)-9-fluoro-2,3,6, 7,12,12a-hexahydro-pyrazino[1',2':1,6]pyrido[3,4-b]indole-1,4-dione;
- 6-Benzo[1,3]dioxol-5-yl-2-(1-benzyl-piperidin-4-yl)-9-fluoro-2,3,6,7,12,12a-hexahydro-pyrazino[1',2':1,6]pyrido[3,4-b]indole-1,4-dione in the form of a mixture of enantiomers (CIS : (1R,3R) and (1S,3S));
- (6R,12aR)-6-Benzo[1,3]dioxol-5-yl-2-((R)-1-benzo[1,3]dioxol-5-ylmethyl-pyrrolidin-3-yl)-2,3,6,7,12,12a-hexahydro-pyrazino[1',2':1,6]pyrido [3,4-*b*]indole -1,4-dione;
- (6R,12aR)-6-Benzo[1,3]dioxol-5-yl-2-[(R)-1-(4-methoxy-benzyl)-pyrrolidin-3-yl]-2,3,6,7,12,12a-hexahydro-pyrazino[1',2':1,6]pyrido[3,4-*b*]indole-1,4-dione;
- (6R,12aR)-6-Benzo[1,3]dioxol-5-yl-2-[(R)-1-(4-dimethyl-amino-benzyl)-pyrrolidin-3-yl]-2,3,6,7,12,12a-hexahydro-pyrazino[1',2':1,6]pyrido[3,4-b]indole-1,4-dione;
- 2-[(R)-3-((6R,12aR)-6-Benzo[1,3]dioxol-5-yl-1,4-dioxo-3,4,6,7,12,12a-hexa hydro-1*H*-pyrazino[1',2':1,6]pyrido[3,4-*b*]indol-2-yl)-pyrrolidin-1-yl]-ethyl}-carbamic acid *tert*-butyl ester;
- (6R,12aR)-6-Benzo[1,3]dioxol-5-yl-2-[1-(1-methyl-1*H*-imidazol-2-ylmethyl)-piperidin-4-yl]-2,3,6,7,12,12a-hexahydro-pyrazino[1',2':1,6]pyrido[3,4-*b*]indole-1,4-dione;
- (6R,12aR)-2-(1-Benzyl-piperidin-4-yl)-6-(4-difluoromethoxy-phenyl)-2,3,6,7, 12,12a-hexahydro-pyrazino[1',2':1,6]pyrido[3,4-b]indole-1,4-dione;
- (6R,12aR)-2-(1-Benzyl-piperidin-4-yl)-6-(2,2-difluoro-benzo[1,3]dioxol-5-yl)-2, 3,6,7,12,12a-hexahydro-pyrazino[1',2':1,6]pyrido[3,4-*b*]indole-1,4-dione;
- (6R,12aR)-6-Benzo[1,2,5]thiadiazol-5-yl-2-((R)-1-benzyl-pyrrolidin-3-yl)-2,3, 6,7,12,12a-hexahydro-pyrazino[1',2':1,6]pyrido[3,4-*b*]indole-1,4-dione;
- (6S,12aR)-6-Benzo[1,2,5]thiadiazol-5-yl-2-((R)-1-benzyl-pyrrolidin-3-yl)-2,3, 6,7,12,12a-hexahydro-pyrazino[1',2':1,6]pyrido[3,4-*b*]indole-1,4-dione;
- (6R,12aR)-6-Benzyl-2-(1-benzyl-piperidin-4-yl)-2,3,6,7,12,12a-hexahydro-pyrazino[1',2':1,6]pyrido[3,4-*b*]indole-1,4-dione;
- (6S, 12aR)-6-Benzyl-2-(1-benzyl-piperidin-4-yl)-2,3,6,7,12,12a-hexahydro-pyrazino[1',2':1,6]pyrido[3,4-*b*]indole-1,4-dione;
- (6R,12aR)-6-Benzyl-2-[1-(1-methyl-1*H*-imidazol-2-ylmethyl)-piperidin-4-yl]-2, 3,6,7,12,12a-hexahydro-pyrazino[1',2':1,6]pyrido[3,4-*b*]indole-1,4-dione;
- (6S,12aR)-6-Benzyl-2-[1-(1-methyl-1*H*-imidazol-2-ylmethyl)-piperidin-4-yl]-2, 3,6,7,12,12a-hexahydro-pyrazino[1',2':1,6]pyrido[3,4-*b*]indole-1,4-dione; and pharmaceutically acceptable salts thereof.

18. Chiral tetra-hydro β-carboline derivatives of formula (I'), as defined in claim 16 or in claim 17, for a use as a drug.

19. Chiral tetra-hydro β-carboline derivatives of formula (I'), as defined in claim 16 or in claim 17, for a use as a drug for the prevention and/or the treatment of parasitic diseases involving parasites having a phosphodiesterase (PDE) activity.

20. Chiral tetra-hydro β-carboline derivatives of formula (I'), as defined in claim 16 or in claim 17, for a use as a drug for the prevention and/or the treatment of malaria.

21. A pharmaceutical composition comprising, as an active principle, at least one chiral tetra-hydro β-carboline derivative of formula (I') as defined in claim 16 or in claim 17, and at least one pharmaceutically acceptable excipient.
